(19) Europäisches Patentamt
European Patent Office
Office européen des brevets

(11) EP 0 705 831 B1

(12) EUROPÄISCHE PATENTSCHRIFT

(45) Veröffentlichungstag und Bekanntmachung des Hinweises auf die Patenterteilung:
**03.12.2003 Patentblatt 2003/49**

(51) Int Cl.7: **C07D 471/04**, C07D 209/86, A61K 31/44
// (C07D471/04, 221:00, 209:00)

(21) Anmeldenummer: **95114877.4**

(22) Anmeldetag: **21.09.1995**

(54) **Cycloalkano-indol- und -azaindol-derivate**

Cycloalkano-indole- and -azaindole derivatives

Dérivés de cycloalkano-indoles et -azaindoles

(84) Benannte Vertragsstaaten:
**AT BE CH DE DK ES FR GB GR IE IT LI LU MC NL PT SE**
Benannte Erstreckungsstaaten:
**LT LV SI**

(30) Priorität: **04.10.1994 DE 4435477**

(43) Veröffentlichungstag der Anmeldung:
**10.04.1996 Patentblatt 1996/15**

(73) Patentinhaber: **BAYER AG**
**51368 Leverkusen (DE)**

(72) Erfinder:
- **Müller, Ulrich, Dr.**
  **D-42111 Wuppertal (DE)**
- **Connell, Richard, Dr.**
  **Trumbull, CT 06611 (US)**
- **Goldmann, Siegfried, Dr.**
  **D-42327 Wuppertal (DE)**
- **Grützmann, Rudi, Dr.**
  **D-42657 Solingen (DE)**
- **Beuck, Martin, Dr.**
  **Nilford, CT 06460 (US)**
- **Bischoff, Hilmar, Dr.**
  **D-42113 Wuppertal (DE)**
- **Denzer, Dirk, Dr.**
  **D-42115 Wuppertal (DE)**
- **Domdey-Bette, Anke, Dr.**
  **D-42499 Hückeswagen (DE)**
- **Wohlfeil, Stefan, Dr.**
  **D-40724 Hilden (DE)**

(56) Entgegenhaltungen:
**EP-A- 0 234 708** **EP-A- 0 300 676**
**EP-A- 0 310 179** **EP-A- 0 496 237**
**EP-A- 0 617 035** **US-A- 4 775 680**

- **HETEROCYCLES, Bd. 22, Nr. 10, 1984, Seiten 2277-2279, XP002019331 C. HERDEIS ET AL.: "synthesis with hydroxylactames III. A facile entry to the 1-oxo-beta-carboline skeleton. Synthesis of Strychnocarpine"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Cycloalkano-indol- und -azaindol-derivate, Verfahren zu ihrer Herstellung und ihre Verwendung als Arzneimittel, insbesondere als antiatherosklerotische Arzneimittel.
Ähnliche Verbindungen werden in der EP-A-234708, EP-A-300676, US-A-4 775 680, EP-A-310179, EP-A-496237, EP-A-617035 und Heterocycles, 1984, 22 , (10), 2277-9 beschrieben.
Es ist bekannt, daß erhöhte Blutspiegel von Triglyzeriden (Hypertriglyzeridämie) und Cholesterin (Hypercholesterinämie) mit der Genese von atherosklerotischen Gefäßwand-Veränderungen und koronaren Herzkrankheiten assoziiert sind.
Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apoliprotein B-100 einhergeht. Es ist daher nach wie vor ein starkes Bedürfnis, wirksame Arzneimittel zur Bekämpfung der Atherosklerose sowie koronnsarer Herzkrankheiten zur Verfügung zu stellen.
Die vorliegende Erfindung betrifft Cycloalkano-indol- und -azaindol-derivate der allgemeinen Formel (I)

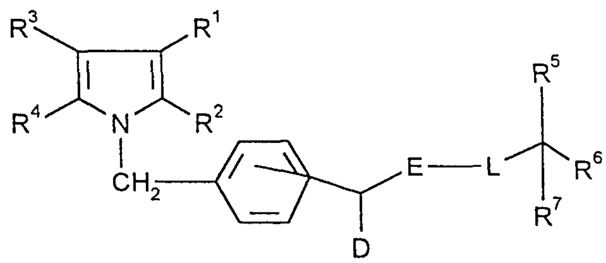

(I),

in welcher

$R^1$ und $R^2$ unter Einbezug der sie verbindenden Doppelbindung gemeinsam einen Phenyl- oder Pyridylring oder einen Ring der Formel

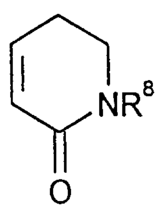

bilden,
worin

$R^8$ Wasserstoff oder geradkettiges oder verzweigtes Alkyl mit bis zu 4 Kohlenstoffatomen bedeutet,

$R^3$ und $R^4$ unter Einbezug der sie verbindenden Doppelbindung gemeinsam einen Phenylring oder einen 4- bis 8-gliedrigen Cycloalken- oder Oxocycloalken-Rest bilden,
wobei alle unter $R^1/R^2$ und $R^3/R^4$ aufgeführten Ringsysteme gegebenenfalls bis zu 3-fach gleich oder verschieden durch Halogen, Trifluormethyl, Carboxy, Hydroxy, durch geradkettiges oder verzweigtes Alkoxy oder Alkoxy-carbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,

D für Cycloalkyl mit 4 bis 12 Kohlenstoffatomen oder für geradkettiges oder verzweigtes Alkyl mit bis zu 12 Kohlenstoffatomen steht,

E für die -CO- oder -CS-Gruppe steht,

L für ein Sauerstoff- oder Schwefelatom steht oder für eine Gruppe der Formel $-NR^9$ steht, worin

$R^9$ Wasserstoff oder geradkettiges oder verzweigtes Alkyl mit bis zu 6 Kohlenstoffatomen steht, das gegebenenfalls durch Hydroxy oder Phenyl substituiert ist,

$R^5$ für Phenyl oder für einen 5- bis 7-gliedrigen gesättigten oder ungesättigten Heterocyclus mit bis zu 3 Heteroatomen aus der Reihe S, N und/oder O steht,
wobei die Cyclen gegebenenfalls bis zu 3-fach gleich oder verschieden durch Nitro, Carboxy, Halogen, Cyano oder durch geradkettiges oder verzweigtes Alkenyl oder Alkoxycarbonyl mit jeweils bis zu 6 Kohlenstoffatomen oder durch geradkettiges oder verzweigtes Alkyl mit bis zu 6 Kohlenstoffatomen substituiert sind, das gegebenenfalls durch Hydroxy, Carboxy oder durch geradkettiges oder verzweigtes Alkoxy oder Alkoxy-carbonyl mit jeweils bis zu 6 Kohlenstoffatomen substituiert ist,
und/oder die Cyclen gegebenenfalls durch eine Gruppe der Formel $-OR^{10}$ oder $-NR^{11}R^{12}$ substituiert sind,
worin

$R^{10}$ Wasserstoff oder geradkettiges oder verzweigtes Alkyl oder Alkenyl mit jeweils bis zu 6 Kohlenstoffatomen bedeutet,

$R^{11}$ bzw. $R^{12}$ gleich oder verschieden sind und Phenyl, Wasserstoff oder geradkettiges oder verzweigtes Alkyl mit bis zu 6 Kohlenstoffatomen bedeuten
oder geradkettiges oder verzweigtes Acyl mit bis zu 8 Kohlenstoffatomen bedeuten, das gegebenenfalls durch eine Gruppe der Formel $-NR^{13}R^{14}$ substituiert ist,
worin

$R^{13}$ und $R^{14}$ gleich oder verschieden sind und Wasserstoff oder geradkettiges oder verzweigtes Acyl mit bis zu 8 Kohlenstoffatomen bedeuten,

$R^6$ für Wasserstoff, Carboxy oder für geradkettiges oder verzweigtes Alkoxycarbonyl mit bis zu 5 Kohlenstoffatomen steht,
oder für geradkettiges oder verzweigtes Alkyl mit bis zu 6 Kohlenstoffatomen steht, das gegebenenfalls durch Hydroxyl oder durch eine Gruppe der Formel $-O-CO-R^{15}$ substituiert ist,
worin

$R^{15}$ Phenyl bedeutet, das gegebenenfalls bis zu 3-fach gleich oder verschieden durch Halogen, Hydroxy oder durch geradkettiges oder verzweigtes Alkyl mit bis zu 5 Kohlenstoffatomen substituiert ist, oder geradkettiges oder verzweigtes Alkyl oder Alkenyl mit jeweils bis zu 22 Kohlenstoffatomen bedeutet, die gegebenenfalls durch eine Gruppe der Formel $-OR^{16}$ substituiert sind,
worin

$R^{16}$ Wasserstoff, Benzyl, Triphenylmethyl oder geradkettiges oder verzweigtes Acyl mit bis zu 6 Kohlenstoffatomen bedeutet,

$R^7$ für Wasserstoff steht oder

$R^6$ und $R^7$ gemeinsam für die Gruppe der Formel =O stehen,

gegebenenfalls in einer isomeren Form und deren Salze.

Die erfindungsgemäßen Cycloalkano-indol- und azaindol-derivate können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen

Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di-bzw. Triethanolamin, Dicyclohexylamin, Dimethylaminoethanol, Arginin, Lysin, Ethylendiamin oder 2-Phenylethylamin.

Der Cycloalken-Rest ($R^3$/$R^4$) steht unter Einbezug der Doppelbindung des Grundgerüstes im Rahmen der Erfindung im allgemeinen für einen 4- bis 8-gliedrigen, vorzugsweise 5- bis 8-gliedrigen Kohlenwasserstoffrest wie beispielsweise für einen Cyclobuten-, Cyclopenten-, Cyclohexen-, Cyclohepten- oder Cycloocten-Rest. Bevorzugt sind der Cyclopenten-, Cyclohexen-, Cycloocten- und Cyclohepten-Rest.

Heterocyclus ($R^5$) steht im Rahmen der Erfindung im allgemeinen für einen gesättigten oder ungesättigten 5- bis 7-gliedrigen, vorzugsweise 5- bis 6-gliedrigen Heterocyclus der bis zu 3 Heteroatome aus der Reihe S, N und/oder O enthalten kann. Beispielsweise seien genannt: Pyridyl, Thienyl, Furyl, Pyrrolyl, Thiazolyl, Oxazolyl, Imidazolyl, Morpholinyl oder Piperidyl. Bevorzugt sind Pyridyl und Thienyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Bevorzugt sind Verbindungen der allgemeinen Formel (I),
in welcher

$R^1$ und $R^2$ unter Einbezug der sie verbindenden Doppelbindung gemeinsam einen Phenyl- oder Pyridylring oder einen Ring der Formel

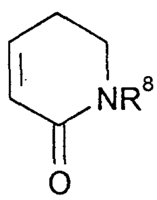

bilden,
worin

$R^8$ Wasserstoff oder geradkettiges oder verzweigtes Alkyl mit bis zu 3 Kohlenstoffatomen bedeutet,

$R^3$ und $R^4$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
wobei alle unter $R^1$/$R^2$ und $R^3$/$R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert sein kann,

D für Cyclobutyl, Cyclopentyl, Cyclohexyl, Cycloheptyl, Cyclooctyl oder für geradkettiges oder verzweigtes Alkyl mit bis zu 10 Kohlenstoffatomen steht,

E für die -CO- oder -CS-Gruppe steht,

L für ein Sauerstoff- oder Schwefelatom steht oder für eine Gruppe der Formel $-NR^9$ steht,
worin

$R^9$ Wasserstoff oder geradkettiges oder verzweigtes Alkyl mit bis zu 5 Kohlenstoffatomen steht, das gegebenenfalls durch Hydroxy oder Phenyl substituiert ist,

$R^5$ für Phenyl, Pyridyl, Furyl, Thienyl oder Imidazolyl steht, die gegebenenfalls bis zu 2-fach gleich oder verschieden durch Nitro, Carboxy, Fluor, Chlor, Brom, Cyano, durch geradkettiges oder verzweigtes

Alkenyl oder Alkoxycarbonyl mit jeweils bis zu 4 Kohlenstoffatomen oder durch geradkettiges oder verzweigtes Alkyl mit bis zu 5 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 5 Kohlenstoffatomen substituiert ist,
und/oder die Cyclen gegebenenfalls durch eine Gruppe der Formel $-OR^{10}$ oder $-NR^{11}R^{12}$ substituiert sind,
worin

$R^{10}$ Wasserstoff oder geradkettiges oder verzweigtes Alkyl oder Alkenyl mit jeweils bis zu 4 Kohlenstoffatomen bedeutet,
$R^{11}$ und $R^{12}$ gleich oder verschieden sind und Phenyl, Wasserstoff oder geradkettiges oder verzweigtes Alkyl mit bis zu 5 Kohlenstoffatomen bedeuten,
oder geradkettiges oder verzweigtes Acyl mit bis zu 6 Kohlenstoffatomen bedeuten, das gegebenenfalls durch eine Gruppe der Formel $-NR^{13}R^{14}$ subsituiert ist,
worin

$R^{13}$ und $R^{14}$ gleich oder verschieden sind und Wasserstoff oder geradkettiges oder verzweigtes Acyl mit bis zu 6 Kohlenstoffatomen bedeuten,

$R^6$ für Wasserstoff, Carboxy oder für geradkettiges oder verzweigtes Alkoxy-carbonyl mit bis zu 4 Kohlenstoffatomen steht,
oder für geradkettiges oder verzweigtes Alkyl mit bis zu 5 Kohlenstoffatomen steht, das gegebenenfalls durch Hydroxyl oder durch eine Gruppe der Formel $-O-CO-R^{15}$ substituiert ist,
worin

$R^{15}$ Phenyl bedeutet, das gegebenenfalls bis zu 3-fach gleich oder verschieden durch Fluor, Chlor, Brom, Hydroxy oder durch geradkettiges oder verzweigtes Alkyl bis zu 4 Kohlenstoffatomen substituiert ist,
oder geradkettiges oder verzweigtes Alkyl oder Alkenyl mit jeweils bis zu 20 Kohlenstoffatomen bedeutet, die gegebenenfalls durch eine Gruppe der Formel $-OR^{16}$ substituiert sind,
worin

$R^{16}$ Wasserstoff, Benzyl, Triphenylmethyl oder geradkettiges oder verzweigtes Acyl mit bis zu 5 Kohlenstoffatomen bedeutet,

$R^7$ für Wasserstoff steht oder
$R^6$ und $R^7$ gemeinsam für die Gruppe der Formel =O stehen,

gegebenenfalls in einer isomeren Form und deren Salze.

Besonders bevorzugt sind Verbindungen der allgemeinen Formel (I), in welcher

$R^1$ und $R^2$ unter Einbezug der sie verbindenden Doppelbindung gemeinsam einen Phenyl- oder Pyridylring oder einen Ring der Formel

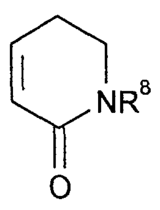

bilden,
worin

$R^8$ Wasserstoff oder Methyl bedeutet,

$R^3$ und $R^4$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
wobei alle unter $R^1$/$R^2$ und $R^3$/$R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert sein kann,

D für Cyclopentyl, Cyclohexyl, Cycloheptyl, Cylcooctyl oder für geradkettiges oder verzweigtes Alkyl mit bis zu 6 Kohlenstoffatomen steht,

E für die -CO- oder -CS-Gruppe steht,

L für ein Sauerstoff- oder Schwefelatom steht oder für eine Gruppe der Formel $-NR^9$ steht,
worin

$R^9$ Wasserstoff oder geradkettiges oder verzweigtes Alkyl mit bis zu 4 Kohlenstoffatomen steht, das gegebenenfalls durch Hydroxy oder Phenyl substituiert ist,

$R^5$ für Phenyl, Pyridyl oder Thienyl steht, die gegebenenfalls bis zu 2-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 3 Kohlenstoffatomen oder durch geradkettiges oder verzweigtes Alkyl mit bis zu 4 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 4 Kohlenstoffatomen substituiert ist,
und/oder die Cyclen gegebenenfalls durch eine Gruppe der Formel $-OR^{10}$ oder $-NR^{11}R^{12}$ substituiert sind,
worin

$R^{10}$ Wasserstoff oder geradkettiges oder verzweigtes Alkyl oder Alkenyl mit jeweils bis zu 3 Kohlenstoffatomen bedeutet,
$R^{11}$ und $R^{12}$ gleich oder verschieden sind und Phenyl, Wasserstoff oder geradkettiges oder verzweigtes Alkyl mit bis zu 4 Kohlenstoffatomen bedeuten,
oder geradkettiges oder verzweigtes Acyl mit bis zu 5 Kohlenstoffatomen bedeuten, das gegebenenfalls durch eine Gruppe der Formel $-NR^{13}R^{14}$ substituiert ist,
worin

$R^{13}$ und $R^{14}$ gleich oder verschieden sind und Wasserstoff oder geradkettiges oder verzweigtes Acyl mit bis zu 5 Kohlenstoffatomen bedeuten,

$R^6$ für Wasserstoff, Carboxy oder für geradkettiges oder verzweigtes Alkoxycarbonyl mit bis zu 3 Kohlenstoffatomen steht,
oder für geradkettiges oder verzweigtes Alkyl mit bis zu 4 Kohlenstoffatomen steht, das gegebenenfalls durch Hydroxyl oder durch eine Gruppe der Formel $-O-CO-R^{15}$ substituiert ist,
worin

$R^{15}$ Phenyl bedeutet, das gegebenenfalls bis zu 3-fach gleich oder verschieden durch geradkettiges oder verzweigtes Alkyl mit bis zu 3 Kohlenstoffatomen substituiert ist,
oder geradkettiges oder verzweigtes Alkyl oder Alkenyl mit jeweils bis zu 19 Kohlenstoffatomen bedeutet, die gegebenenfalls durch eine Gruppe der Formel $-OR^{16}$ substituiert sind,
worin

$R^{16}$ Wasserstoff, Benzyl, Triphenylmethyl oder geradkettiges oder verzweigtes Acyl mit bis zu 4 Kohlenstoffatomen bedeutet,

$R^7$ für Wasserstoff steht oder

$R^6$ und $R^7$ gemeinsam für die Gruppe der Formel =O stehen,

gegebenenfalls in einer isomeren Form und deren Salze.

Außerdem wurde ein Verfahren zur Herstellung der erfindungsgemäßen Verbindungen der allgemeinen Formel (I) gefunden, dadurch gekennzeichnet, daß man
Carbonsäuren der allgemeinen Formel (II)

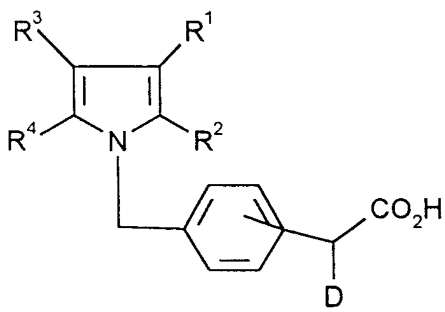

(II),

in welcher
$R^1$, $R^2$, $R^3$, $R^4$ und D die angegebene Bedeutung haben,
mit Verbindungen der allgemeinen Formel (III)

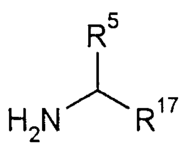

(III),

in welcher

$R^5$ die angegebene Bedeutung hat

und

$R^{17}$ die angegebene Bedeutung von $R^6$ hat, aber nicht für Carboxy steht,

in einem inerten Lösemittel und in Anwesenheit von Basen und/oder Hilfsstoffen amidiert,
und gegebenenfalls funktionelle Gruppen durch Hydrolyse, Veresterung oder Reduktion variiert.

Die erfindungsgemäßen Verfahren können durch folgendes Formelschema beispielhaft erläutert werden:

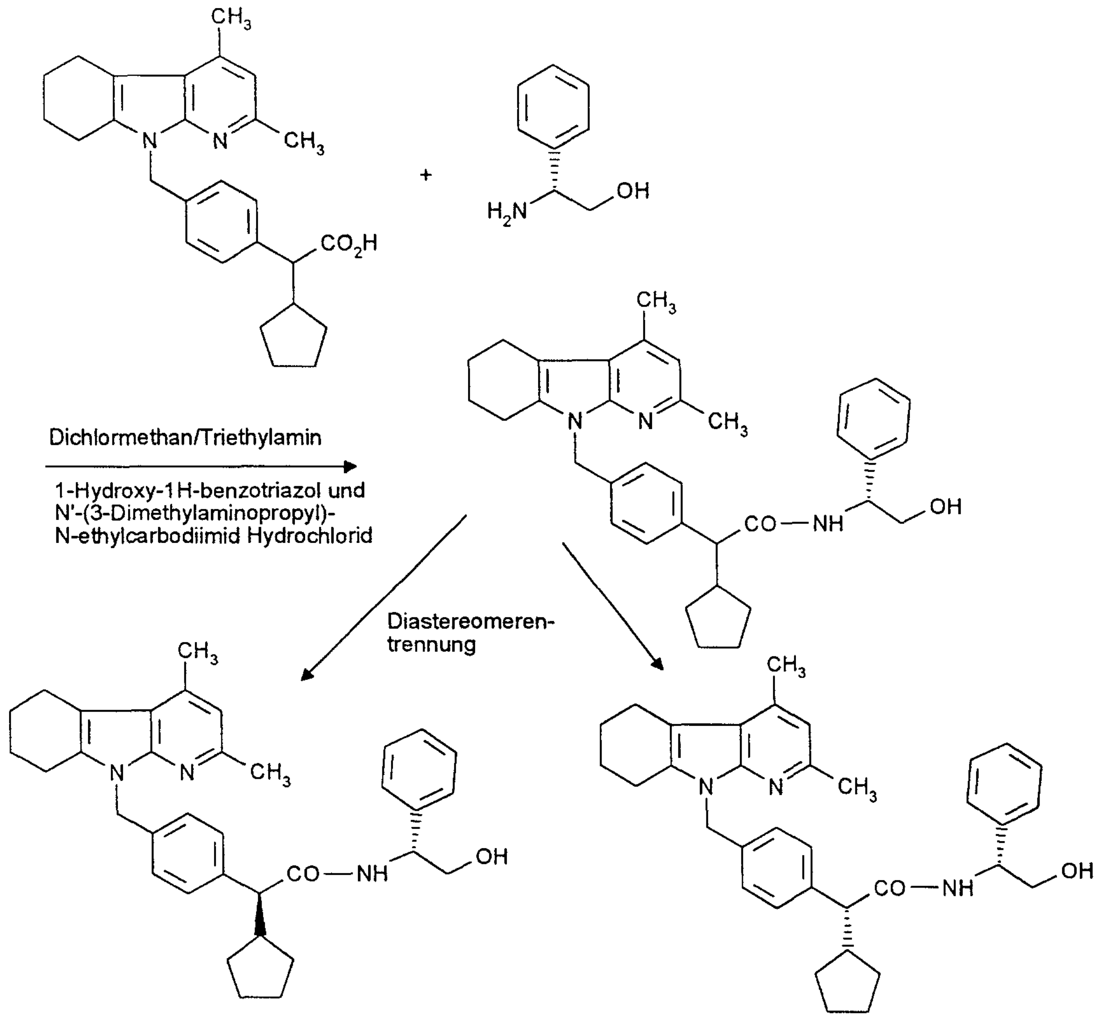


Als Lösemittel für die Amidierung eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton oder Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natriumoder Kaliumethanolat oder Kalium-tert.butylat, oder organische Amine (Trialkyl-($C_1$-$C_6$)amine) wie Triethylamin, oder Heterocyclen wie 1,4-Diazabicyclo[2.2.2]-octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium und deren Hydride wie Natriumhydrid, einzusetzen. Bevorzugt sind Natrium- und Kaliumcarbonat und Triethylamin.

Die Base wird in einer Menge von 1 mol bis 5 mol, bevorzugt von 1 mol bis 3 mol, bezogen auf 1 mol der Verbindung der allgemeinen Formel (II), eingesetzt.

Die Reaktion wird im allgemeinen in einem Temperaturbereich von 0°C bis 150°C, bevorzugt von +20°C bis +110°C, durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Die Amidierung kann gegebenenfalls auch über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die oben aufgeführten Basen können gegebenenfalls auch als säurebindende Hilfsmittel für die Amidierung eingesetzt werden.

Als Hilfsmittel eignen sich ebenso Dehydratisierungsreagenzien. Dazu gehören beispielsweise Carbodiimide wie Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Iso-butylchloroformat oder Benzotriazolyloxy-tris-(dimethylamino)phosphoniumhexyl-fluorophosphat oder Phosphonsäurediphenyl-esteramid oder Methan-sulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Variation von funktionellen Gruppen wie beispielsweise Hydrolyse, Veresterung und Reduktion, sowie die Isomerentrennung und Salzbildung erfolgt nach üblichen Methoden.

Die Carbonsäuren der allgemeinen Formel (II) sind neu und können hergestellt werden, indem man Verbindungen der allgemeinen Formel (IV)

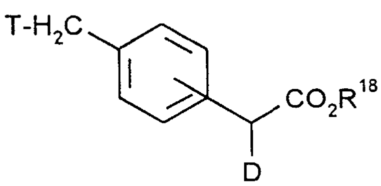

(IV),

in welcher

D die angegebene Bedeutung hat,

T für eine typische Abgangsgruppe wie beispielsweise Chlor, Brom, Jod, Tosylat oder Mesylat, vorzugsweise für Brom, steht,

und

$R^{18}$ für $(C_1-C_4)$-Alkyl steht,

mit Verbindungen der allgemeinen Formel (V)

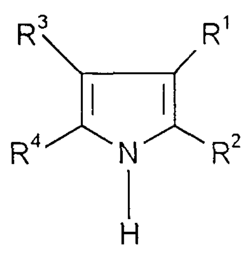

(V),

in welcher
$R^1$, $R^2$, $R^3$ und $R^4$ die angegebene Bedeutung haben,
in inerten Lösemitteln, gegebenenfalls in Anwesenheit einer Base, umsetzt.

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natriumoder Kaliumethanolat oder Kalium-tert.butylat, oder organische Amine (Trialkyl-($C_1$-$C_6$)amine) wie Triethylamin, oder Heterocyclen wie 1,4-Diazabicyclo[2.2.2]-octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formel (IV), ein.

Das erfindungsgemäße Verfahren wird im allgemeinen in einem Temperaturbereich von -30°C bis +100°C, bevorzugt von -10°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (III) sind an sich bekannt.

Die Verbindungen der allgemeinen Formel (IV) sind bekannt oder können in Analogie zu bekannten Methoden hergestellt werden.

Die Verbindungen der allgemeinen Formeln (V) sind bekannt oder können in Analogie zu bekannten Methoden hergestellt werden.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche

befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

| **Bsp. -Nr.** | **$IC_{50}$ [$10^{-9}$ mol/l]** |
|---|---|
| 1 | 28 |
| 5 | 1,1 |
| 31 | 170 |
| 50 | 29 |

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i. p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nM in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

| **Bsp. Nr.** | **$ED_{50}$ [mg/kg] p.o.** |
|---|---|
| 2 | 10-15 |
| 5 | 3-6 |
| 7 | 10-20 |

**3. Hemmung der intestinalen Triglyceridabsorption in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspension, die

Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Trigylceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{Substanz} - \Delta TG_{Traganthkontrolle}}{\Delta TG_{Ölbelastung} - \Delta TG_{Traganthkontrolle}} \times 100$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

| | **Serumtriglyceridanstieg in % (2 h pp)** |
|---|---|
| **Triglyceridbelastung** | 100 |
| **Traganthkontrolle** | 0 |
| **Substanz 10 mg/kg KG p. o.** | |
| Bsp.-Nr. 10 | 34 |
| Bsp.-Nr. 66 | 67 |
| Bsp.-Nr. 54 | 54 |
| Bsp.-Nr. 71 | 18 |
| Bsp.-Nr. 5 | -16 |
| Bsp.-Nr. 20 | 35 |

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0{,}05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339, gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholesterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von Cycloalkano-indol- und -azaindol-Derivaten der allgemeinen Formel (I) mit einem Glucosidase- und/oder Amylasehemmer zur Behandlung von familiärer Hyperlipidamien, der Fettsucht (Adipositas) und des Diabetes mellitus. Glucosidase- und/oder Amylasehemmer im Rahmen der Erfindung sind beispielsweise Acarbase, Adiposine, Voglibase, Miglitol, Emiglitate, MDL-25637, Camiglibase (MDL-73945), Tendamistate, AI-3688, Trestatin, Pradimilin-Q und Salbostatin.

Bevorzugt ist die Kombination von Acarbase, Miglitol, Emiglitate oder Voglibase mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Definition der Isomerentypen:**

| | |
|---|---|
| 4 dia = | Gemisch der vier möglichen Diastereomeren bei zwei Asymmetriezentren im Molekül |
| dia A = | Diastereomer mit dem größeren $R_f$-Wert |
| dia B = | Diastereomer mit dem kleineren $R_f$-Wert |
| ent = | Enantiomer |
| 2 ent dia = | Gemisch zweier enantiomerenreiner Diastereomere |
| ent dia A = | enantiomerenreines Diastereomer mit dem größeren $R_f$-Wert |
| ent dia B = | enantiomerenreines Diastereomer mit dem kleineren $R_f$-Wert |
| R = | R-Enantiomer |
| rac = | Racemat |
| rac dia A = | racemisches Diastereomer mit dem größeren $R_f$-Wert |
| rac dia B = | racemisches Diastereomer mit dem kleineren $R_f$-Wert |
| S = | S-Enantiomer |

**Verwendete Abkürzungen:**

| | |
|---|---|
| Ac = | Acetyl |

| | |
|---|---|
| Bn = | Benzyl |
| Bz = | Benzoyl |
| iBu = | iso Butyl |
| nBu = | normal Butyl |
| sBu = | sekundär Butyl |
| tBu = | tertiär Butyl |
| DDQ = | 2,3-Dichlor-5,6-dicyano-1,4-benzochinon |
| cDec = | cyclo-Decyl |
| DMF = | N,N-Dimethylformamid |
| DMSO = | Dimethylsulfoxid |
| cDodec = | cyclo-Dodecyl |
| Et = | Ethyl |
| cHept = | cyclo-Heptyl |
| cHex = | cyclo-Hexyl |
| HOBT = | 1-Hydroxy-1H-benzotriazol |
| Me = | Methyl |
| Mes = | Mesyl |
| cNon = | cyclo-Nonyl |
| cOct = | cyclo-Octyl |
| cPent = | cyclo-Pentyl |
| nPent = | normal Pentyl |
| Ph = | Phenyl |
| cPr = | cyclo-Propyl |
| nPr = | normal Propyl |
| iPr = | iso Propyl |
| THF = | Tetrahydrofuran |
| TMS = | Tetramethylsilan |
| pTol = | para Tolyl |
| pTos = | para Tosyl |
| cUndec = | cyclo-Undecyl |

| **Solvens** | **Bezeichnung** |
|---|---|
| Dichlormethan : Methanol = 20:1 | A |
| Dichlormethan : Methanol = 50:1 | B |
| Dichlormethan : Ethanol = 20:1 | C |
| Dichlormethan : Ethanol = 50:1 | D |
| Petrolether : Essigsäureethylester = 1:1 | E |
| Dichlormethan : Methanol : Essigsäure = 90:10:2 | F |
| Petrolether : Essigsäureethylester = 2:1 | G |
| Petrolether : Essigsäureethylester = 10:1 | H |
| Toluol | I |
| Toluol : Essigsäureethylester = 1:1 | K |
| Petrolether : Essigsäureethylester = 5:1 | L |
| Dichlormethan | M |
| Petrolether : Essigsäureethylester = 20:1 | N |
| Dichlormethan : Methanol = 10:1 | O |
| Cyclohexan : Essigsäureethylester = 1:1 | P |
| Toluol : Essigsäureethylester = 9:1 | Q |
| Toluol : Essigsäureethylester = 8:1 | R |
| Petrolether : Essigsäureethylester = 1:2 | S |
| Dichlormethan : Ethanol = 5:1 | T |
| Dichlormethan : Ethanol = 10:1 | U |

**Zubereitungsvorschrift für das DC-Laufmittel BABA:**

87,9 ml einer wäßrigen 0,06667 molaren Kaliumdihydrogenphosphatlösung und 12,1 ml einer wäßrigen 0,06667 molaren Dinatriumhydrogenphosphatlösung werden gemischt. 60 ml der so zubereiteten Lösung werden mit 200 ml n-Butylacetat, 36 ml n-Butanol und 100 ml Eisessig geschüttelt und die wäßrige Phase abgetrennt. Die organische Phase ist das Laufmittel BABA.

**Ausgangsverbindungen**

**Beispiel I**

1-Allyloxy-2-chlormethyl-benzol

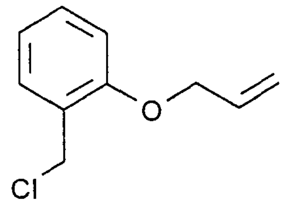

11,5 g (70 mmol) 1-Allyloxy-2-hydroxymethyl-benzol werden bei 0°C in 110 ml Dichlormethan mit 11,6 ml (84 mmol) Triethylamin versetzt und dann langsam mit 5,4 ml (70 mmol) Methansulfonsäurechlorid umgesetzt. Nach 4 Stunden wird mehrfach mit Wasser extrahiert, die organische Phase über Magnesiumsulfat getrocknet und eingedampft. Restlösemittel wird im Hochvakuum entfernt.
Ausbeute: 8,5 g $R_f$ = 0,23 (Dichlormethan : Ethanol = 20:1)

**Beispiel II**

(2-Allyloxy-benzyl)amin

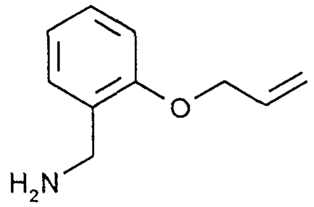

3,0 g (16,4 mmol) der Verbindung aus Beispiel I werden 17 Stunden in 250 ml einer gesättigten methanolischen Ammoniaklösung unter Rückfluß gekocht. Die Reaktionsmsichung wird im Vakuum eingedampft, in Methanol aufgenommen und wieder eingedampft; dieser Vorgang wird einige Male wiederholt. Das rohe Produkt wird in Dichlormethan aufgenommen und mehrfach mit Wasser extrahiert. Die wäßrige Phase wird weitgehendst eingedampft, wobei ein Öl anfällt, das beim Stehen kristallisiert.
Ausbeute: 0,454 g (roh)
Das Produkt wird ohne weitere Reinigung weiter umgesetzt.
$R_f$ = 0,41 (Laufmittel: BABA)

**Beispiel III**

6-Chlor-2,4-lutidin

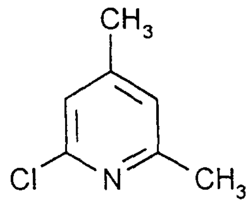

Zur Darstellung der Titelverbindung [US 36 32 807] werden 600 g (4,91 mol) 6-Amino-2,4-lutidin in 2 l Methanol gelöst und die Lösung bei ca. 0°C mit Chlorwasserstoffgas gesättigt. Bei einer Innentemperatur unter 10°C werden 1,307 l (9,82 mol) Isopentylnitrit zugetropft (ca. 2,5 h) und das Gemisch unter Erwärmung auf Raumtemperatur (ca. 25°C) 15 h so belassen. Die Lösung wird im Vakuum weitgehend vom Lösemittel befreit, mit 3 l Dichlormethan und 1,5 l Wasser versetzt und unter Kühlung (< 20°C) mit konzentrierter wäßriger Ammoniaklösung auf einen pH = 9,5 gestellt. Die abgetrennte organische Phase wird mit Natriumsulfat getrocknet, zuerst am Rotationsverdampfer im Vakuum eingeengt und dann über eine Vigreux-Kolonne destilliert:
Fraktion 1) Kp. = 47-49°C (12 Torr), 603 g
Fraktion 2) Kp. = 82-85°C (12 Torr), 612 g (ca. 88% roh)
$R_f$ = 0,39 (Petrolether : Essigester = 10:1)
$^1$H-NMR ($CDCl_3$, 200 MHz, TMS): δ = 2,28 (S, 3H), 2,47 (S, 3H), 6,88 (S, 1H), 6,96 (S, 1H) ppm.
Das rohe Produkt, das kleine Mengen 6-Methoxy-2,4-lutidin enthalten kann, wird ohne weitere Reinigung weiter umgesetzt.

**Beispiel IV**

6-Hydrazino-2,4-lutidin (4,6-Dimethyl-2-hydrazino-pyridin)

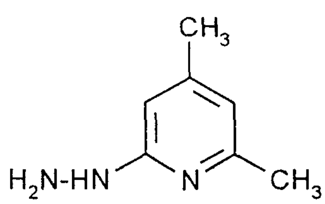

580 g (4,10 mol) der Verbindung aus Beispiel III werden in 800 ml Diethylenglycol gelöst und mit 1050 ml Hydrazin-Hydrat 48 h bei einer Badtemperatur von ca. 140°C gerührt. Der abgekühlte Ansatz wird auf 4,5 l Ether und 4,5 l Wasser gegossen und die organische Phase wird zweimal mit je 2,3 l Dichlormethan extrahiert. Die vereinigten organischen Phasen werden mit Natriumsulfat getrocknet und im Vakuum eingedampft. Es fallen 784 g lösemittelhaltiges Rohprodukt an, das ohne Aufarbeitung weiter umgesetzt wird.
$R_f \simeq$ 0,37 (Dichlormethan : Methanol = 10:1)
$^1$H-NMR ($d_6$-DMSO, 250 MHz, TMS): δ = 2,13 (S, 3H), 2,22 (S, 3H), 4,02 (S, 2H), 6,26 (S, 1H), 6,35 (S, 1H), 7,11 (S, 1H) ppm.

**Beispiel V**

2-Hydrazino-4-picolin (2-Hydrazino-4-methylpyridin)

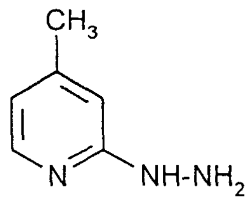

In Analogie zur Vorschrift des Beispiels IV wird 2-Hydrazino-4-picolin aus 2-Chlor-4-picolin hergestellt.
$R_f$ = 0,06 (Dichlormethan : Methanol = 10:1)

**Beispiel VI**

2,4-Dimethyl-5,6,7,8-tetrahydro-α-carbolin

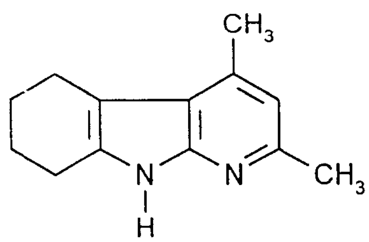

78 g (max. 0,49 mol) rohe Verbindung aus Beispiel IV werden bei Raumtemperatur (ca. 25°C) mit 59 ml (0,56 mol) Cyclohexanon umgesetzt, wobei die Innentemperatur ansteigt. Nach 2 h ist das Edukt verschwunden (DC-Kontrolle; Dichlormethan : Methanol = 10:1). Die Mischung wird in 40 ml Diethylenglycol aufgenommen und unter Rückfluß umgesetzt, dabei werden tiefer als das Lösemittel siedende Bestandteile (z.B. Reaktionswasser und überschüssiges Cyclohexanon) destillativ entfernt (Wasserabscheider). Nach 3 h ist das intermediäre Hydrazon verschwunden (DC-Kontrolle; Petrolether : Essigester = 1:1); das Reaktionsgemisch wird auf Raumtemperatur abgekühlt und mit Aceton verrührt. Der anfallende Niederschlag wird abgesaugt, mit Aceton nachgewaschen und im Vakuum getrocknet (34,4 g). Die vom Lösemittel weitgehend befreiten Mutterlaugen werden wiederum mit Aceton behandelt, wobei weitere 9,3 g Produkt anfallen (Gesamtausbeute über 3 Stufen: 43,7 g / 0,22 mol / 47%).
Schmp.: 248°C (unkorrigiert)
$R_f$ = 0,41 (Dichlormethan : Ethanol = 20:1)
$^1$H-NMR ($d_6$-DMSO, 200 MHz, TMS): δ = 1,78 (M, 4H), 2,40 (S, 3H), 2,48 (S, 3H), 2,64 (M, 2H), 2,82 (M, 2H), 6,57 (S, 1H), 10,84 (S, 1H) ppm.

Die Verbindungen der Tabelle I werden analog der Vorschrift von Beispiel VI hergestellt:

**Tabelle I:**

| Bsp.-Nr. | | $R_f$ (Solvens) | Ausgangsmaterial (Hydrazin *) |
|---|---|---|---|
| VII | 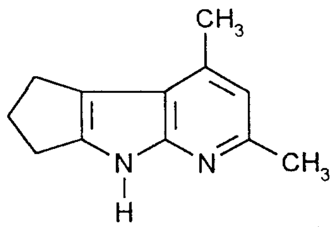 | 0,59 (A) | Bsp.-Nr. IV |
| VIII | 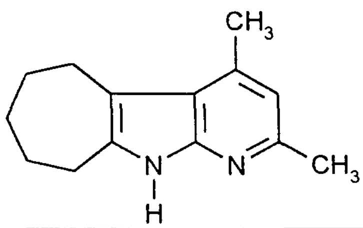 | 0,36 (E) | Bsp.-Nr. IV |
| IX | 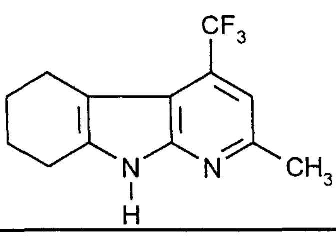 | 0,45 (G) | |
| X | 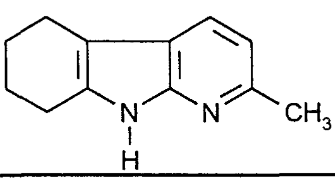 | 0,46 (E) | |
| XI | 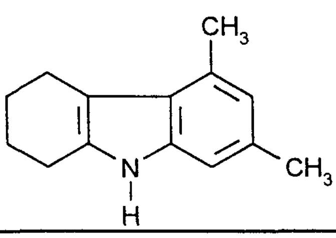 | 0,06 (L) | |
| XII | 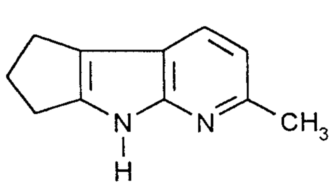 | 0,41 (E) | |
| XIII | 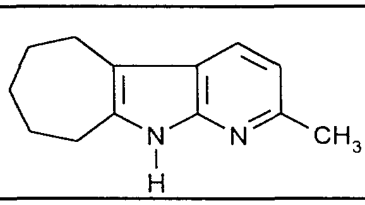 | 0,40 (E) | |

| Bsp.-Nr. | | $R_f$ (Solvens) | Ausgangsmaterial (Hydrazin *) |
|---|---|---|---|
| XIV | 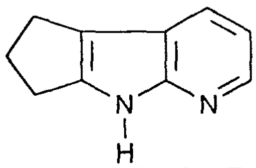 | 0,59 (O) | |
| XV | 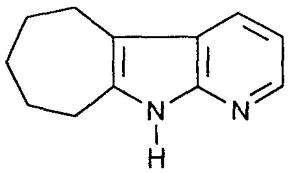 | 0,34 (E) | |
| XVI | 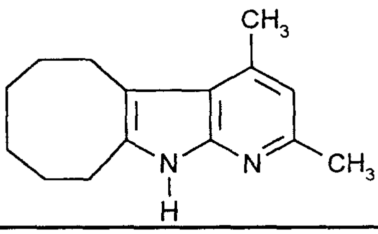 | 0,42 (E) | |
| XVII | 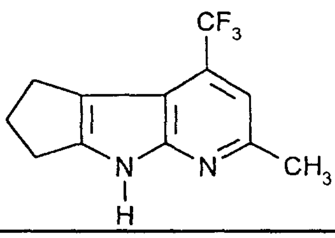 | 0,59 (G) | |
| XVIII | 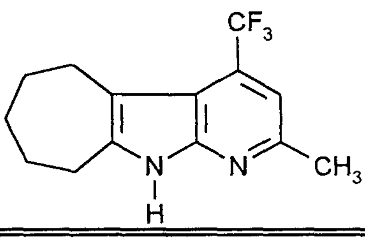 | 0,85 (G) | |

**Beispiel XIX**

2,4-Dimethyl-α-carbolin

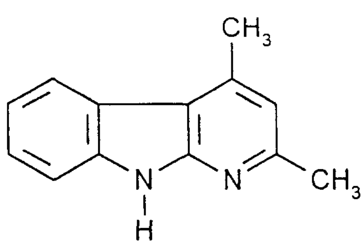

100 g (499 mmol) der Verbindung aus Beispiel VI werden in 700 ml Diethylenglykol mit 164 ml (1 mol) Fumarsäurediethylester an 52 g Palladium (5% auf Kohle) unter Rückfluß umgesetzt. Bei der hohen Innentemperatur destilliert eine kleine Menge Ethanol ab (ggfs. Wasserabscheider verwenden). Nach ca. 8 h ist das Edukt verschwunden (DC-Kontrolle; Petrolether : Essigester = 1:1, Detektion in der Jodkammer). Das abgekühlte Gemisch wird mit 3 l Aceton versetzt, aufgekocht, heiß über einen Klärfilter (Fa. Seitz) abgesaugt und mit 1 l heißem Aceton nachgewaschen. Beim Abkühlen fällt ein Niederschlag an, der nach Absaugen, Spülen mit kaltem Aceton und Trocknen im Vakuum 58,3 g Produkt liefert. Die Mutterlauge wird im Vakuum weitgehend vom Aceton befreit, wobei der ausfallende Niederschlag wie oben aufgearbeitet wird (9,4 g). Das Filtrat wird wiederum vom Aceton befreit; nach Zugabe von n-Pentan fällt ein weiteres Mal Produkt aus (3,1 g / Aufarbeitung s.o.); Gesamtausbeute: 72%.
Schmp.: 220-221°C (unkorrigiert)
$R_f$ = 0,47 (Petrolether : Essigester =1:1)
$^1$H-NMR ($d_6$-DMSO, 200 MHz, TMS): $\delta$ = 2,54 (S, 3H), 2,75 (S, 3H), 6,89 (S, 1H), 7,20 (M, 1H), 7,40 (M, 1H), 7,48 (DD, 1H), 8,05 (DD, 1H), 11,61 (S, 1H) ppm.

**Beispiel XX**

4-Methylphenyl-essigsäure-tert.butylester

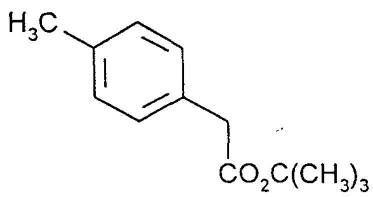

450 g (3 mol) 4-Methylphenyl-essigsäure (Aldrich), 1,13 l (12 mol) tert.Butanol und 90 g (0,74 mol) 4-(N, N-Dimethylamino)pyridin werden in 2 l Dichlormethan gelöst. Nach Zugabe von 680 g (3,3 mol) Dicylcohexylcarbodiimid, gelöst in 400 ml Dichlormethan, wird 20 h bei 25°C gerührt, der ausgefallene Harnstoff abgesaugt, mit 200 ml Dichlormethan gewaschen und die organische Phase je zweimal mit 500 ml 2 M Salzsäure und Wasser gewachen. Die organische Phase wird eingeengt und destilliert.
Ausbeute: 408 g (66% d.Th.)
Siedepunkt: 73-78°C / 0,2 mm

**Beispiel XXI**

2-Cyclopentyl-2-(4-methylphenyl)essigsäure-tert.butylester

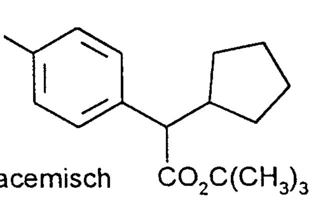

racemisch

33,5 g (0,3 mol) Kalium-tert.butylat werden in 100 ml wasserfreiem DMF bei 0°C vorgelegt, und 51,6 g (0,25 mol) der Verbindung aus Beispiel XX in 250 ml wasserfreiem DMF zugetropft. Es wird 30 min. bei 0°C gerührt und 32,2 ml (0,3 mol) Cyclopentylbromid in 150 ml wasserfreiem DMF bei 5-15°C zugetropft und 20 h bei 25°C gerührt. Nach Einengen wird der Rückstand zwischen Wasser und Diethylether verteilt, die Etherphase über Natriumsulfat getrocknet und eingeengt. Das Produkt kristallisiert aus.

Ausbeute: 67 g (97,5% d.Th.)
Festpunkt: 51-53°C
In Analogie zur Vorschrift des Beispiels XXI werden die Verbindungen der Tabelle II hergestellt:

**Tabelle II:**

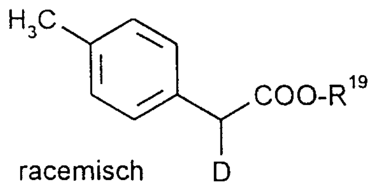

racemisch

| Bsp.-Nr. | D | $R^{19}$ | $R_f$ (Solvens) | Ausgangsmaterial * |
|---|---|---|---|---|
| XXII | cHex | tBu | 0,71 (I) | Bsp.-Nr. XX |
| XXIII | cHept | tBu | 0,32 (I) | Bsp.-Nr. XX |
| XXIV | iPr | $CH_3$ | 0,86 (Q) | Sigma |
| XXV | iBu | tBu | 0,84 (R) | Bsp.-Nr. XX |
| XXVI | cPent | $CH_3$ | 0,59 (H) | Sigma |
| XXVII | cHept | $CH_3$ | 0,57 (I) | Sigma |

**Beispiel XXVIII**

2-(4-Brommethyl-phenyl)-2-cyclopentyl-essigsäure-tert.butylester

racemisch

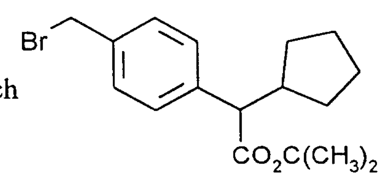

27,4 g (0,1 mol) der Verbindung aus Beispiel XXI werden in 200 ml Tetrachlormethan gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0°C abgekühlt und vom Succinimid abfiltiert. Nach Einengen des Filtrates fällt das Produkt aus. Es wird mit Petrolether (40/60) gewaschen und getrocknet.
Ausbeute: 20 g (57% d.Th.)
Fp.: 73-76°C
Die Verbindungen der Tabelle III werden analog der Vorschrift des Beispiels XXVIII hergestellt:

**Tabelle III:**

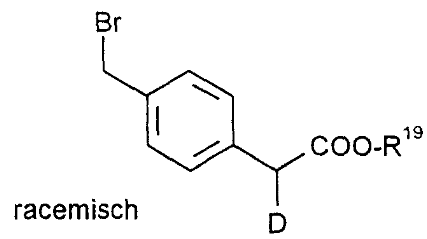

| Bsp.-Nr. | D | $R^{19}$ | $R_f$ (Solvens) | Ausgangsmaterial (Syn. aus Bsp.-Nr.) |
|---|---|---|---|---|
| XXIX | cHex | tBu | 0,58 (H) | Bsp.-Nr. XXII |
| XXX | cHept | tBu | 0,84 (M) | Bsp.-Nr. XXIII |
| XXXI | iPr | $CH_3$ | 0,78 (M) | Bsp.-Nr. XXIV |
| XXXII | iBu | tBu | 0,86 (M) | Bsp.-Nr. XXV |
| XXXIII | cPent | $CH_3$ | 0,63 (H) | Bsp.-Nr. XXVI |
| XXXIV | cHept | $CH_3$ | 0,59 (I) | Bsp.-Nr. XXVII |

**Beispiel XXXV**

2(R,S)-2-Cyclopentyl-2-[4-(2,4-dimethyl-α-carbolin-9-yl)methyl]phenyl-essigsäuretert.butylester

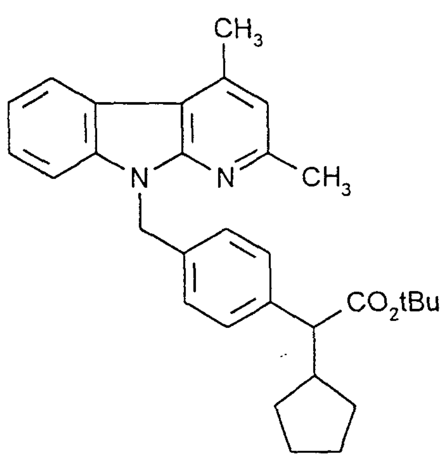

73,6 g (375 mmol) der Verbindung aus Beispiel XIX werden bei 25°C in 700 ml wasserfreiem N,N-Dimethylformamid mit 42,13 g (375 mmol) Kalium-tert.butanolat 30 min. umgesetzt und dann mit 161,7 g (375 mmol) der Verbindung aus Beispiel XXVIII, gelöst in 680 ml wasserfreiem N,N-Dimethylformamid, versetzt. Nach 1 h ist die Umsetzung beendet (DC-Kontrolle; Petrolether : Essigester = 10:1). Zur Aufarbeitung werden 2 l Pufferlösung (pH = 4 / Fa. Merck) und 2 l Wasser zugegeben, der anfallende Niederschlag abgesaugt, mit Wasser gewaschen und wieder scharf abgesaugt. Der mäßig feuchte Feststoff wird nun nacheinander mit Petrolether und Methanol verrührt und abgesaugt. Die Vakuumtrocknung über Phosphorpentoxid liefert 139,8 g (298 mmol / 79%) Produkt.
Schmp.: 160-161°C (unkorrigiert)
$R_f$ = 0,39 (Petrolether : Essigester = 10:1)
$^1$H-NMR ($CDCl_3$, 250 MHz, TMS): $\delta$ = 0,91 (M, 1H), 1,18-1,68 (M, 6H), 1,87 (M, 1H), 1,47 (S, 9H), 2,42 (M, 1H), 2,66 (S, 3H), 2,83 (S, 3H), 3,09 (D, 1H), 5,67 (S, 2H), 6,88 (S, 1H), 7,13-7,41 (M, 7H), 8,09 (D, 1H) ppm

Die Verbindungen der Tabellen IV und V werden analog der Vorschrift des Beispiels XXXV hergestellt:

**Tabelle IV:**

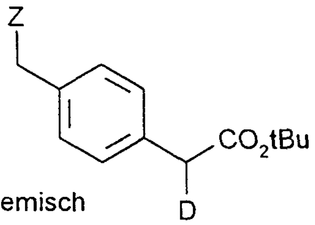

racemisch

| Bsp.-Nr. | Z | D | $R_f$ (Solvens) | Ausgangsmaterial Syn. aus Bsp.-Nr. |
|---|---|---|---|---|
| XXXVI | 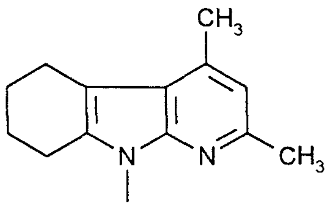 | cPent | 0,28 (H) | Benzylbromid: Bsp.-Nr. XXVIII Heterocyclus: Bsp.Nr. VI |
| XXXVII | 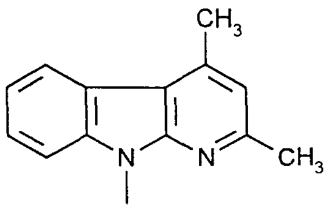 | cHept | 0,47 (H) | Benzylbromid: Bsp.-Nr. XXX Heterocyclus: Bsp.Nr. XIX |
| XXXVIII | 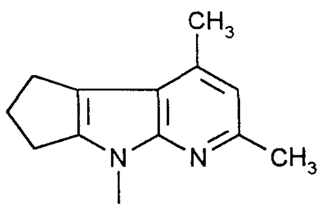 | cHept | 0,54 (L) | Benzylbromid: Bsp.-Nr. XXX Heterocyclus: Bsp.Nr. VII |
| XXXIX | 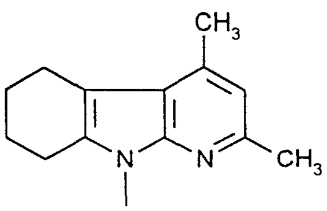 | cHept | 0,27 (H) | Benzylbromid: Bsp.-Nr. XXX Heterocyclus: Bsp.Nr. VI |
| XL | 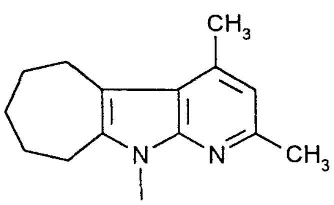 | cPent | 0,59 (D) | Benzylbromid: Bsp.-Nr. XXVIII Heterocyclus: Bsp.Nr. VIII |
| XLI | 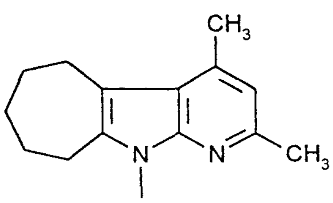 | cHept | 0,29 (H) | Benzylbromid: Bsp.-Nr. XXX Heterocyclus: Bsp.Nr. VIII |

| Bsp.-Nr. | Z | D | $R_f$ (Solvens) | Ausgangsmaterial Syn. aus Bsp.-Nr. |
|---|---|---|---|---|
| XLII | 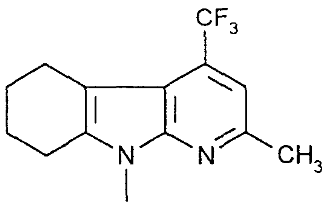 | cPent | 0,70 (M) | Benzylbromid: Bsp.-Nr. XXVIII Heterocyclus: Bsp.Nr. IX |
| XLIII | 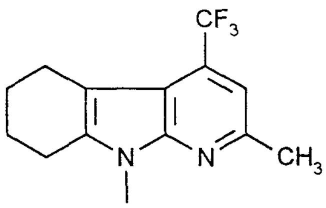 | cHept | 0,36 (H) | Benzylbromid: Bsp.-Nr. XXX Heterocyclus: Bsp.Nr. IX |
| XLIV | 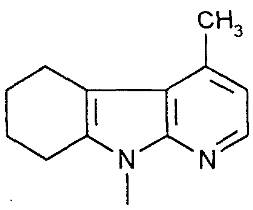 | cHept | 0,48 (L) | Benzylbromid: Bsp.-Nr. XXX |
| XLV | 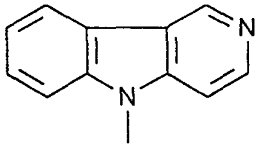 | cPent | 0,49 (C) | Benzylbromid: Bsp.-Nr. XXVIII |
| XLVI | 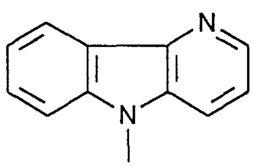 | cPent | 0,51 (C) | Benzylbromid: Bsp.-Nr. XXVIII |
| XLVII | 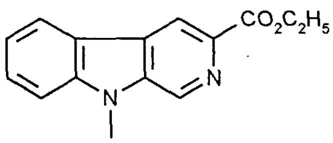 | cPent | 0,54 (C) | Benzylbromid: Bsp.-Nr. XXVIII |
| XLVIII | 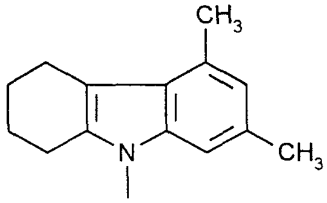 | cPent | 0,37 (N) | Benzylbromid: Bsp.-Nr. XXVIII Heterocyclus: Bsp.Nr. XI |
| IL | 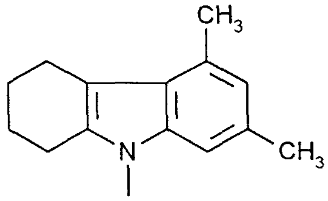 | cHept | 0,56 (H) | Benzylbromid: Bsp.-Nr. XXX Heterocyclus: Bsp.Nr. XI |

| Bsp.-Nr. | Z | D | $R_f$ (Solvens) | Ausgangsmaterial Syn. aus Bsp.-Nr. |
|---|---|---|---|---|
| L | 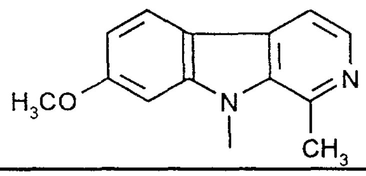 | cPent | 0,57 (C | Benzylbromid: Bsp.-Nr. XXVIII |
| LI | 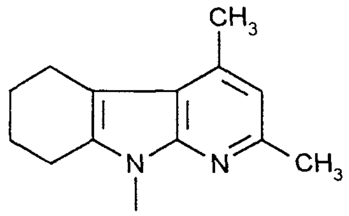 | cHex | 0,35 (H) | Benzylbromid: Bsp.-Nr. XXIX Heterocyclus: Bsp.Nr. VI |
| LII | 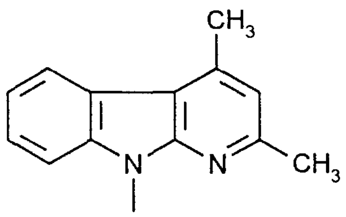 | cHex | 0,57 (B) | Benzylbromid: Bsp.-Nr. XXIX Heterocyclus: Bsp.Nr. XIX |
| LIII | 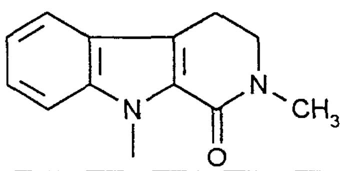 | cPent | Fp.= 189-190°C | Benzylbromid: Bsp.-Nr. XXVIII Heterocyclus: a) C. Herdeis et al., Heterocycles **22**, 2277 (1984). |
| LIV | 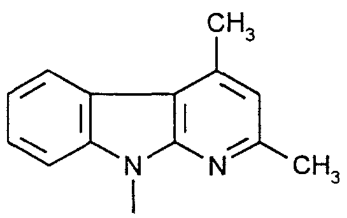 | iBu | 0,49 (M) Fp.: 142°C MS ($CI/NH_3$): 457 (100%) | Benzylbromid: c) Bsp.-Nr. XXXII Heterocyclus: c) Bsp.Nr. XIX |

**Tabelle V:**

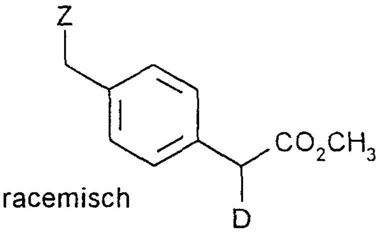

racemisch

| Bsp.-Nr. | Z | D | $R_f$ (Solvens) MS/Fp. | Ausgangsmaterial Syn. aus Bsp.-Nr. |
|---|---|---|---|---|
| LV | 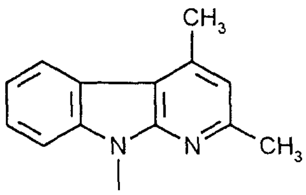 | iPr | 0,39 (M) Fp:= 159°C MS(CI/$NH_3$): 401 (100%) | Benzylbromid: Bsp.-Nr. XXXI Heterocyclus: Bsp.-Nr. XIX |
| LVI | 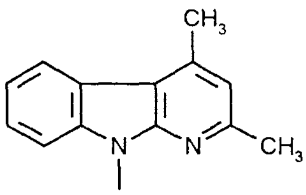 | cPent | 0,76 (B) | Benzylbromid: Bsp.-Nr. XXXIII Heterocyclus: Bsp.-Nr. XIX |
| LVII | 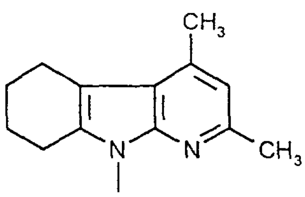 | cHept | 0,26 (H) | Benzylbromid: Bsp.-Nr. XXXIV Heterocyclus: Bsp.-Nr. VI |
| LVIII | 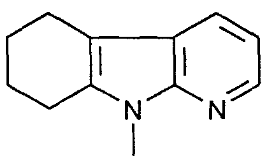 | cHept | 0,64 (K) | Benzylbromid: Bsp.-Nr. XXXIV |
| LIX | 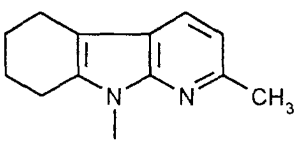 | cHept | 0,29 (H) | Benzylbromid: Bsp.-Nr. XXXIV Heterocyclus: Bsp.-Nr. X |
| LX | 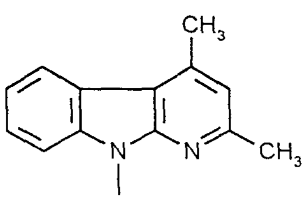 | cHept | 0,30 (H) | Benzylbromid: Bsp.-Nr. XXXIV Heterocyclus: Bsp.-Nr. XIX |

**Beispiel LXI**

2-(R,S)-2-Cyclopentyl-2-[4-(2,4-dimethyl-α-carbolin-9-yl)methyl]phenylessigsäure Hydrochlorid

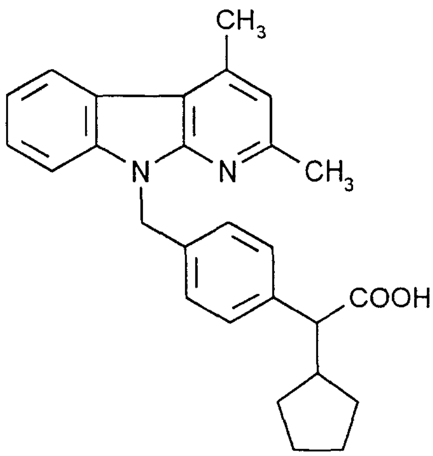

x HCl

139,8 g (298 mmol) der Verbindung aus Beispiel XXXV werden in 1 l 1,4-Dioxan gelöst und 3 h mit 240 ml konzentrierter Salzsäure (37%ig) bei 70°C gerührt. Nach beendeter Umsetzung (DC-Kontrolle; Petrolether : Essigester = 10:1) wird der Ansatz auf ca. 15°C gekühlt und dann portionsweise auf 5 l Wasser gegossen. Der pH-Wert wird mit 2 M wäßriger Natronlauge auf 2,8 gestellt, der anfallende Niederschlag über einen Papierfilter abgesaugt und mit Wasser so oft nachgewaschen, bis das Waschwasser einen pH > 4 besitzt. Der scharf abgesaugte Feststoff wird mit 1 l Petrolether (Siedebereich 60-80°C) verrührt, wiederum abgesaugt und im Vakuum über Phosphorpentoxid getrocknet.
Ausbeute: 130,3 g (290 mmol / 97%)
Schmp.: 260-262°C (unkorrigiert)
$R_f$ = 0,51 (Dichlormethan : Ethanol = 20:1)
$^1$H-NMR ($d_6$-DMSO, 200 MHz, TMS): δ = 0,88 (M, 1H), 1,09-1,67 (M, 6H), 1,79 (M, 1H), 2,38 (M, 1H), 2,68 (S, 3H), 2,84 (S, 3H), 3,16 (D, 1H), 4,7-5,9 (1H), 5,80 (S, 2H), 7,12-7,26 (M, 5H), 7,32 (M, 1H), 7,49 (M, 1H), 7,59 (D, 1H), 8,17 (D, 1H) ppm.

Die Verbindungen der Tabelle VI werden analog der Vorschrift des Beispiels LXI hergestellt:

**Tabelle VI:**

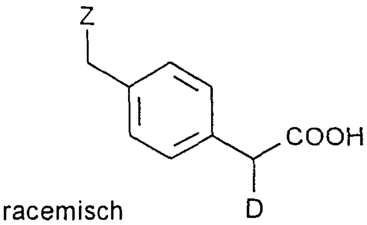

racemisch

| Bsp.-Nr. | Z | D | $R_f$ (Solvens) | Ausgangsmaterial (Syn. aus Bsp.-Nr.) |
|---|---|---|---|---|
| LXII | 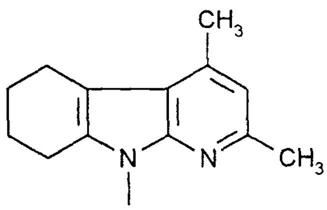 | cPent | 0,37 (A) | Bsp.-Nr. XXXVI |
| LXIII | 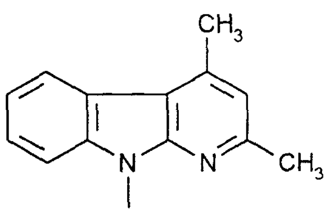 | cHept | 0,23 (G) | Bsp.-Nr. XXXVII |
| LXIV | 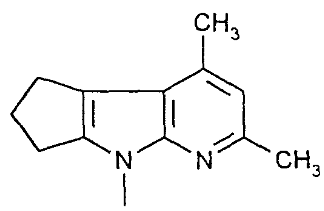 | cHept | 0,30 (E) | Bsp.-Nr. XXXVIII |
| LXV | 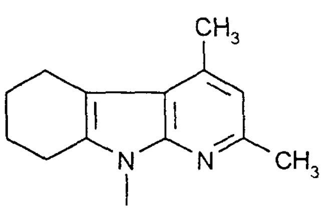 | cHept | 0,27 (D) | Bsp.-Nr. XXXIX |

| Bsp.-Nr. | Z | D | $R_f$ (Solvens) | Ausgangsmaterial (Syn. aus Bsp.-Nr.) |
|---|---|---|---|---|
| LXVI | 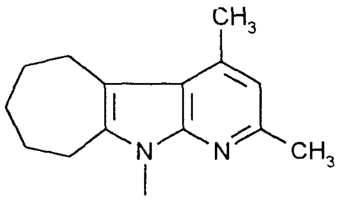 | cPent | 0,37 (C) | Bsp.-Nr. XL |
| LXVII | 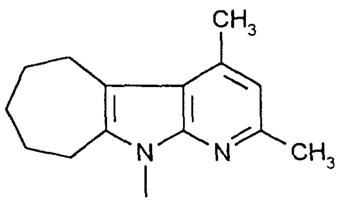 | cHept | 0,15 (C) | Bsp.-Nr. XLI |
| LXVIII | 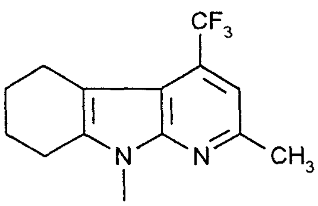 | cPent | 0,43 (A) | Bsp.-Nr. XLII |
| LXIX | 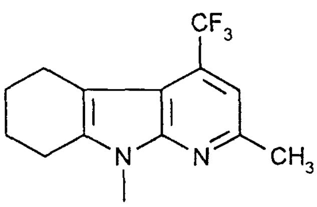 | cHept | 0,27 (C) | Bsp.-Nr. XLIII |
| LXX | 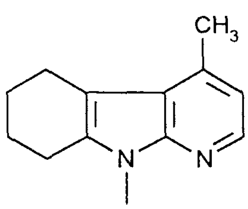 | cHept | 0,17 (E) | Bsp.-Nr. XLIV |

| **Bsp.-Nr.** | **Z** | **D** | **$R_f$ (Solvens)** | **Ausgangsmaterial (Syn. aus Bsp.-Nr.)** |
|---|---|---|---|---|
| LXXI | 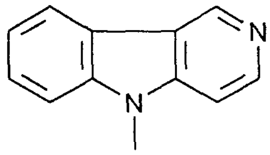 | cPent | 0,07 (C) | Bsp.-Nr. XLV |
| LXXII | 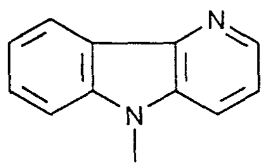 | cPent | 0,26 (C) | Bsp.-Nr. XLVI |
| LXXIII | 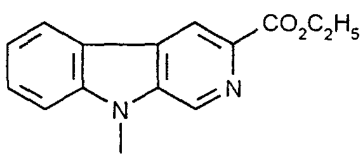 | cPent | 0,39 (C) | Bsp.-Nr. XLVII |
| LXXIV | 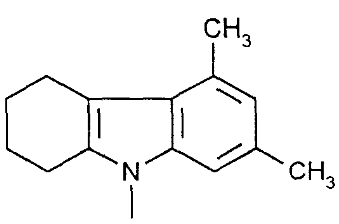 | cPent | 0,46 (C) | Bsp.-Nr. XLVIII |

| Bsp.-Nr. | Z | D | $R_f$ (Solvens) | Ausgangsmaterial (Syn. aus Bsp.-Nr.) |
|---|---|---|---|---|
| LXXV | 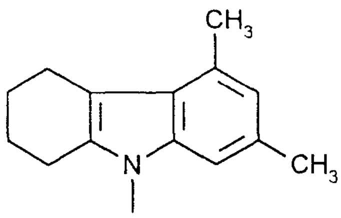 | cHept | 0,68 (E) | Bsp.-Nr. IL |
| LXXVI | 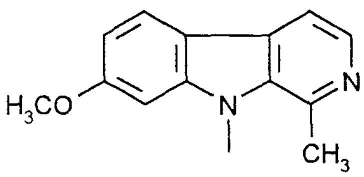 | cPent | 0,44 (C) | Bsp.-Nr. L |
| LXXVII | 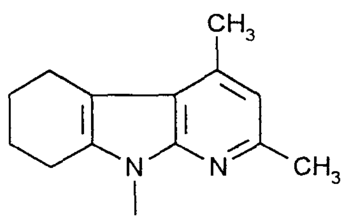 | cHex | 0,44 (C) | Bsp.-Nr. LI |
| LXXVIII | 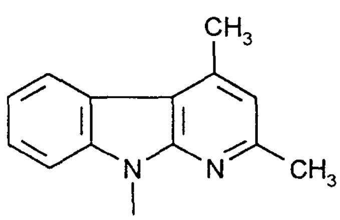 | cHex | 0,55 (C) | Bsp.-Nr. LII |
| LXXIX | 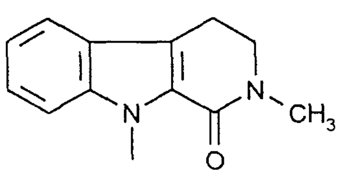 | cPent | Fp. 204-205°C | Bsp.-Nr. LIII |

| Bsp.-Nr. | Z | D | $R_f$ (Solvens) | Ausgangsmaterial (Syn. aus Bsp.-Nr.) |
|---|---|---|---|---|
| LXXX | 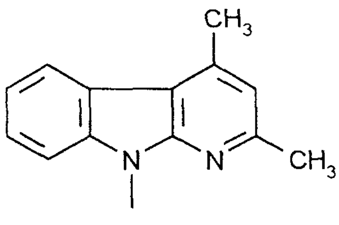 | iBu | 0,36 (A)<br>Fp.: 156°C<br>MS(FAB):<br>401(100%)<br>154 (90%) | Bsp.-Nr. LIV |

**Beispiel LXXXI**

2-(R,S)-2-[4-(2-Methyl-5,6,7,8-tetrahydro-α-carbolin-9-yl)-methyl-phenyl]-2-cycloheptyl-essigsäure

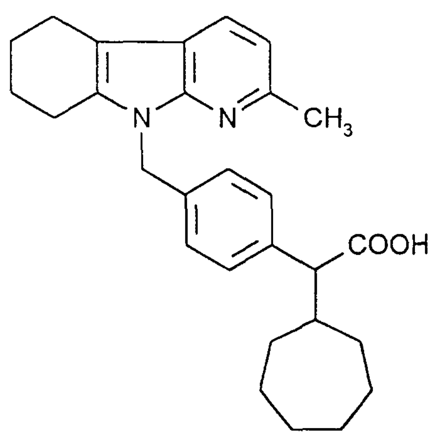

1,5 g (3,37 mmol) der Verbindung aus Bespiel LIX werden 48 h mit 20 ml 1 M methanolischer Natronlauge umgesetzt. Darauf wird Wasser zugesetzt und der Methanolanteil abgedampft. Die alkalische wäßrige Phase wird mehrfach mit Ether extrahiert, im Vakuum von Resten organischen Lösemittels befreit und bei 0° - 5°C mit wäßriger 1 M Salzsäure auf einen pH-Wert von ca. 2 gestellt. Der dabei ausfallende Niederschlag wird abgesaugt, gründlich mit Wasser nachgewaschen und im Hochvakuum über Phosphorpentoxid getrocknet.
Ausbeute: 1,18 g
Unter Verwendung von Kaliumhydroxid an Stelle von Natriumhydroxid und unter Zusatz von 1,4,7,10,13,16-Hexaoxacyclooctadecan läßt sich die Reaktion beschleunigen.
$R_f$ = 0,39 (Petrolether : Essigsäureethylester = 2:1)
In Analogie zur Vorschrift des Beispiels LXXXI werden die Verbindungen der Tabelle VII hergestellt:

**Tabelle VII:**

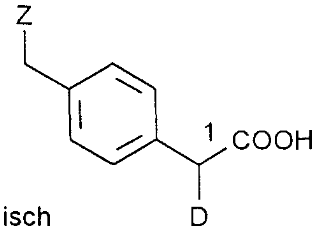

racemisch

| **Bsp.-Nr.** | **Z** | **D** | **1** | **$R_f$ (Solvens)** MS/Fp. | **Ausgangsmaterial (Synthese aus Bsp.-Nr.)** |
|---|---|---|---|---|---|
| LXXXII Methode 1 | 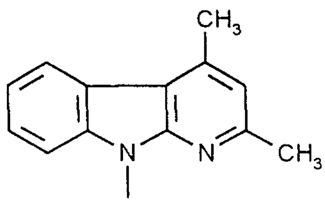 | iPr | rac | 0,28 (A) Fp.=225°C MS(FAB): 387 (100%), 154 (80%) | Bsp.-Nr. LV |
| LXXXIII | 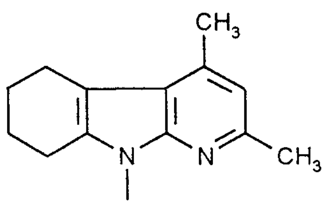 | cHept | rac | 0,05 (L) | Bsp.-Nr. LVII |
| LXXXIV | 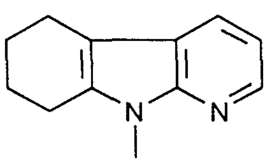 | cHept | rac | 0,11 (K) | Bsp.-Nr. LVIII |
| LXXXV | 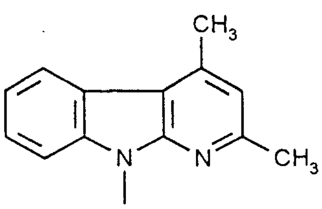 | cHept | rac | 0,23 (G) | Bsp.-Nr. LX |
| LXXXVI | 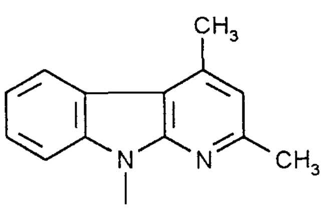 | cPent | rac | 0,51 (C) | Bsp.-Nr. LVI |

Das Beispiel LXXXII kann auch nach folgender Methode 2 hergestellt werden:

2-[4-(2,4-Dimethyl-α-carbolin-9-yl)-methyl-phenyl]-2-(prop-2-yl)-essigsäure

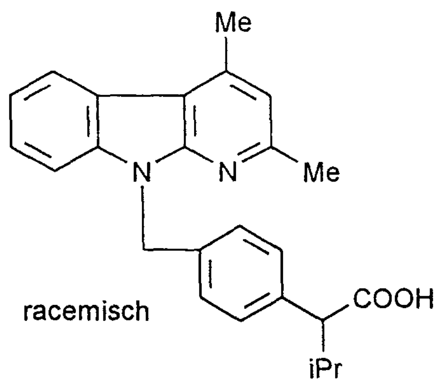

1,11 g (2,77 mmol) der Verbindung aus Beispiel Nr. LV werden in 45 ml Methanol und 3 ml 2 M wäßriger Natronlauge 18 Stunden unter Rückfluß gekocht. Da die Umsetzung laut DC (Dichlormethan : Methanol = 20 : 1) unvollständig ist, werden 30 ml Tetrahydrofuran und weitere 3 ml 2 M wäßriger Natronlauge zugegeben, wobei eine klare Lösung erhalten wird. Nach vierstündigem Kochem unter Rückfluß ist die Reaktion (DC, s.o.) beendet. Der Ansatz wird abgekühlt, mit Wasser verdünnt und mit 2 M wäßriger Salzsäure neutralisiert. Der dabei anfallende Niederschalg wird abgesaugt, mit Wasser gewaschen und im Vakuum über Phosphorpentoxid getrocknet.
Ausbeute: 0,597 g.
Fp. = 225°C
$R_f$ = 0,28 (Dichlormethan : Methanol = 20 : 1)

Die Verbindungen der Tabelle VIII werden analog der Vorschrift des Beispiels XXXV hergestellt:

## Tabelle VIII:

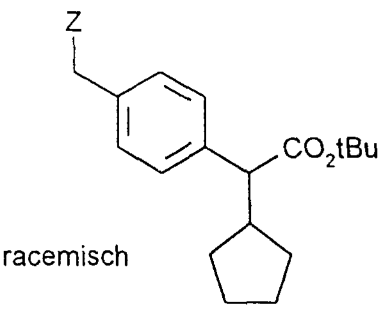

racemisch

| Bsp.-Nr. | -Z | Fp. (°C) | Ausgangsmaterial * |
|---|---|---|---|
| LXXXVII | 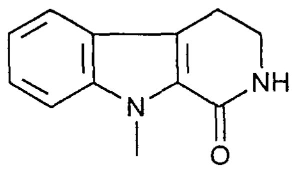 | 164-165 | |
| LXXXVIII | 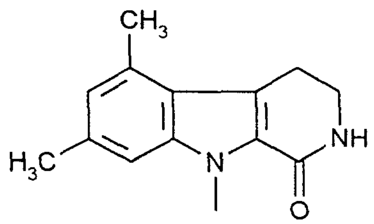 | 201-202 | |

* Als Benzylbromid wurde Bsp.-Nr. XXVIII eingesetzt.

Die Verbindungen der Tabelle IX werden analog der Vorschrift des Beispiels LXI hergestellt:

**Tabelle IX:**

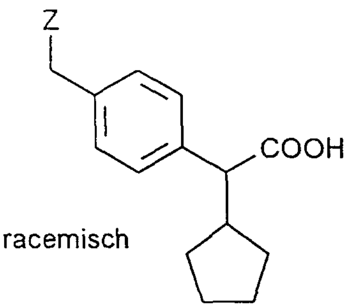

racemisch

| Bsp.-Nr. | -Z | Fp. (°C) | Ausgangsmaterial aus Bsp.-Nr. |
|---|---|---|---|
| LXXXIX | 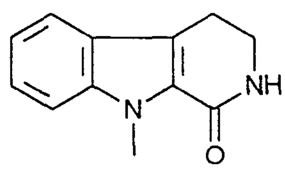 | 262-263 | LXXXVII |
| XC | 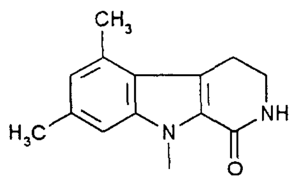 | 279-280 | LXXXVIII |

**Beispiel XCI**

2-Hydrazino-5-trifluormethylpyridin

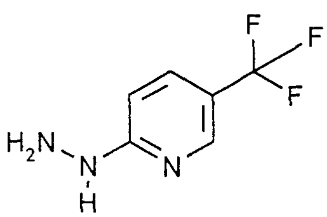

In Analogie zur Vorschrift von Beispiel Nr. IV wird 2-Hydrazino-5-trifluormethylpyridin aus 2-Chlor-5-trifluormethylpyridin hergestellt.
$R_f$ = 0,37 (BABA)

**Beispiel XCII**

5-Oxo-5,6,7-tetrahydro-α-carbolin

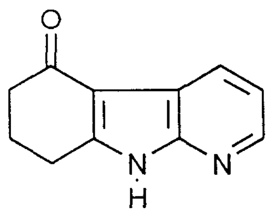

3,3 g (19,2 mmol) 5,6,7,8-Tetrahydro-α-carbolin (Lit.: S. Okuda und M.M Robinson, J. Am. Chem. Soc. 81, 740 (1959)) werden in 43 ml Tetrahydrofuran unter Rühren bei 0°C vorgelegt und tropfenweise mit einer Lösung von 15,5 g (68,2 mmol) DDQ in 277 ml Tetrahydrofuran und 31 ml Wasser versetzt. Die Reaktionsmischung wird 5 Minuten bei 0°C und 2 Stunden bei 20°C nachgerührt, dann mit einem Puffer von pH = 10 (Merck) versetzt und mit Diethylether extrahiert. Die eingedampfte organische Phase liefert ein Rohprodukt, das chromatographisch gereinigt wird (Kieselgel 60, Merck, zuerst Petrolether : Essigsäureethylester = 1 : 1 / dann Dichlormethan : Methanol = 20 : 1). Die so erhaltenen Fraktionen werden mit Aceton ausgerührt, abgesaugt und im Vakuum vom Lösemittel befreit.
Ausbeute: 0,92 g
$R_f$ = 0,17 (Petrolether : Essigsäureethylester = 1 : 4).

Die Verbindungen der Tabelle X werden analog der Vorschrift von Beispiel No. VI hergestellt:

**Tabelle X:**

| Bsp.-Nr. | -Z- | Fp. (°C) Rf (Solvens) | MS (EI) | Ausgangsmaterial aus Bsp.-Nr. |
|---|---|---|---|---|
| XCIII | 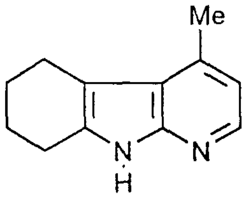 | 0,27 (E) | | V |
| XCIV | 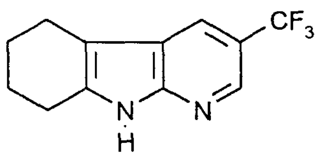 | 0,46 (G) | 240 (52%) 212 (100%) | XCI |

Die Verbindungen der Tabelle XI werden analog der Vorschrift von Beispiel Nr. XIX hergestellt.

**Tabelle XI:**

| Bsp.-Nr. | -Z- | Fp. [°C] $R_f$ (Solvens) | MS (EI) | Ausgangsmaterial aus Bsp.-Nr. |
|---|---|---|---|---|
| XCV | 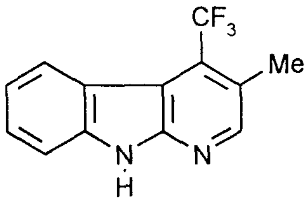 | 0,39 (G) | 250 (100%) | IX |
| XCVI | 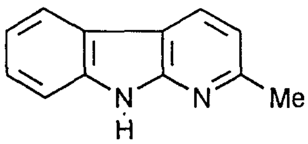 | 0,45 (G) | | X |
| XCVII | 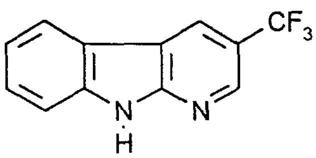 | 0,48 (G) | 236 (100%) | XCIV |
| XCVIII | 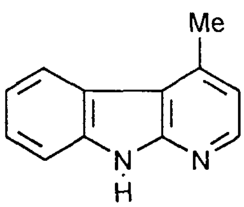 | 0,3 (E) | | XCIII |

Die Verbindungen der Tabelle XII werden analog der Vorschrift von Beispiel Nr. XXXV hergestellt:

**Tabelle XII:**

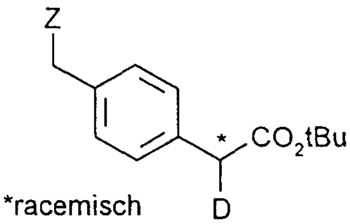

*racemisch

| Bsp.-Nr. | Z | D | Fp.[°C] $R_f$ (Solvens) | Ausgangsmaterial aus Bsp.-Nr. |
|---|---|---|---|---|
| IC | 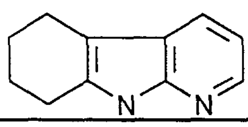 | cPent | 0,73 (C) | Benzylbromid: Bsp.-Nr. XXVIII |
| C | 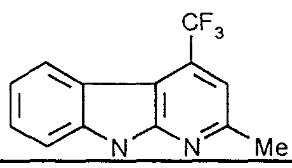 | cPent | 0,63 (H) | Benzylbromid: Bsp.-Nr. XXVIII Heterocyclus: Bsp.-Nr. XCV |
| CI | 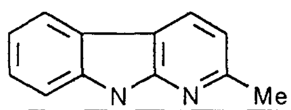 | cPent | 0,27 (H) | Benzylbromid: Bsp.-Nr. XXVIII heterocyclus: Bsp.-Nr. XCVI |
| CII | 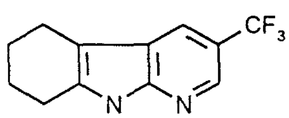 | cPent | 0,33 (H) | Benzylbromid: Bsp.-Nr. XXVIII Heterocyclus: Bsp.-Nr. XCI |
| CIII | 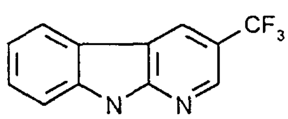 | cPent | 0,41 (H) | Benzylbromid: Bsp.-Nr. XXVIII Heterocyclus: Bsp.-Nr. XCVII |
| CIV | 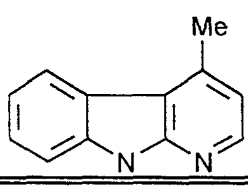 | cPent | 0,18 (H) | Benzylbromid: Bsp.-Nr. XXVIII Heterocyclus: Bsp.-Nr. XCVIII |

Die Verbindungen der Tabelle XIII werden analog der Vorschrift von Beispiel Nr. LXI hergestellt:

**Tabelle XIII**:

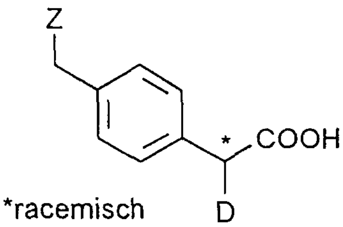

*racemisch

| Bsp.-Nr. | Z | D | Fp.[0°]<br>$R_f$ (Solvens) | Ausgangsmaterial aus Bsp.-Nr. |
|---|---|---|---|---|
| CV | 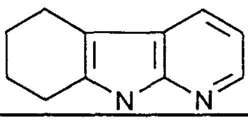 | cPent | 0,27 (C) | IC |
| CVI | 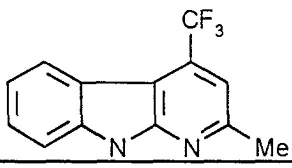 | cPent | 0,49 (C) | C |
| CVII | 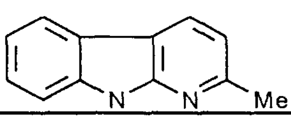 | cPent | 0,38 (C) | CI |
| CVIII | 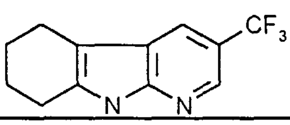 | cPent | 0,35 (C) | CII |
| CIX | 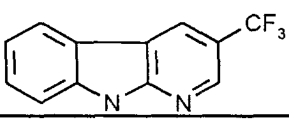 | cPent | 0,43 (C) | CIII |
| CX | 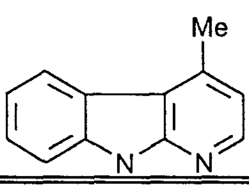 | cPent | 0,29 (C) | CIV |

**Beispiel Nr. CXI**

1-(R,S)-1-Phenyl-2-triphenylmethyloxy-ethanol

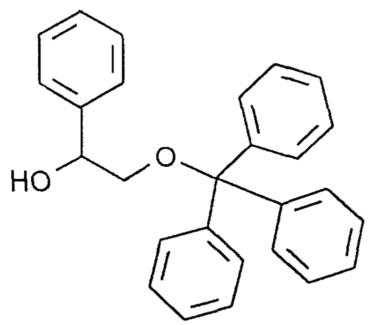

13 g (94 mmol) 1-(R,S)-1-Phenyl-2-hydroxy-ethanol werden bei 20°C mit 15,6 ml (113 mmol) Triethylamin und 23,6 g (84,6 mmol) Triphenylmethylchlorid in 200 ml DMF umgesetzt. Nach 20 h gießt man auf Puffer von pH = 4 (Merck), trennt die Phasen, trocknet die organische Phase mit Magnesiumsulfat und dampft zur Trockne ein. Das Rohprodukt wird chromatographisch an Kieselgel 60 (Merck/Petrolether : Essigsäureethylester = 20 : 1 später 10 : 1) gereinigt; Ausbeute: 27 g.
$R_f$ = 0,36 (Petrolether : Essigsäureethylester = 5: 1)

**Beispiel Nr. CXII**

6-Chlor-5-methyl-3-nitro-2-(2-oxo-cyclohexyl)-pyridin

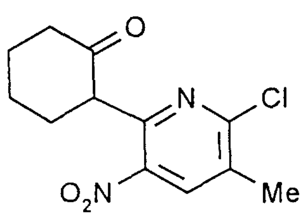

20 g (95,7 mmol) 2,6-Dichlor-5-methyl-3-nitro-pyridin werden unter Argon als Schutzgas bei 20°C in 200 ml DMF mit 13,3 ml (95,7 mmol) Triethylamin und 14,5 g (95,7 mmol) frisch destilliertem 1-Pyrrolidino-cyclopenten umgesetzt. Nachdem laut Dünnschichtchromatographie (Kieselgel/Petrolether : Essigsäureethylester = 4 : 1) das Ausgangsmaterial verschwunden ist, werden 200 ml 1 M Salzsäure zugegeben und mit ca. 600 ml Wasser verdünnt. Der ausfallende Niederschlag wird abgesaugt, im Hochvakuum über Phosphorpentoxid getrocknet und chromatographisch (Kieselgel 60 / Merck / Petrolether : Essigsäureethylester = 2 : 1) gereinigt.
$R_f$ = 0,69 (Petrolether : Essigsäureethylester = 4 : 1)

**Beispiel Nr. CXIII**

2-Methyl-5,6,7,8-tetrahydro-δ-carbolin

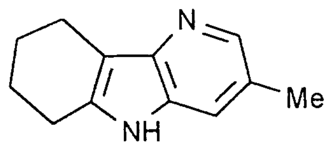

2,8 g (10,4 mmol) der Verbindung aus Beispiel Nr. CXII werden in 30 ml THF an 0,5 g Palladium (5 %) / Kohle unter 3 bar Wasserstoffdruck 18 h umgesetzt. Darauf wird der Katalysator abgesaugt und mehrfach mit Methanol und Dichlormethan gewaschen. Das Filtrat wird eingedampft und im Hochvakuum getrocknet; Ausbeute: 2,1 g.
$R_f$ = 0,53 (Dichlormethan : Ethanol = 5 : 1)

**Beispiel Nr. CXIV**

3-Methyl-5,6,7,8-tetrahydro-α-carbolin Hydrochlorid

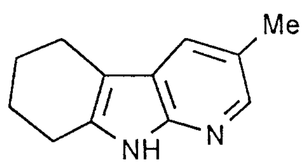

13,0 g (120,2 mmol) 2-Amino-5-methyl-pyridin werden in 150 ml Ethanol gelöst mit 60 ml 2 M Salzsäure verrührt, zur Trockne eingedampft und zuletzt im Hochvakuum über Natriumhydroxid und Phosphorpentoxid getrocknet. Das so erhaltene Produkt wird in 120 ml 1,2-Dichlorbenzol mit 2,2 g (20,1 mmol) 2-Amino-5-methyl-pyridin und 11,4 g (50,0 mmol) 2-Hydroxy-cyclohexanon-Dimer 6 h unter Rückfluß am Wasserabscheider gekocht. Darauf setzt man wiederum 11,4 g (50,0 mmol) 2-Hydroxy-cyclohexanon-Dimer zu und kocht weitere 3 h unter Rückfluß. Beim Abkühlen fällt bei 20°C ein Niederschlag aus. Man setzt 150 ml Aceton zu, kühlt unter Rühren auf 0° bis 5°C, saugt den Niederschlag ab und wäscht mit kaltem Ether nach. Das erhaltene Produkt wird im Hochvakuum über Phosphorpentoxid getrocknet; Ausbeute 18 g.
$R_f$ = 0,29 (Dichlormethan : Ethanol = 20 : 1)

In Analogie zur Vorschrift des Beispiels Nr. XIX werden die Verbindungen der folgenden Tabelle XIV erhalten:

**Tabelle XIV**

| Besp.-Nr. | Heterocyclus | $R_f$ (Solvens) | Ausgangs-material |
|---|---|---|---|
| CXV | 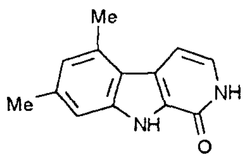 | 0,16 (C) | |
| CXVI | 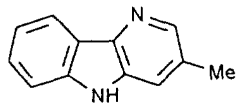 | 0,37 (C) | Bsp.-Nr. CXIII |
| CXVII | 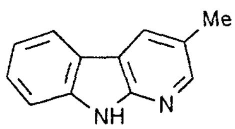 | 0, 17 (D) | Bsp.-Nr. CXIV |

**Beispiel Nr. CXVIII**

1-Chlor-5,7-dimethyl-β-carbolin

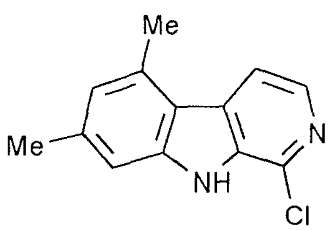

10,2 g (49 mmol) der Verbindung aus Beispiel Nr. CXV werden mit 222 ml (2,4 mol) Phosphoroxychlorid und 155 µl N,N-Dimethyl-anilin 24 h bei 125°C umgesetzt. Der Ansatz wird nach dem Abkühlen auf 1 l Eiswasser gegossen, darauf mit wäßriger Natriumcarbonatlösung neutralisiert und mehrfach mit Essigsäureethylester extrahiert. Die organische Phase wird mit Magnesiumsulfat getrocknet, eingedampft und im Hochvakuum vom Restlösemittel befreit. Das Rohprodukt wird an Kieselgel 60 (Merck / Dichlormethan) chromatographisch gereinigt;
Ausbeute: 4,3 g.
$R_f$ = 0,39 (Dichlormethan : Ethanol = 20 : 1)

**Beispiel Nr. CXIX**

5,7-Dimethyl-β-carbolin

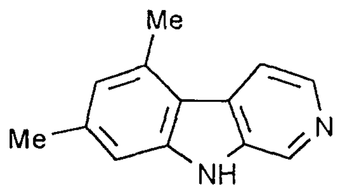

3,8 g (16,5 mmol) der Verbindung aus Beispiel Nr. CXVIII werden 10 d in 40 ml THF mit 1,3 g Natriumhydrogencarbonat an 700 mg Palladium (10 %) / Kohle bei ca. 3 bar Wasserstoffdruck und 20°C umgesetzt, wobei an jedem zweiten Tag 300 mg Palladium (10 %) / Kohle und 5 ml Methanol zugegeben werden. Darauf wird der Katalysator über Kieselgur abgesaugt, mit THF nachgewaschen, in Methanol und Dichlormethan ausgekocht und wiederum abgesaugt. Die vereinigten organischen Lösungen werden eingedampft, der Rückstand mit Ether ausgerührt und abgesaugt. Nach Vakuumtrocknung fallen 3 g Produkt an.
$R_f$ = 0,13 (Dichlormethan : Ethanol = 20 : 1)

**Beispiel Nr. CXX**

5,6-Dimethyl-1-(pyrid-2-yl)-1H-benzotriazol

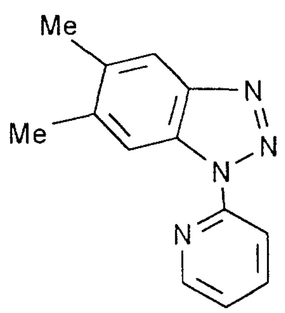

14,85 g (103 mmol) 5,6-Dimethyl-1H-benzotriazol werden in 150 ml wasserfreiem DMSO gelöst, mit 5 g (104 mmol) 50 %igem Natriumhydrid (+40 % Paraffinöl) bei 20°C bis zur Beendigung der Wasserstoffentwicklung umgesetzt, mit 10 g (103 mmol) 2-Fluor-pyridin versetzt und 18 h unter Rückfluß gekocht. Nach Abkühlen auf 20°C wird mit Wasser auf ca. 1 l Volumen aufgefüllt, der entstehende Niederschlag abgesaugt und mit Wasser gewaschen. Die im Hochvakuum über Phosphorpentoxid getrocknete Substanz wird chromatographisch an Kieselgel 60 (Merck / Dichlormethan bis Dichlormethan : Ethanol = 100 : 1) aufgereinigt; Ausbeute: 10,6 g.
$R_f$ = 0,38 (Dichlormethan : Ethanol = 50 : 1)

**Beispiel Nr. CXXI**

6,7-Dimethyl-α-carbolin

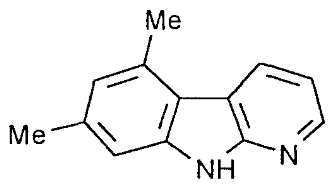

8,9 g (39,7 mmol) der Verbindung aus Beispiel Nr. CXX werden in 140 g Polyphosphorsäure unter Argon langsam auf 165°C erwärmt, wobei schon vor Verschwinden des Ausgangsmittel (DC-Kontrolle/Dichlormethan:Ethanol:20:1) gießt man auf 1,5 l Wasser und stellt mit 1 M wäßriger Natronlauge auf pH = 6 - 7. Der anfallende Niederschlag wird abgesaugt, mit Wasser gewaschen, scharf abgesaugt, darauf mit Petrolether gewaschen und wiederum abgesaugt. Nach Vakuumtrocknung fallen 1,8 g Produkt an.
$R_f$ = 0,32 (Dichlormethan : Ethanol = 20 : 1)
In Analogie zur Vorschrift von Beispiel Nr. XXI werden die Verbindungen in der Tabelle XV hergestellt:

## Tabelle XV

| Bsp.-Nr. | | $R_f$ (Solvens |
|---|---|---|
| CXXII | 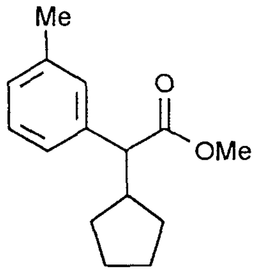 | 0,56 (H) |
| CXXIII | 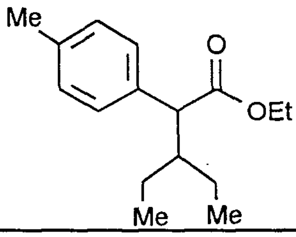 | |
| CXXIV | 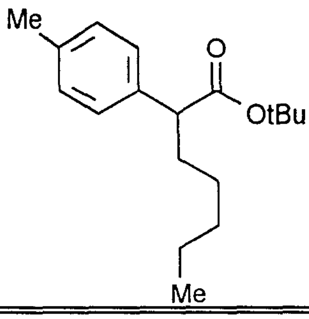 | |

In Analogie zur Vorschrift von Beispiel Nr. XXVIII werden die Verbindungen in der Tabelle XVI hergestellt:

**Tabelle XVI**

| Bsp.-Nr. | | $R_f$ (Solvens) | Ausgangs-Material (Bsp.-Nr.) |
|---|---|---|---|
| CXXV | 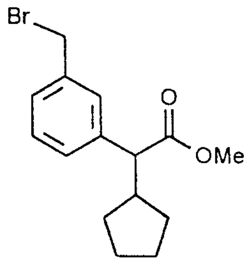 | 0,40 (H) | CXXII |
| CXXVI | 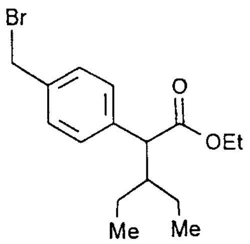 | | CXXIII |
| CXXVII | 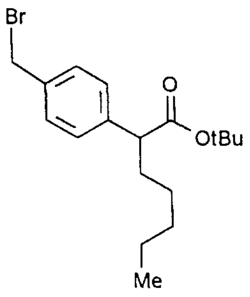 | | CXXIV |

Die Verbindungen der Tabelle XVII werden analog der Vorschrift von Beispiel Nr. XXXV hergestellt:

**Tabelle XVII**

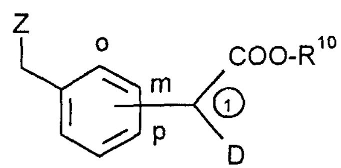

| Bsp.-Nr. | Z | Position (o,m oder p) | ① | D | $R^{10}$ | $R_f$(Solvens) | MS | Ausgangs-material Bsp.-Nr. |
|---|---|---|---|---|---|---|---|---|
| CXXIX | 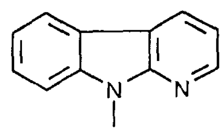 | p | rac | cPent | Me | 0,51 (D) | | XXXIII |

| Bsp.-Nr. | Z | Position (o,m oder p) | ① | D | $R^{10}$ | $R_f$(Solvens) | MS | Ausgangs-material Bsp.-Nr. |
|---|---|---|---|---|---|---|---|---|
| CXXX | 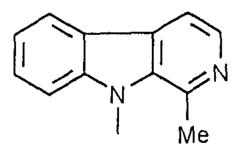 | p | rac | cPent | Me | 0,22 (C) | | XXXIII (Harman ist bei Aldrich käuflich). |
| CXXXI | 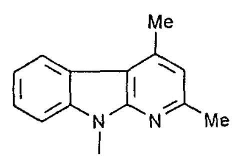 | m | rac | cPent | Me | 0,55 (D) | | XIX und CXXV |
| CXXXII | 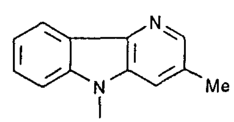 | p | rac | cPent | Me | 0,21 (D) | | CXVI und XXXIII |
| CXXXIII | 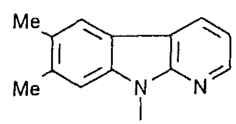 | p | rac | cPent | tBu | | | XXVIII und CXXI |

| Bsp.-Nr. | Z | Position (o,m oder p) | ① | D | $R^{10}$ | $R_f$(Solvens) | MS | Ausgangs-material Bsp.-Nr. |
|---|---|---|---|---|---|---|---|---|
| CXXXIV | 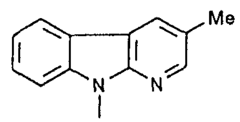 | p | rac | cPent | tBu | | | XXVIII und CXVII |
| CXXXV | 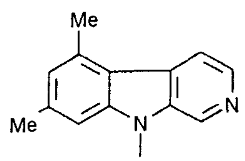 | p | rac | cPent | tBu | | | XXVIII und CXIX |
| CXXXVI | 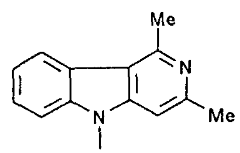 | p | rac | cPent | Me | 0,13 (L) | | XXXIII |
| CXXXVII | 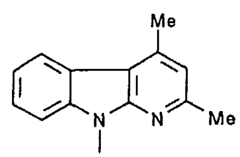 | p | rac | Me | tBu | 0,43 (L) | | XIX |

| Bsp.-Nr. | Z | Position (o,m oder p) | ① | D | $R^{10}$ | $R_f$(Solvens) | MS | Ausgangsmaterial Bsp.-Nr. |
|---|---|---|---|---|---|---|---|---|
| CXXXVIII | 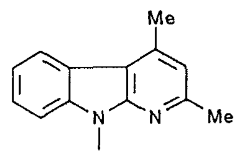 | p | rac | Et | tBu | 0,51 (L) | | XIX |
| CXXXIX | 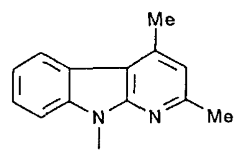 | p | rac | nPent | Et | | | XIX und CXXVII |
| CXL | 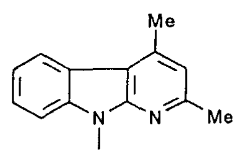 | p | rac | 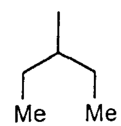 | Et | | | XIX und CXXVI |

Die Verbindungen der Tabelle XVIII werden analog der Vorschrift von Beispiel Nr. LXI, LXXXI, LXXXII (2. Methode) oder 73 hergestellt:

**Tabelle XVIII**

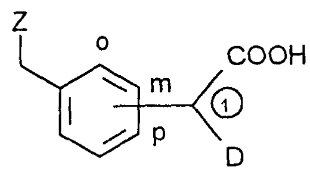

| Bsp.-Nr. | Z | Position (o,m oder p) | ① | D | $R_f$(Solvens) | MS | Ausgangs-material Bsp.-Nr. | Herstellung analog Bsp.-Nr. |
|---|---|---|---|---|---|---|---|---|
| CXLII | 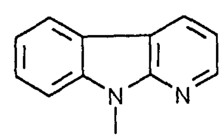 | p | rac | cPent | 0,14 (G) | | CXXIX | LXXXI |

| Bsp.-Nr. | Z | Position (o,m oder p) | ① | D | $R_f$(Solvens) | MS | Ausgangs-material Bsp.-Nr. | Herstellung analog Bsp.-Nr. |
|---|---|---|---|---|---|---|---|---|
| CXLIII | 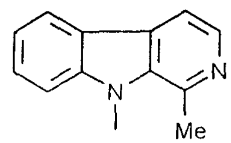 | p | rac | cPent | 0,50 (U) | | CXXX | LXXXI |
| CXLIV | 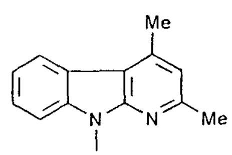 | m | rac | cPent | 0,14 (D) | | CXXXI | LXXXI |
| CVL | 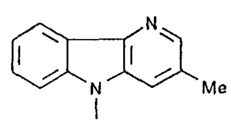 | p | rac | cPent | 0,10 (D) | | CXXXII | LXXXI |
| CVLI | 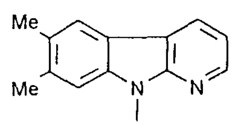 | p | rac | cPent | 0,34 (C) | | CXXXIII | LXI |

| Bsp.-Nr. | Z | Position (o,m oder p) | ① | D | $R_f$(Solvens) | MS | Ausgangs-material Bsp.-Nr. | Herstellung analog Bsp.-Nr. |
|---|---|---|---|---|---|---|---|---|
| CVLII | 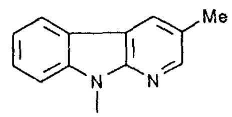 | p | rac | cPent | | | CXXXIV | LXI |
| CVLIII | 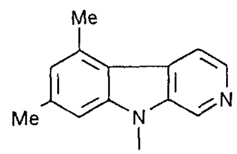 | p | rac | cPent | 0,15 (C) | | CXXXV | LXI |
| CIL | 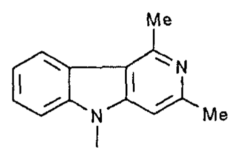 | p | rac | cPent | | | CXXXVI | LXXXI |
| CL | 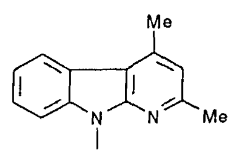 | p | rac | Et | | | CXXXVIII | LXI |

| Bsp.-Nr. | Z | Position (o,m oder p) | ① | D | $R_f$(Solvens) | MS | Ausgangs-material Bsp.-Nr. | Herstellung analog Bsp.-Nr. |
|---|---|---|---|---|---|---|---|---|
| CLI | 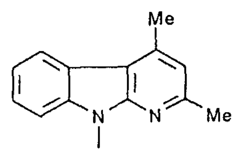 | p | rac | Me | | | CXXXVII | LXI |
| CLII | 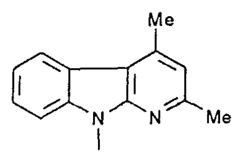 | p | rac | nPent | | | CXXXIX | LXXXI |
| CLIII | 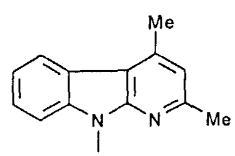 | p | rac | 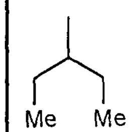 | | | CXL | LXXXI |

**Herstellungsbeispiele**

**Beispiele 1, 2 und 3**

2-(S)- und 2-(R)-2-[4-(2,4-Dimethyl-5,6,7,8-tetrahydro-α-carbolin-9-yl)-methylphenyl]-2-cyclopentyl-essigsäure-N-[(R)-phenylglycinolamid]

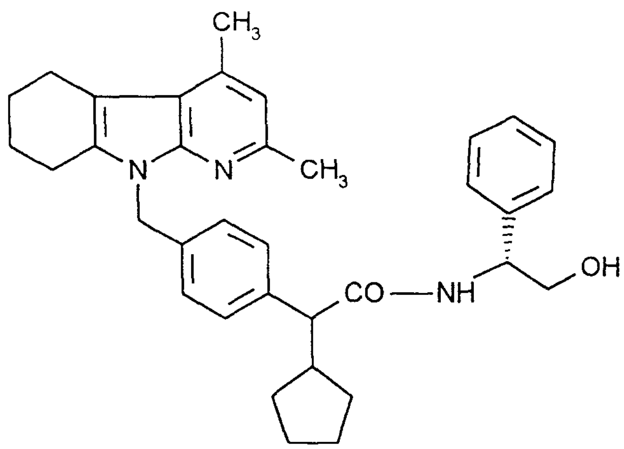

3,00 g (7,2 mmol) der Verbindung aus Beispiel LXII werden mit 0,99 g (7,2 mmol) (R)-Phenylglycinol (Aldrich) in 70 ml Dichlormethan gelöst, bei 0°C nacheinander mit 1,07 g (7,9 mmol) 1-Hydroxy-1H-benzotriazol-Hydrat (Aldrich), 1,58 g (8,3 mmol) N'-(3-Dimethylaminopropyl)-N-ethylcarbodiimid Hydrochlorid (Aldrich) und 2 ml Triethylamin versetzt und darauf 20 Stunden bei Raumtemperatur nachgerührt. Die organische Lösung wird mit wäßriger Ammoniumchloridlösung, mit wäßriger Natriumhydrogencarbonatlösung und mit einem Puffer von pH = 4 (Pufferlösung gebrauchsfertig, E. Merck, Darmstadt) extrahiert, mit festem wasserfreiem Natriumsulfat getrocknet und eingedampft.
Ausbeute des Diastereomerengemisches: 3,50 g (Beispiel 1).
Das Produktgemisch wird chromatographisch (Kieselgel, Dichlormethan : Ethanol = 50:1) aufgetrennt:

Beispiel Nr. 2:

Diastereomer A [2(S)-Diastereomer]: 1,23 g
$R_f$ = 0,18 (Dichlormethan : Ethanol = 50:1)
$^1$H-NMR (d-DMSO, 250 MHz, TMS): δ = 0,87 (M, 1H), 1,19-1,63 (M, 6H), 1,72 (M, 1H), 2,45 (M, 1H), 2,58 (S, 3H), 2,79 (S, 3H), 3,26 (D, 1H), 3,44-3,53 (M, 2H), 4,21-4,31 (M, 2H), 5,63 (S, 2H), 6,97-7,11 (M, 8H), 7,20-7,28 (M, 3H), 7,41 (M, 1H), 7,54 (D, 1H), 8,12 (D, 1H), 8,24 (D, 1H) ppm.

Beispiel Nr. 3:

Diastereomer B [2(R)-Diastereomer]: 1,12 g
$R_f$ = 0,16 (Dichlormethan : Ethanol = 50:1)
$^1$H-NMR (d-DMSO, 250 MHz, TMS): δ = 0,84 (M, 1H), 1,07-1,59 (M, 7H), 2,34 (M, 1H), 2,61 (S, 3H), 2,80 (S, 3H), 3,25 (D, 1H), 3,43 (M, 2H), 4,63-4,72 (M, 2H), 5,66 (S, 2H), 6,98 (S, 1H), 7,13 (M, 2H), 7,20-7,30 (M, 8H), 7,43 (M, 1H), 7,57 (D, 1H), 8,12 (D, 1H), 8,36 (D, 1H) ppm.

Die absolute Konfiguration der enantiomerenreinen Carbonsäuren 2-(S)- und 2-(R)-2-{4-[(Chinolin-2-yl)methoxy]phenyl}-2-cyclopentyl-essigsäure [vgl. EP 509 359] sind bekannt, so daß die absoluten Konfigurationen der daraus analog der Vorschrift zu den Beispielen 1 und 2 hergestellen Amiden Bsp.-Nr. C1 und Bsp.-Nr. C2 abgeleitet werden können. Die $^1$H-NMR-Spektren der beiden diastereomeren Produkte (200 MHz, $d_6$-DMSO, TMS für Beispiel-Nr. C1 und 250 MHz, $d_6$-DMSO, TMS für Beispiel-Nr. C2 / Abbildung 1) weisen im Aromatenbereich signifikante Unterschiede auf: Die H-Signale des Phenylrestes von Bsp.-Nr. C1 liegen bei ca. 7,1 ppm (3H) und 7,3 ppm (2H) die H-Signale von Bsp.-Nr. C2 bei ca. 7,3 ppm (5H). Dieser Befund ist auf die Verbindungen der Beispiele 2 und 3 (Abbildung 2) sowie auf viele andere Derivate diesen Typs übertragbar.

In Analogie zur Vorschrift der Beispiele 1, 2 und 3 werden die in den Tabellen 1, 2 und 3 aufgeführten Beispiele hergestellt:

## Tabelle 1:

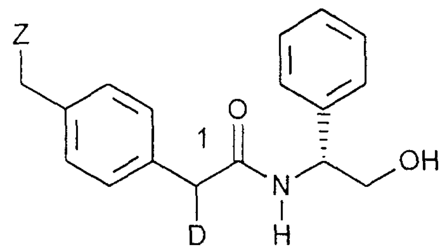

| Bsp.-Nr. | Z | D | 1 | $R_f$ (Solvens) | Ausgangsmaterial * (Bsp.-Nr.) |
|---|---|---|---|---|---|
| 4 | 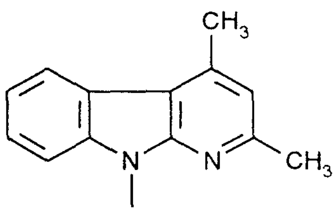 | cPent | rac | 0,41 / 0,46 (E) | LXI |
| 5 | 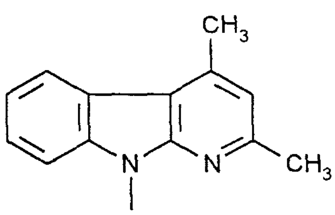 | cPent | S | 0,46 (E) | LXI |
| 6 | 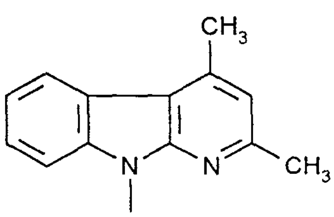 | cPent | R | 0,41 (E) | LXI |

| Bsp.-Nr. | Z | D | I | $R_f$ (Solvens) | Ausgangsmaterial * (Bsp.-Nr.) |
|---|---|---|---|---|---|
| 7 | 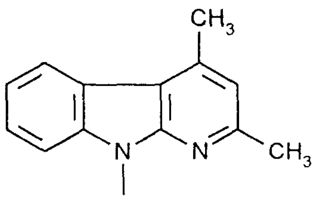 | cHept | rac | 0,26 / 0,29 (D) | LXIII |
| 8 | 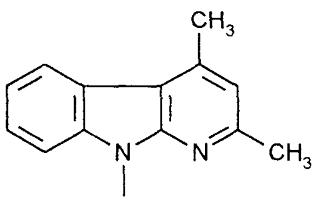 | cHept | S | 0,29 (D) | LXIII |
| 9 | 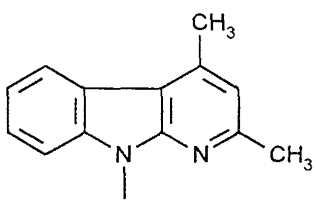 | cHept | R | 0,26 (D) | LXIII |
| 10 | 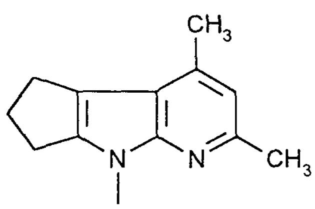 | cHept | rac | 0,20 / 0,24 (E) | LXIV |
| 11 | 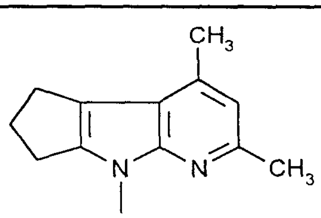 | cHept | S | 0,24 (E) | LXIV |

| Bsp.-Nr. | Z | D | 1 | $R_f$ (Solvens) | Ausgangsmaterial * (Bsp.-Nr.) |
|---|---|---|---|---|---|
| 12 | 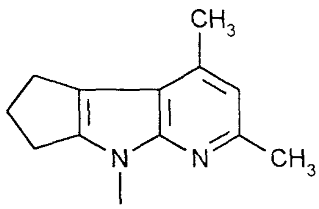 | cHept | R | 0,20 (E) | LXIV |
| 13 | 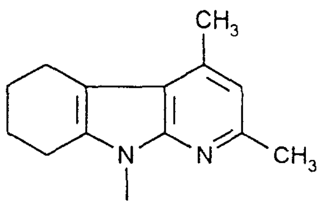 | cHept | rac | 0,35 (C) | LXV |
| 14 | 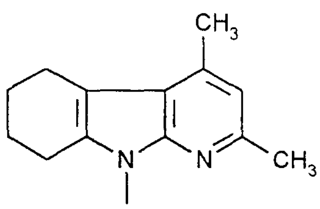 | cHept | S | 0,35 (C) | LXV |
| 15 | 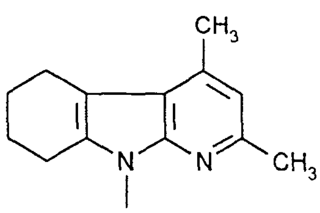 | cHept | R | 0,35 (C) | LXV |
| 16 | 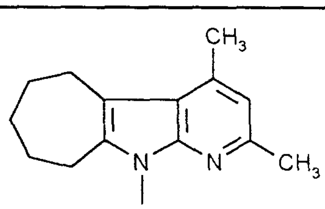 | cPent | rac | 0,33 / 0,37 (C) | LXVI |

| Bsp.-Nr. | Z | D | 1 | $R_f$ (Solvens) | Ausgangsmaterial * (Bsp.-Nr.) |
|---|---|---|---|---|---|
| 17 | 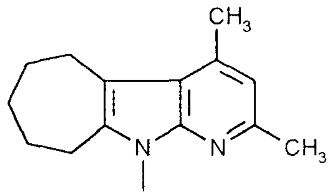 | cHept | rac | 0,25 / 0,38 (C) | LXVII |
| 18 | 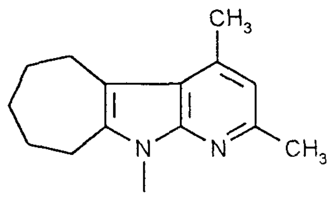 | cHept | S | 0,38 (C) | LXVII |
| 19 | 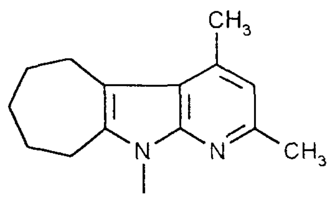 | cHept | R | 0,25 (C) | LXVII |
| 20 | 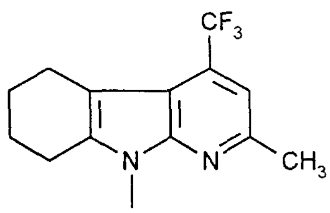 | cPent | rac | 0,29 (A) | LXVIII |
| 21 | 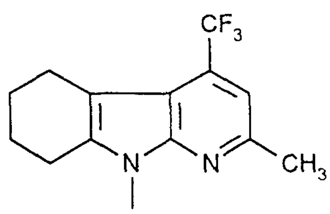 | cHept | rac | 0,23 / 0,28 (D) | LXIX |

| Bsp.-Nr. | Z | D | 1 | $R_f$ (Solvens) | Ausgangsmaterial * (Bsp.-Nr.) |
|---|---|---|---|---|---|
| 22 | 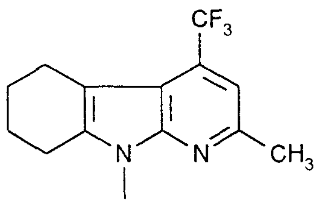 | cHept | S | 0,28 (D) | LXIX |
| 23 | 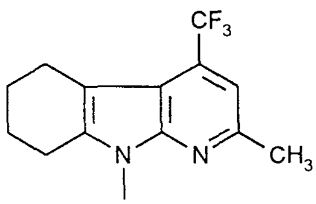 | cHept | R | 0,23 (D) | LXIX |
| 24 | 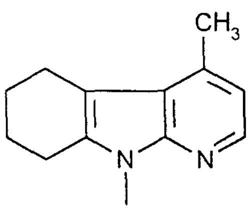 | cHept | rac | 0,10 / 0,18 (E) | LXX |
| 25 | 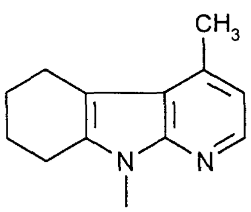 | cHept | S | 0,18 (E) | LXX |
| 26 | 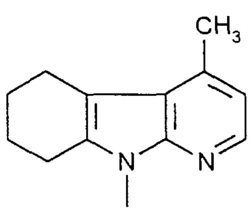 | cHept | R | 0,10 (E) | LXX |

| Bsp.-Nr. | Z | D | 1 | $R_f$ (Solvens) | Ausgangsmaterial * (Bsp.-Nr.) |
|---|---|---|---|---|---|
| 27 | 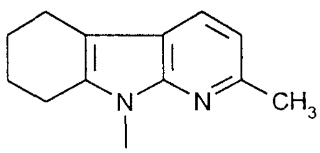 | cHept | rac | 0,17 / 0,23 (B) | LXXXI |
| 28 | 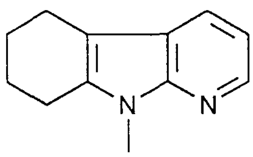 | cHept | rac | 0,12 / 0,15 (B) | LXXXIV |
| 29 | 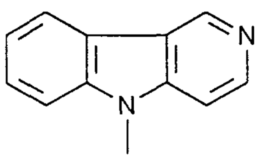 | cPent | rac | 0,28 (E) | LXXI |
| 30 | 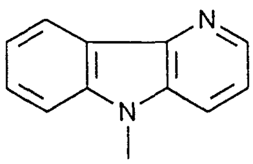 | cPent | rac | 0,29 (C) | LXXII |
| 31 | 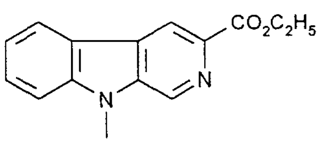 | cPent | rac | 0,24 (C) | LXXIII. |
| 32 | 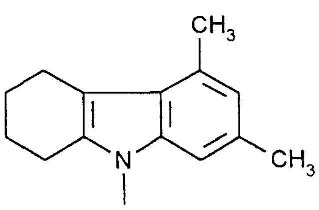 | cPent | rac | 0,39 / 0,48 (C) | LXXIV |

| Bsp.-Nr. | Z | D | 1 | $R_f$ (Solvens) | Ausgangsmaterial * (Bsp.-Nr.) |
|---|---|---|---|---|---|
| 33 | 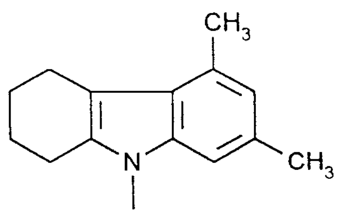 | cPent | S | 0,48 (C) | LXXIV |
| 34 | 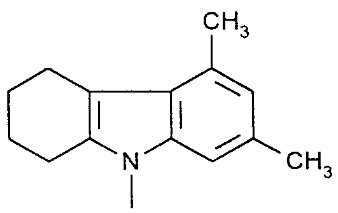 | cPent | R | 0,39 (C) | LXXIV |
| 35 | 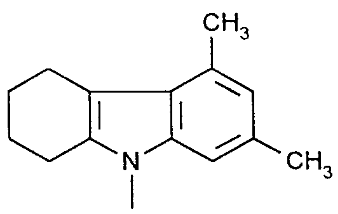 | cHept | rac | 0,23 / 0,29 (D) | LXXV |
| 36 | 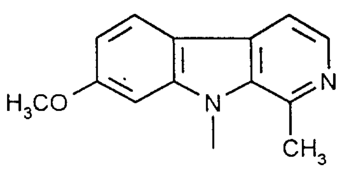 | cPent | rac | 0,26 (A) | LXXVI |
| 37 | 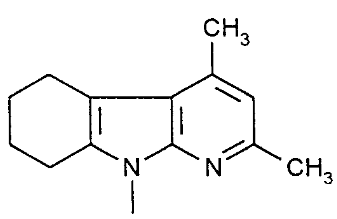 | cHex | rac | 0,28 / 0,30 (D) | LXXVII |

| Bsp.-Nr. | Z | D | 1 | $R_f$ (Solvens) | Ausgangsmaterial * (Bsp.-Nr.) |
|---|---|---|---|---|---|
| 38 | 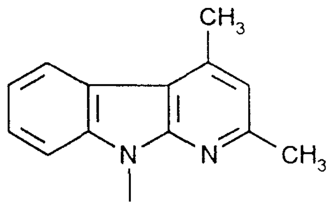 | cHex | rac | 0,21 / 0,23 (D) | LXXVIII |

* Das (R)-Phenylglycinol ist bei Aldrich käuflich.

**Tabelle 2:**

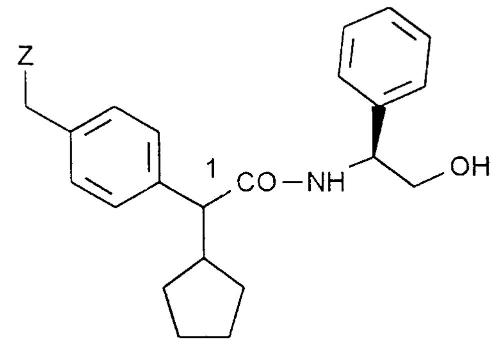

| Bsp.-Nr. | Z | 1 | Rf (Solvens) | Ausgangsmaterial * ( Bsp.-Nr.) |
|---|---|---|---|---|
| 39 | 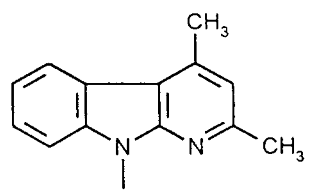 | rac | 0,42 (C) | LXI |
| 40 | 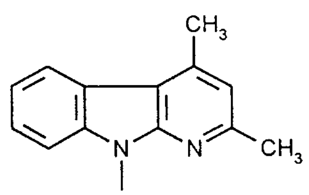 | R | 0,42 (C) | LXI |
| 41 | 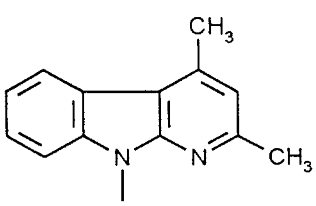 | S | 0,42 (C) | LXI |

* Das (S)-Phenylglycinol ist bei Aldrich käuflich

**Tabelle 3:**

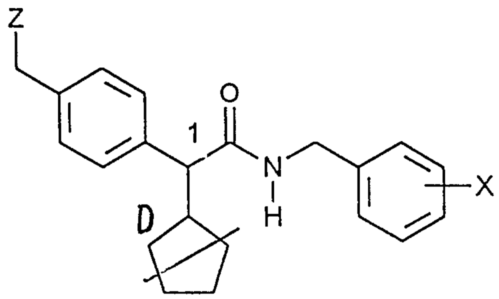

| Bsp.-Nr. | Z | D | X | 1 | $R_f$ (Solvens) | Ausgangs-material (Bsp.-Nr.) |
|---|---|---|---|---|---|---|
| 42 | 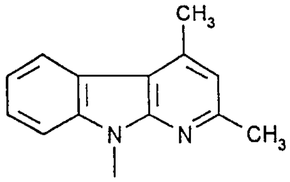 | cHept | H | rac | 0,39 (C) | Carbonsäure: Bsp.-Nr. LXIII Amin von Aldrich |
| 43 | 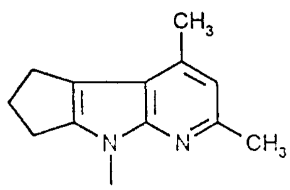 | cHept | H | rac | 0,78 (E) | Carbonsäure: Bsp.-Nr. LXIV Amin von Aldrich |
| 44 | 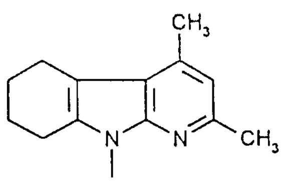 | cPent | H | rac | 0,34 (D) | Carbonsäure: Bsp.-Nr. LXII Amin von Aldrich |
| 45 | 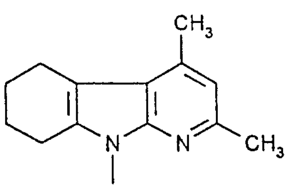 | cPent | H | (-)-ent * | 0,34 (D) | Carbonsäure: Bsp.-Nr. LXII Amin von Aldrich |
| 46 | 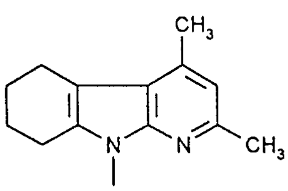 | cPent | H | (+)-ent * | 0,34 (D) | Carbonsäure: Bsp.-Nr. LXII Amin von Aldrich |

| Bsp.-Nr. | Z | D | X | 1 | $R_f$ (Solvens) | Ausgangs-material (Bsp.-Nr.) |
|---|---|---|---|---|---|---|
| 47 | 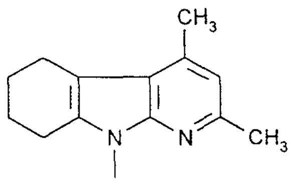 | cHept | H | rac | 0,25 (C) | Carbonsäure: Bsp.-Nr. LXV Amin von Aldrich |
| 48 | 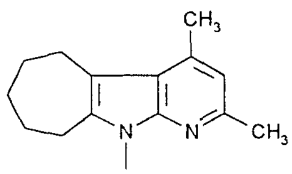 | cHept | H | rac | 0,42 (C) | Carbonsäure: Bsp.-Nr. LXVII Amin von Aldrich |
| 49 | 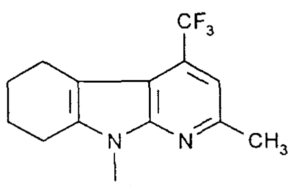 | cHept | H | rac | 0,45 (C) | Carbonsäure: Bsp.-Nr. LXIX Amin von Aldrich |
| 50 | 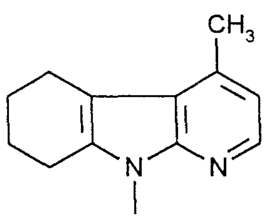 | cHept | H | rac | 0,71 (E) | Carbonsäure: Bsp.-Nr. LXX Amin von Aldrich |
| 51 | 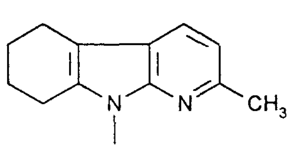 | cHept | H | rac | 0,59 (B) | Carbonsäure: Bsp.-Nr. LXXXI Amin von Aldrich |
| 52 | 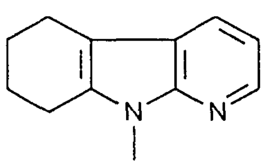 | cHept | H | rac | 0,40 (B) | Carbonsäure: Bsp.-Nr. LXXXIV Amin von Aldrich |
| 53 | 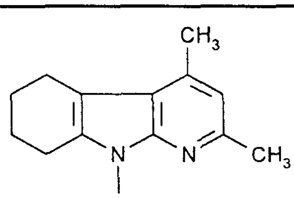 | cHept | 3-OH | rac | 0,45 (D) | Carbonsäure: Bsp.-Nr. LXV Amin: Lit.: US 43 88 250 |
| 54 | 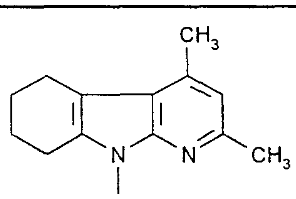 | cHept | 4-OH | rac | 0,39 (A) | Carbonsäure: Bsp.-Nr. LXV Amin: Lit.: C. Hartmann und J.P. Klinman, Biochemistry, **30**, 4605 (1991). |

| **Bsp.-Nr.** | **Z** | **D** | **X** | **l** | **$R_f$ (Solvens)** | **Ausgangs-material (Bsp.-Nr.)** |
|---|---|---|---|---|---|---|
| 55 | 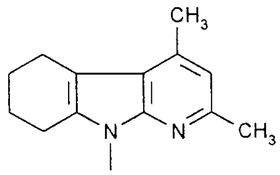 | cHept | 2-$OCH_3$ | rac | 0,15 (B) | Carbonsäure: Bsp.-Nr. LXV Amin von Aldrich |
| 56 | 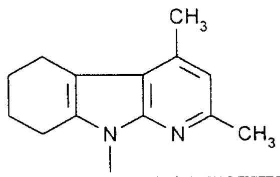 | cHept | 3-$OCH_3$ | rac | 0,37 (D) | Carbonsäure: Bsp.-Nr. LXV Amin von Lancaster |
| 57 | 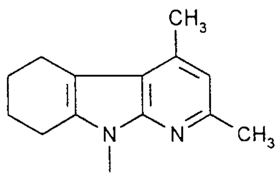 | cHept | 4-$OCH_3$ | rac | 0,24 (B) | Carbonsäure: Bsp.-Nr. LXV Amin von Aldrich |
| 58 | 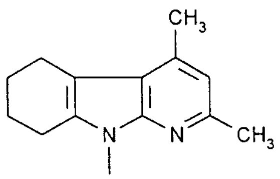 | cHept | 2-O-$CH_2$-CH=$CH_2$ | rac | 0,51 (C) | Carbonsäure: Bsp.-Nr. LXV Amin: Bsp.-Nr. II |
| 59 | 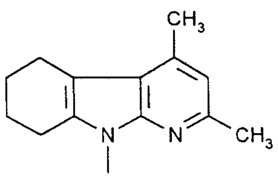 | cHept | 3-$CO_2CH_3$ | rac | 0,73 (C) | Carbonsäure: Bsp.-Nr. LXV Amin: Lit.: F.M. Markwardt et al., Pharmazie **22**, 465 (1967). |
| 60 | 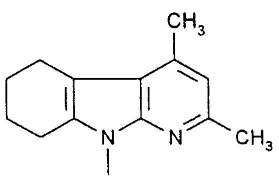 | cHept | 4-$CO_2CH_3$ | rac | 0,33 (B) | Carbonsäure: Bsp.-Nr. LXV Amin: Lit.: M.G. Nair und C.M. Baugh, J. Org. Chem. **38**, 2185 (1973). |
| 61 | 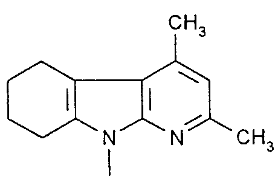 | cHept | 3-$CH_3$ | rac | 0,19 (B) | Carbonsäure: Bsp.-Nr. LXV Amin von Aldrich |
| 62 | 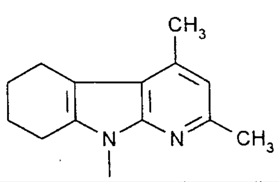 | cHept | 2-$NO_2$ | rac | 0,39 (B) | Carbonsäure: Bsp.-Nr. LXV Amin: Lit.: EP 373 891 |

| **Bsp.-Nr.** | **Z** | **D** | **X** | **I** | **$R_f$ (Solvens)** | **Ausgangs-material (Bsp.-Nr.)** |
|---|---|---|---|---|---|---|
| 63 | 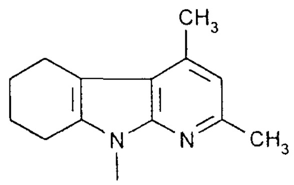 | cHept | 3-$NO_2$ | rac | 0,28 (B) | Carbonsäure: Bsp.-Nr. LXV Amin von Aldrich |
| 64 | 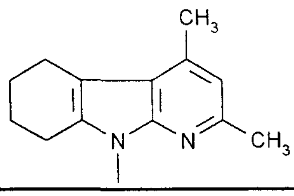 | cHept | 4-$NO_2$ | rac | 0,21 (B) | Carbonsäure: Bsp.-Nr. LXV Amin von Aldrich |
| 65 | 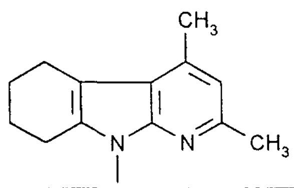 | cHept | 2-Cl | rac | 0,75 (D) | Carbonsäure: Bsp.-Nr. LXV Amin von Aldrich |
| 66 | 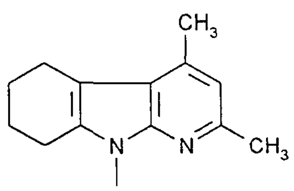 | cHept | 3-Cl | rac | 0,71 (D) | Carbonsäure: Bsp.-Nr. LXV Amin von Lancaster |
| 67 | 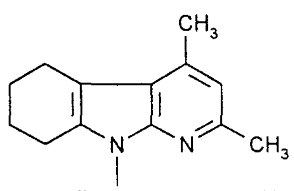 | cHept | 4-Cl | rac | 0,61 (D) | Carbonsäure: Bsp.-Nr. LXV Amin von Aldrich |
| 68 | 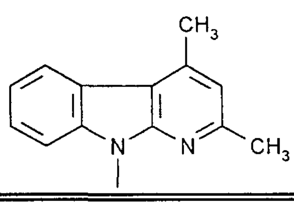 | cPent | H | rac | 0,28 (D) | Carbonsäure: Bsp.-Nr. LXI Amin von Aldrich |

* Die Enantiomerentrennung gelingt über HPLC (Chiralpak AD, Länge 250 mm, Durchmesser 4,6 mm, Partikelgröße 10 µ, Laufmittel: 95% n-Heptan + 5 % Ethanol (letzteres mit 1% Wasser und 0,2% Trifluoressigsäure)).

**Beispiel 69**

2-(R,S)-2-[4-(2,4-Dimethyl-5,6,7,8-tetrahydro-α-carbolin-9-yl)-methyl-phenyl]-2-cycloheptyl-essigsäure-N-(2-hydroxy-benzyl)amid

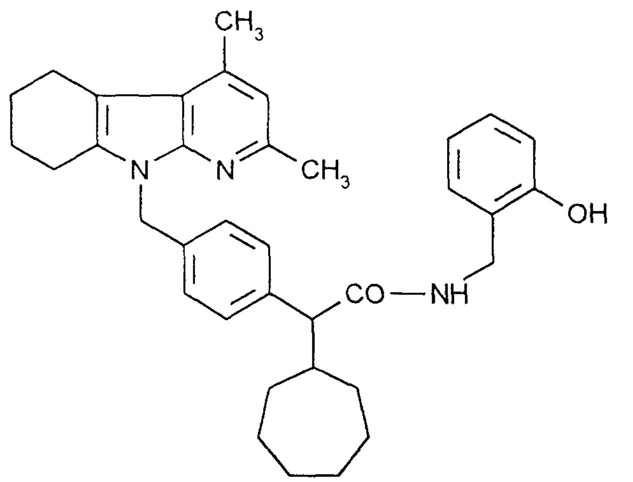

0,60 g der Verbindung aus Beispiel 58 werden in 3ml Methanol und 0,6 ml Wasser unter Argon als Schutzgas mit 33 mg Palladium (10% auf Tierkohle) und 33 mg para-Toluolsulfonsäure Monohydrat 22 Stunden unter Rückfluß gekocht. Bei unvollständiger Umsetzung (DC-Kontrolle, Dichlormethan : Ethanol = 50:1) gibt man noch einmal 33 mg Palladium (10% auf Tierkohle) und 33 mg para-Toluolsulfonsäure Monohydrat zu und kocht weitere 24 Stunden unter Rückfluß. Der Katalysator wird heiß abgesaugt, mit viel heißem Methanol nachgewaschen und das Filtrat eingedampft. Nach der Trocknung im Hochvakuum über Phosphorpentoxid fallen 0,52 g Produkt an.
$R_f$ = 0,33 (Dichlormethan : Ethanol = 50:1)

**Beispiel 70**

2-(R,S)-2-[4-(3-Hydroxymethyl-β-carbolin-9-yl)-methyl-phenyl]-2-cyclo-pentylessigsäure-N-(R)-phenylglycinolamid

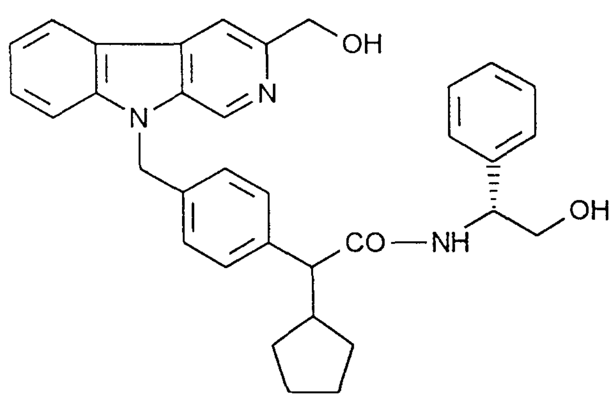

500 mg (0,868 mmol) der Verbindung aus Beispiel 31 werden unter Argon bei 0°C in 5 ml wasserfreiem Tetrahydrofuran tropfenweise mit 1,737 ml (1,737 mmol) einer 1 M Lithiumalanatlösung in Tetrahydrofuran versetzt und 4 h bei ca. 20°C nachgerührt. Die Reaktionsmischung wird vorsichtig mit 5 ml Wasser versetzt und mit 2 M wäßriger Salzsäure auf einen pH-Wert von ca. 2 gestellt. Die wäßrige Phase wird mehrfach mit Diethylether und Dichlormethan

extrahiert, mit Natriumsulfat getrocknet und eingedampft. Das Rohprodukt wird an Kieselgel 60 (Merck, Dichlormethan bis Dichlormethan : Methanol = 50:1) chromatographisch gereinigt.
Ausbeute: 0,12 g
$R_f$ = 0,26 (Dichlormethan : Ethanol = 20:1)

Die Verbindungen der Tabelle 4 werden in Analogie zur Vorschrift des Beispiels 70 hergestellt:

**Tabelle 4:**

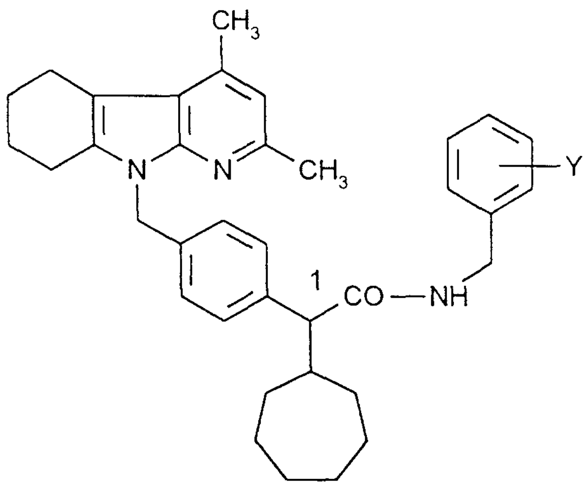

| Bsp.-Nr. | Y | 1 | $R_f$ (Solvens) | Ausgangsmaterial |
|---|---|---|---|---|
| 71 | 4-$CH_2OH$ | rac | 0,47 (C) | Bsp.-Nr. 60 |
| 72 | 3-$CH_2OH$ | rac | 0,26 (C) | Bsp.-Nr. 59 |

**Beispiel 73**

2-(R,S)-2-[4-(2,4-Dimethyl-5,6,7,8-tetrahydro-α-carbolin-9-yl)-methyl-phenyl]-2-cycloheptyl-essigsäure-N-(4-carboxy-benzyl)amid

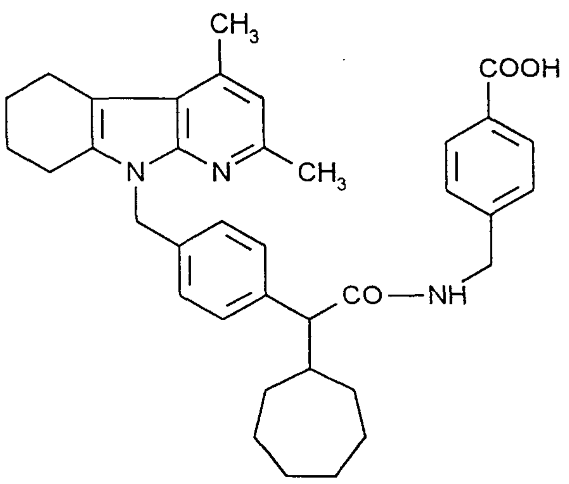

0,325 g (0,55 mmol) der Verbindung aus Beispiel 60 werden 18 h mit 0,5 ml wäßriger 2 M Natronlauge in 3 ml Methanol bei 60°C umgesetzt. Ist die Umsetzung laut Dünnschichtanalytik (Solvens F) noch nicht beendet, setzt man weitere 0,5 ml wäßrige 2 M Natronlauge in 1 ml Methanol zu und kocht nun 24 h unter Rückfluß. Die Reaktiosmischung wird abgekühlt, mit 1 M Salzsäure auf einen pH-Wert von ca. 4 gestellt und der ausfallende Niederschlag abgesaugt, mit Wasser und Petrolether : Diethylether = 5:1 gewaschen und im Hochvakuum über Phosphorpentoxid vom Restlösemittel befreit.
Ausbeute: 0,154 g
$R_f$ = 0,50 (Dichlormethan : Methanol : Essigsäure = 90:10:2)

**Beispiel 74**

2-(R,S)-2-[4-(2,4-Dimethyl-5,6,7,8-tetrahydro-α-carbolin-9-yl)-methyl-phenyl]-2-cycloheptyl-essigsäure-N-(3-carboxybenzyl)amid

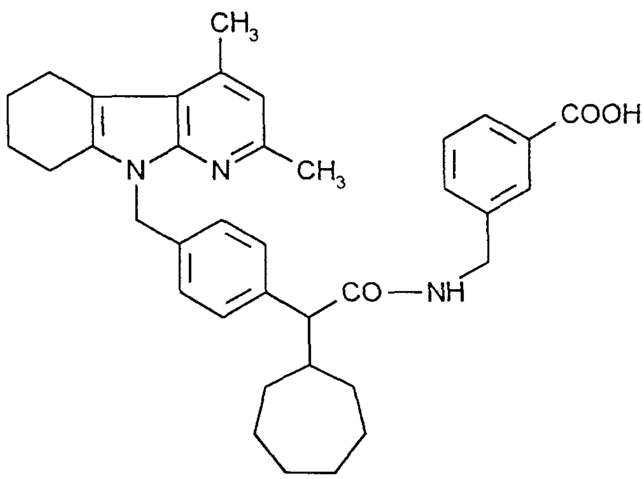

Analog der Vorschrift des Beispiels 73 läßt sich die Titelverbindung aus der Verbindung des Beispiels 59 herstellen.
$R_f$ = 0,27 (Dichlormethan : Ethanol = 20:1)
In Analogie zur Vorschrift des Beispiels 1 werden die in den Tabellen 5, 6, 7, 8, 9 und 10 aufgeführten Verbindungen hergestellt:

Tabelle 5:

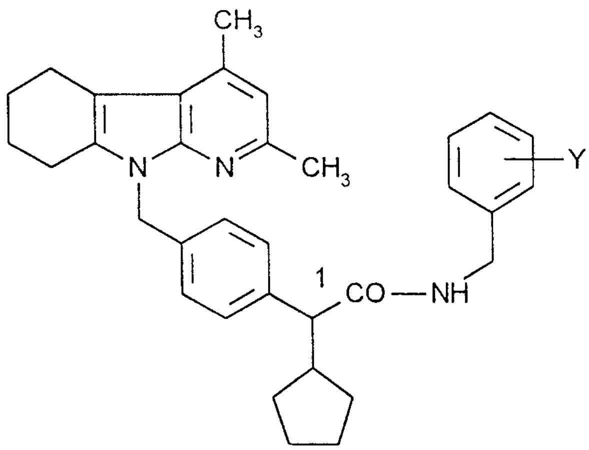

| Bsp.-Nr. | Y | 1 | Fp. (°C) | Ausgangsmaterial |
|---|---|---|---|---|
| 75 | 3-OH | rac | 177-178 | Carbonsäure: Bsp.-Nr. LXII Amin: US 43 88 250. |
| 76 | 4-OH | rac | 183-184 | Carbonsäure: Bsp.-Nr. LXII Amin: Lit.: C. Hartmann und J.P. Klinman, Biochemistry **30**, 4605 (1991). |

**Tabelle 6:**

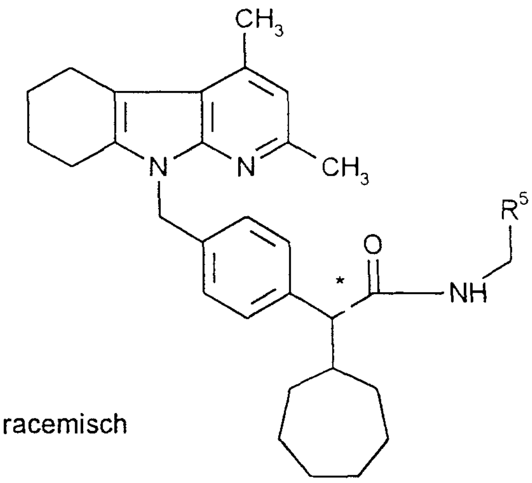

* racemisch

| Bsp.-Nr. | $R^5$ | $R_f$ (Solvens) | Ausgangsmaterial |
|---|---|---|---|
| 77 | 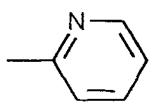 | 0,20 (C) | Carbonsäure: Bsp.-Nr. LXV<br>Amin von Aldrich |
| 78 | 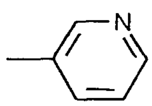 | 0,12 (C) | Carbonsäure: Bsp.-Nr. LXV<br>Amin von Aldrich |
| 79 | 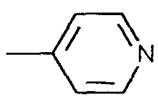 | 0,19 (C) | Carbonsäure: Bsp.-Nr. LXV<br>Amin von Aldrich |
| 80 | 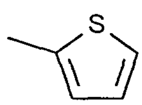 | 0,24 (D) | Carbonsäure: Bsp.-Nr. LXV<br>Amin von Aldrich |

**Tabelle 7:**

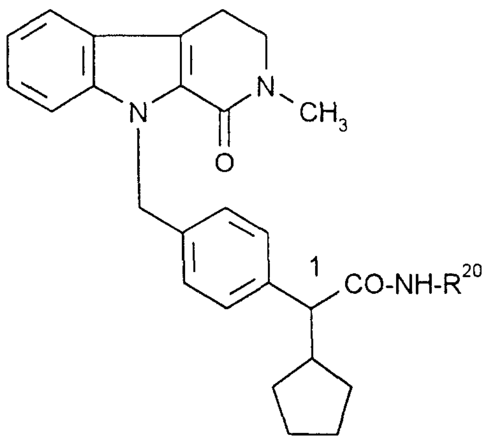

| Bsp.-Nr. | 1 | $-R^{20}$ | $R_f$ (Solvens) | Ausgangsmaterial |
|---|---|---|---|---|
| 81 | rac | 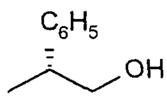 | 0,10 (P) | Säure: Bsp.-Nr. LXXIX Amin von Aldrich |
| 82 | rac | 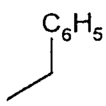 | 0,28 (P) | Säure: Bsp.-Nr. LXXIX Amin von Aldrich |

**Tabelle 8:**

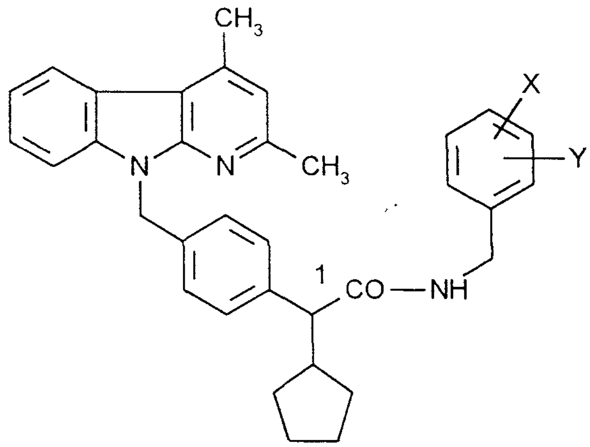

| **Bsp.-Nr.** | **1** | **X** | **Y** | **Fp. (°C)**<br>**$R_f$**<br>**(Solvens)** | **MS (FAB)** | **Ausgangsmaterial**<br>**a) Literatur**<br>**b) Vertreiberfirma**<br>**c) Syn.aus Bsp.-Nr.** |
|---|---|---|---|---|---|---|
| 83 | rac | 3-$OCH_3$ | 4-$OCH_3$ | 179<br>0,50 (A) | 562 (100%)<br>154 (80%) | Carbonsäure:<br>c) Bsp.-Nr. LXI<br>Amin von Aldrich. |
| 84 | rac | 3-$CH_3$ | 5-$CH_3$ | 212<br>0,60 (B) | 530 (100%) | Carbonsäure:<br>c) Bsp.-Nr. LXI<br>Amin von Emka-Chemie. |
| 85 | rac | 3-Cl | 5-Cl | 212<br>0,18 (M) | 570 (100%)<br>196 (50%) | Carbonsäure:<br>c) Bsp.-Nr. LXI<br>Amin von Maybridge. |
| 86 | rac | 3-OH | 4-OH | 137<br>0,39 (A) | 534 (100%)<br>307 (60%) | Carbonsäure:<br>c) Bsp.-Nr. LXI<br>Amin von Aldrich. |
| 87 | rac | 3-$OCH_3$ | 4-OH | 135<br>0,65 (A) | 548 (80%)<br>154 (100%) | Carbonsäure:<br>c) Bsp.-Nr. LXI<br>Amin von Aldrich. |

Tabelle 9:

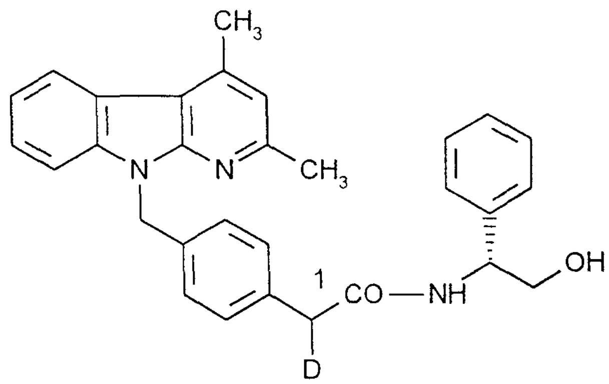

| **Bsp.-Nr.** | **1** | **D** | **Fp. (°C)**<br>**$R_f$ (Solvens)** | **MS (FAB)** | **Ausgangsmaterial ***<br>**a) Literatur**<br>**b) Vertreiberfirma**<br>**c) Syn. aus Bsp.-Nr.** |
|---|---|---|---|---|---|
| 88 | rac | iPr | 210<br>0,37/0,31 (A) | 506 (100%)<br>154 (60%) | Carbonsäure:<br>Bsp.-Nr. LXXXII |
| 89 | rac | iBu | -<br>0,30 (A) | 520 (100%)<br>154 (50%) | Carbonsäure:<br>Bsp.-Nr. LXXX |

* Das (R)-Phenylglycinol ist bei Aldrich käuflich.

**Tabelle 10:**

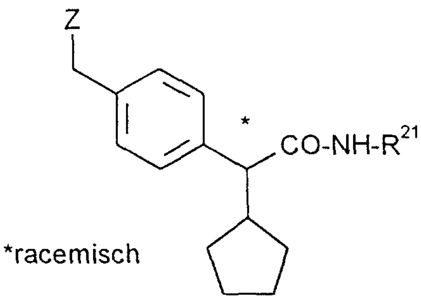

*racemisch

| Bsp.-Nr. | -Z | $-R^{21}$ | Fp. (°C) $R_f$ (Solvens) | Ausgangsmaterial aus Bsp.-Nr. |
|---|---|---|---|---|
| 90 | 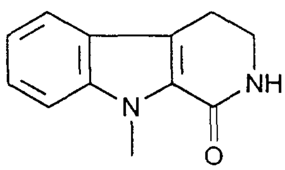 | 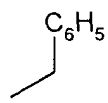 | 188-189 | LXXXIX |
| 91 | 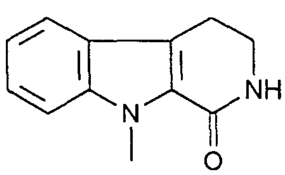 | 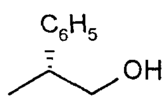 | 0,024 (P) | LXXXIX |
| 92 | 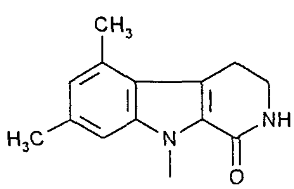 | 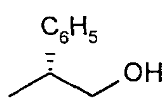 | 207-208 | XC |
| 93 | 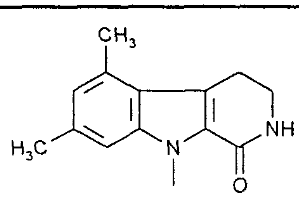 | 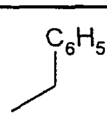 | 211-212 | XC |

Die Verbindungen der Tabelle 11 werden analog der Vorschrift von Beispiel Nr. 1, 2 und 3 hergestellt:

**Tabelle 11:**

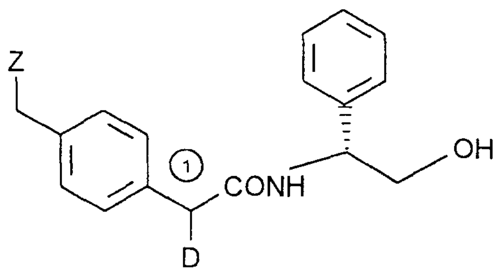

| Bsp.-Nr. | Z | D | (1) | Fp.[0°] $R_f$ (Solvens) | MS (FAB) | Ausgangs-material aus Bsp.-Nr. |
|---|---|---|---|---|---|---|
| 94 | 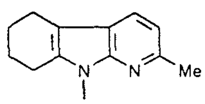 | cHept | S | 0,23 (B) | | 27 |
| 95 | 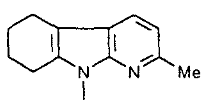 | cHept | R | 0,17 (B) | | 27 |
| 96 | 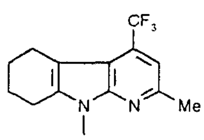 | cPent | S | 0,29 (A) | | 20 |
| 97 | 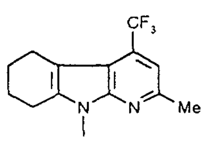 | cPent | R | 0,29 (A) | | 20 |
| 98 | 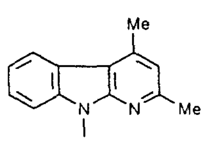 | cHex | S | 0,23 (D) | | 38 |

Tabelle 11 - Fortsetzung

| Bsp.-Nr. | Z | D | ① | Fp.[0°] $R_f$ (Solvens) | MS (FAB) | Ausgangs-material aus Bsp.-Nr. |
|---|---|---|---|---|---|---|
| 99 | 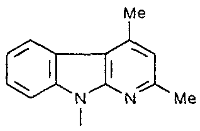 | cHex | R | 0,21 (D) | | 38 |
| 100 | 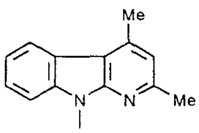 | iPr | S | 208°C | 506 (100 %) 154 (40 %) | 88 |
| 101 | 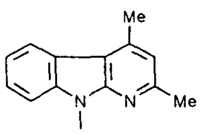 | iPr | R | 204°C | 506 (100 %) 154 (40 %) | 88 |
| 102 | 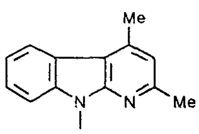 | iBu | S | 182°C | | 89 |
| 103 | 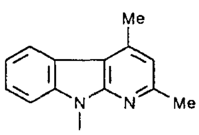 | iBu | R | 206°C | | 89 |
| 104 | 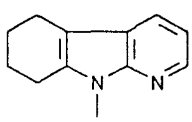 | cPent | rac | 0,34 (C) | | CV |
| 105 | 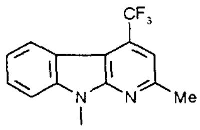 | cPent | rac | 0,44 (E) 0,56 | | CVI |

<u>Tabelle 11</u> - Fortsetzung

| Bsp.-Nr. | Z | D | ① | Fp.[0°] $R_f$ (Solvens) | MS (FAB) | Ausgangs-material aus Bsp.-Nr. |
|---|---|---|---|---|---|---|
| 106 | 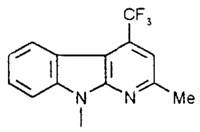 | cPent | S | 0,56 (E) | 586 (100 %)<br>154 (94 %) | CVI |
| 107 | 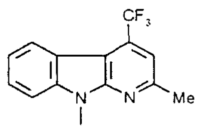 | cPent | R | 0,44 (E) | | CVI |
| 108 | 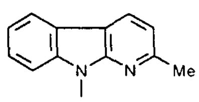 | cPent | rac | 0,26 (E)<br>0,31 | | CVII |
| 109 | 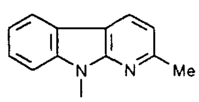 | cPent | S | 0,55 (C) | | CVII |
| 110 | 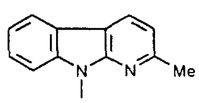 | cPent | R | 0,57 (C) | | CVII |
| 111 | 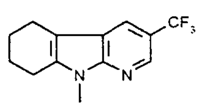 | cPent | rac | 0,45 (C) | | CVIII |
| 112 | 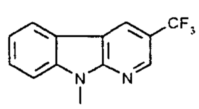 | cPent | rac | 0,4 C | | CIX |
| 113 | 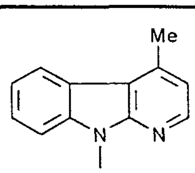 | cPent | rac | 0,37 C | | CX |

| Bsp.-Nr. | Z | D | ① | Fp.[0°] $R_f$ (Solvens) | MS (FAB) | Ausgangs-material aus Bsp.-Nr. |
|---|---|---|---|---|---|---|
| 114 | 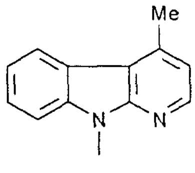 | cPent | S | 0,37 (C) | | CX |
| 115 | 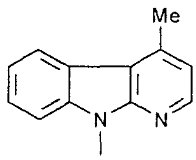 | cPent | R | 0,37 (C) | | CX |
| 116 | 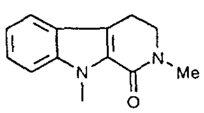 | cPent | diaA | 194°C | | 81 |
| 117 | 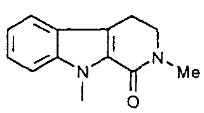 | cPent | diaB | 137°C | | 81 |

Die Verbindungen der Tabelle 12 werden analog der Vorschrift von Beispiel Nr. 1, 2 und 3 hergestellt:

## Tabelle 12:

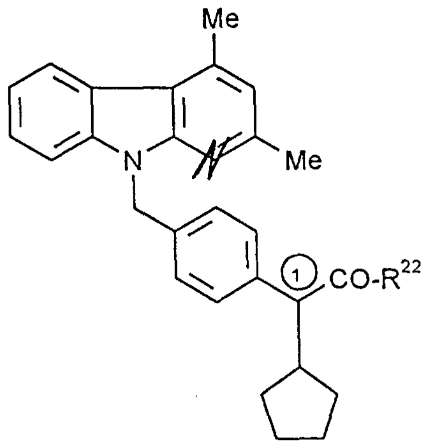

| Bsp.-Nr. | ① | $R^{22}$ | Fp.[0°] Rf (Solvens) | MS (FAB) | Ausgangs-material aus Bsp.-Nr. |
|---|---|---|---|---|---|
| 118 | rac | 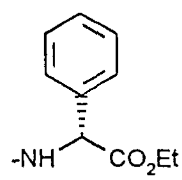 | 0,82 (C) | 574 (100 %) | LXI |
| 119 | rac | 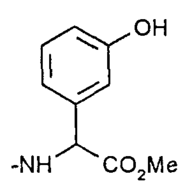 | 0,57 (C)<br>0,62 | 576 (100 %) | LXI |
| 120 | rac | 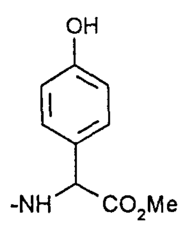 | 0,43 (C)<br>0,48 | | LXI |

Tabelle 12: - Fortsetzung

| **Bsp.-Nr.** | ① | **$R^{22}$** | **Fp.[0°] Rf (Solvens)** | **MS (FAB)** | **Ausgangs-material aus Bsp.-Nr.** |
|---|---|---|---|---|---|
| 121 | rac | 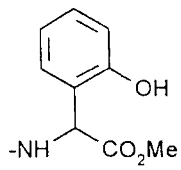 | 0,52 (C) | | LXI |
| 122 | rac | 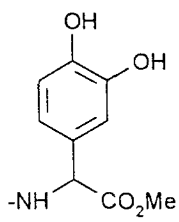 | 0,47 (C) | | LXI |
| 123 | rac | 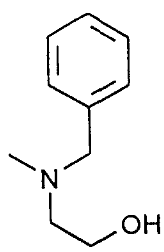 | 0,17 (D) 0,32 | | LXI |
| 124 | rac | 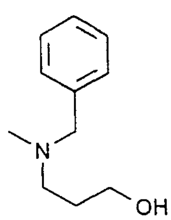 | 0,43 (C) | | LXI |

Tabelle 12: - Fortsetzung

| Bsp.-Nr. | ① | $R^{22}$ | Fp.[0°] Rf (Solvens) | MS (FAB) | Ausgangs-material aus Bsp.-Nr. |
|---|---|---|---|---|---|
| 125 | rac | 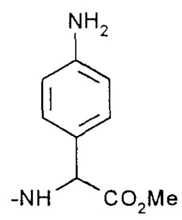 | 0,57 (C) | | LXI |
| 126 | rac | 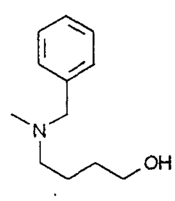 | 0,41 (C) | | LXI |
| 127 | rac | 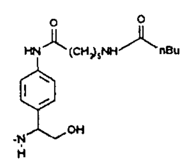 | 0,14 (C) | | 137 |
| 128 | S | 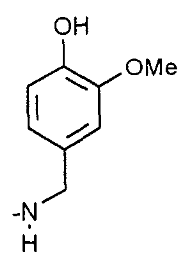 | 187°C | 548 (100 %)<br>154 (80 %)<br>137 (85 %) | LXI |

Die Verbindungen der Tabelle 13 werden analog der Vorschrift von Beispiel Nr. 73 hergestellt:

## Tabelle 13

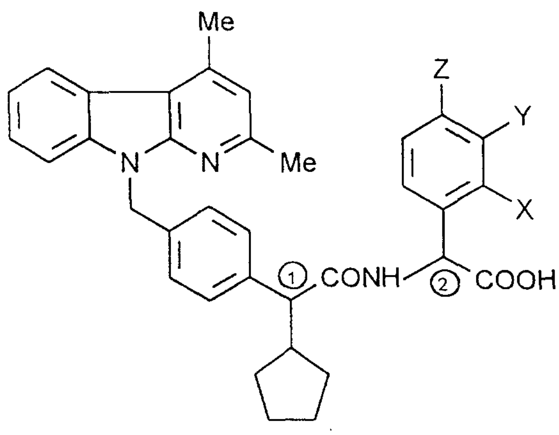

| Beispiel-Nr. | ①/② | X | Y | Z | Fp. [°C] $R_f$(Solvens | Ausgangs-Material aus Bsp.Nr. |
|---|---|---|---|---|---|---|
| 129 | rac/rac | H | H | H | 0,15 (S) | 118 |
| 130 | rac/rac | H | OH | H | 0,18 (T) 0,24 | 119 |
| 131 | rac/rac | H | H | OH | 0,68 (S) 0,76 | 120 |
| 132 | rac/rac | OH | H | H | 0,16 (T) 0,24 | 121 |

Die Verbindungen der Tabelle 14 werden analog der Vorschrift von Beispiel Nr. 70 hergestellt:

**Tabelle 14**

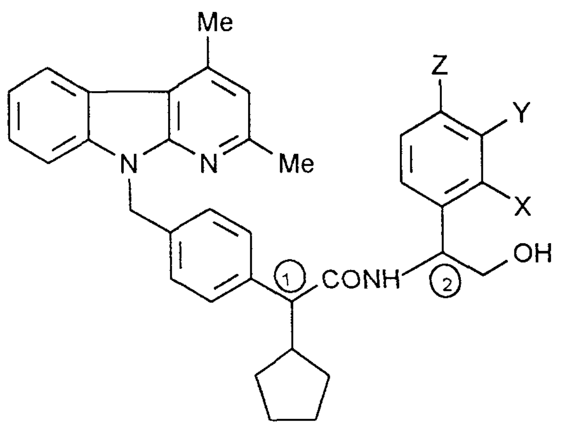

| **Beispiel-Nr.** | ①/② | **X** | **Y** | **Z** | **Fp. [°C] $R_f$(Solvens** | **Ausgangs-Material aus Bsp.Nr.** |
|---|---|---|---|---|---|---|
| 133 | rac/rac | H | OH | H | 0,30 (A) | 119 |
| 134 | rac/rac | H | H | OH | 0,25 (A) | 120 |
| 135 | rac/rac | OH | H | H | 0,33 (A) | 121 |
| 136 | rac/rac | H | OH | OH | 0,23 (A) | 122 |
| 137 | rac/rac | H | H | $NH_2$ | 0,31 (C) | 125 |

**Beispiel Nr. 138**

2-(R,S)-2-[4-(2,4-Dimethyl-α-carbolin-9-yl)-methyl-phenyl]-2-cyclopentyl-essigsäure-N-[1-(R,S)-1-(4-acetamido-phenyl)-2-hydroxy-ethyl]amid

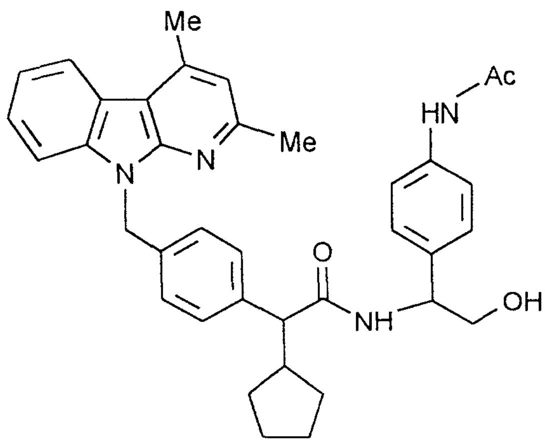

**Teil A)**

0,60 g (1,10 mmol) der Verbindung aus Beispiel Nr. 137 werden in 10 ml Dichlormethan mit 192 µl (3,29 mmol) Triethylamin versetzt und darauf bei 0°C mit 70 µl (0,99 mmol) Acetylchlorid zur Reaktion gebracht. Nach 3 Stunden Rührzeit, in der die Reaktionstemperatur auf 20°C anstieg, wurde nacheinander mit 1 M Salzsäure, 0,1 M wäßriger Natronlauge und Wasser geschüttet, die organische Phase mit Magnesiumsulfat getrocknet und eingedampft.

**Teil B)**

Das so erhaltene Rohprodukt zeigte im Massenspektrum (FAB) eine doppelte Acetylierung (631, 57 %, $M^+ + H$ / 653, 6 %, $M^+ + Na$). Daher setzte man es bei 20°C eine Stunde in 6 ml Methanol mit 2 M Natronlauge um. Der pH-Wert wurde darauf mit 1 M Salzsäure auf 2 gestellt und die erhaltene Mischung mit Essigester extrahiert. Die organische Phase wurde mit Wasser neutral gewaschen, mit Magnesiumsulfat getrocknet und im Vakuum eingedampft. Die Trocknung im Hochvakuum lieferte 0,28 g Produkt.
$R_f$ = 0,17 (Dichlormethan : Ethanol = 20 : 1)

**Beispiel Nr. 139**

2-(R,S)-2-[4-(2,4-Dimethyl-α-carbolin-9-yl)-methyl-phenyl]-2-cyclopentyl-essigsäure-N-[1-(R,S)-1-(4-acetamido-phenyl)-2-acetoxy-ethyl]amid

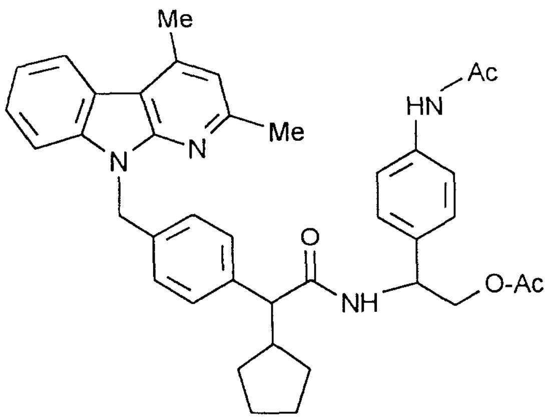

Setzt man die Verbindung aus Beispiel Nr. 137 analog Teil A der Vorschrift aus Beispiel Nr. 138 mit je 4 Äquivalenten Triethylamin und Acetylchlorid um, so erhält man die Titelverbindung.

$R_f$ = 0,56 (Dichlormethan : Ethanol = 20 : 1)

Die Verbindungen der Tabelle 15 werden analog der Vorschrift von Beispiel Nr. 138 hergestellt:

## Tabelle 15

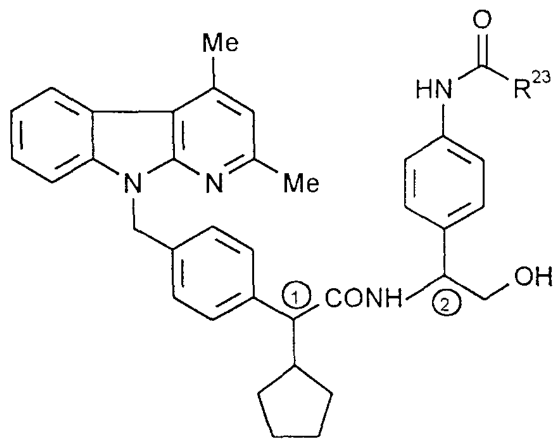

| Beispiel-Nr. | ①/② | $R^{23}$ | Fp. [°C] $R_f$(Solvens | Ausgangs-Material aus Bsp.Nr. |
|---|---|---|---|---|
| 140 | rac/rac | nBu | 0,49 (A) | 137 |
| 141 | rac/rac | Et | 0,81 (U) | 137 |

**Beispiel Nr. 142**

2-(S)-2-[4-(2,4-Dimethyl-α-carbolin-9-yl)-methyl-phenyl]-2-cyclopentyl-essigsäure-N-[1-(R)-1-phenyl-2-acetoxy-ethyl] amid

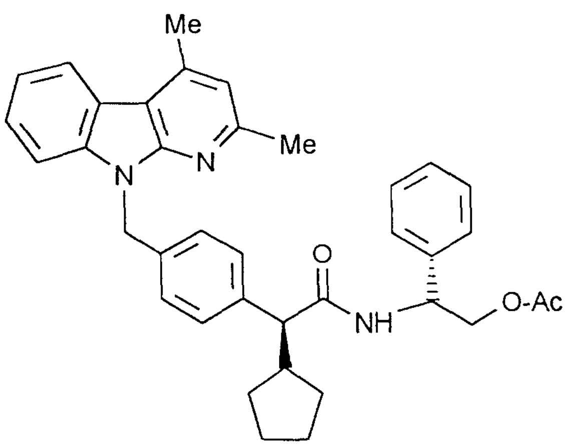

4,5 g (8,46 mmol) der Verbindung aus Beispiel Nr. 2 werden in 300 ml Dichlormethan suspendiert, mit 2,05 ml (25,4 mmol) Pyridin sowie 1,82 ml (25,4 mmol) Acetylchlorid in 30 ml Dichlormethan versetzt und 20 Stunden bei

20°C umgesetzt. Der Ansatz wird mit Puffer (Merck) vom pH = 2 und Wasser extrahiert, mit Natriumsulfat getrocknet und eingedampft. Nach dem Ausrühren mit Methanol und anschließendem Trocknen im Hochvakuum über Phosphorpentoxid fallen 3,6 g Produkt an.
$R_f$ = 0,62 (Petrolether : Essigester = 1: 1)

Die Verbindungen der Tabelle 16 werden analog der Vorschrift von Beispiel Nr. 142 hergestellt:

**Tabelle 16**

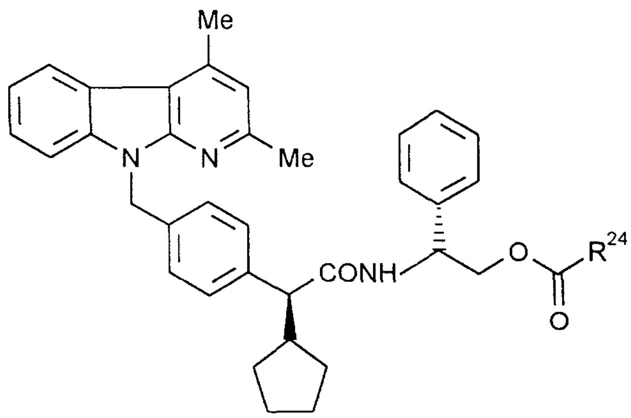

| Beispiel-Nr. | $R^{24}$ | Fp. [°C] $R_f$(Solvens | Ausgangs-Material aus Bsp.Nr. |
|---|---|---|---|
| 143 | -Et | 0,25 (D) | 2 |
| 144 | $-CH_2OAc$ | 0,29 (D) | 2 |
| 145 | $-CH_2OCH_2Ph$ | 0,27 (D) | 2 |
| 146 | cis-$(CH_2)_7$-Z-CH=CH-$(CH_2)_7$ $CH_3$ | 0,52 (D) | 2 |
| 147 | $-(CH_2)_{14}-CH_3$ | 0,69 (G) | 2 |
| 148 | -Ph | 0,65 (C) | 2 |
| 149 | 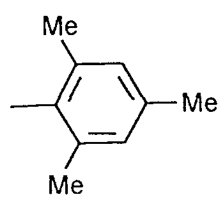 | | 2 |
| 150 | -tBu | 0,38 (C) | 2 |

**Beispiel Nr 151**

2-(S)-2-[4-(2,4-Dimethyl-α-carbolin-9-yl)-methyl-phenyl]-2-cyclopentyl-thioessigsäure-N-[1-(R)-1-phenyl-2-acetoxy-ethyl]amid

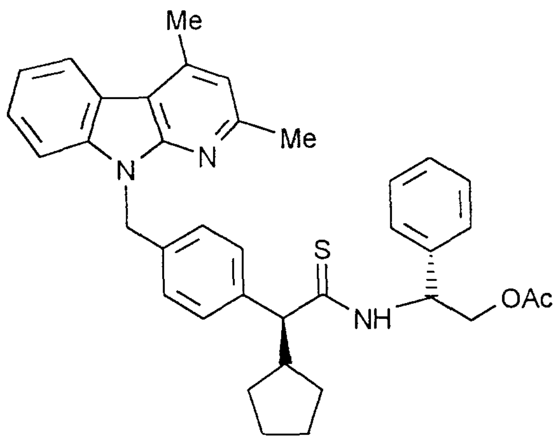

1,5 g (2,6 mmol) der Verbindung aus Beispiel Nr. 142 werden in 50 ml Dioxan mit 1,27 g (3,13 mmol) 2,4-Bis-(4-methoxyphenyl)-1,3-dithia-2,4-diphosphetan-2,4-disulfid (Lawesson's Reagenz) versetzt und 5 Stunden unter Rückfluß gekocht. Die Reaktionsmischung wird im Vakuum zur Trockne eingedampft und chromatographisch an Kieselgel MATREX$^{TR}$ Silica Si (Amicon, Grace Company / 20 μ / MPLC-Säule / Dichlormethan : Ethanol = 100 : 1) gereinigt; Ausbeute: 665 mg.
$R_f$ = 0,53 (Petrolether : Essigsäureethylester = 2: 1)
MS (FAB): m/z = 612 (4 %, $[M + Na]^+$), 590 (100 %, $[M + H]^+$), 529 (19 %, $M^+$ -AcOH).

**Beispiel Nr. 152**

2-(S)-2-[4-(2,4-Dimethyl-α-carbolin-9-yl)-methyl-phenyl]-2-cyclopentyl-essigsäure-N-[1-(R)-1-phenyl-2-[(2-hydroxy-acet)-oxy]-ethyl]amid

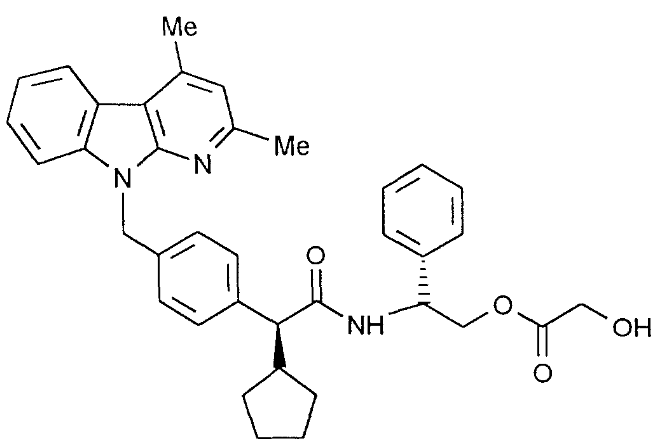

1,45 g (2,13 mmol) der Verbindung aus Beispiel 145 werden in 100 ml THF an Palladium (5 %ig auf Tierkohle) bei 20°C und Normaldruck Wasserstoff hydriert. Nach 18 Stunden saugt man den Ansatz über Kieselgur ab, wäscht

mehrfach mit Methanol und Dichlormethan und dampft die vereinigten organischen Lösungen ein. Der feste Rückstand wird mit Pentan verrührt, abgesaugt und im Hochvakuum vom Restlösemittel befreit.
$R_f$ = 0,31 (Petrolether : Essigester = 1 : 1)

**Beispiel Nr. 153**

2-(S)-2-[4-(2,4-Dimethyl-α-carbolin-9-yl)-methyl-phenyl)-2-cyclopentyl-thioessigsäure-N-[1-(R)-1-phenyl-2-hydroxy-ethyl]-amid

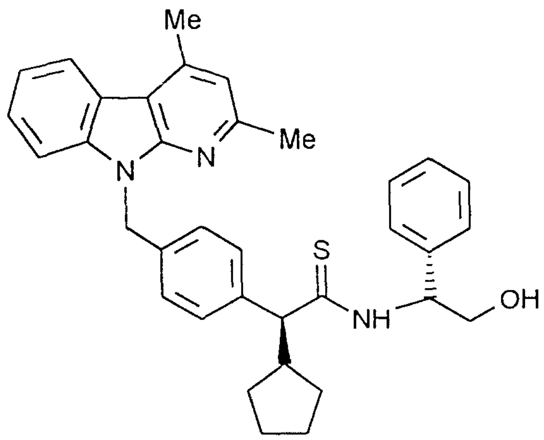

Die Titelverbindung wird analog der Synthesevorschrift aus Beispiel Nr. 73 aus der Verbindung von Beispiel Nr. 151 in DME als Lösungsmittel bei 20°C hergestellt.
$R_f$ = 0,24 (Dichlormethan : Ethanol = 50 : 1)

**Beispiel Nr. 154**

2-[4-(2,4-Dimethyl-α-carbolin-9-yl)-methyl-phenyl]-2-cyclopentyl-essigsäure-N-[1-(thien-2-yl)-1-methoxycarbonyl-methyl]-amid

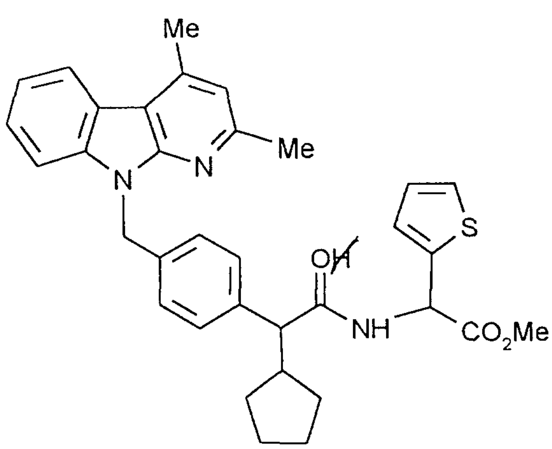

Die Titelverbindung wird analog der Synthesevorschrift aus Beispiel Nr. 1, 2 und 3 aus der Verbindung von Beispiel Nr. LXII und (R,S)-(Thien-2-yl)-glycinmethylester hergestellt.

$R_f$ = 0,67 (Dichlormethan : Ethanol = 20 : 1)

**Beispiel Nr. 155**

2-[4-(2,4-Dimethyl-α-carbolin-9-yl)-methyl-phenyl]-2-cyclopentyl-essigsäure-N-[1-(thien-2-yl)-2-hydroxy-ethyl]-amid

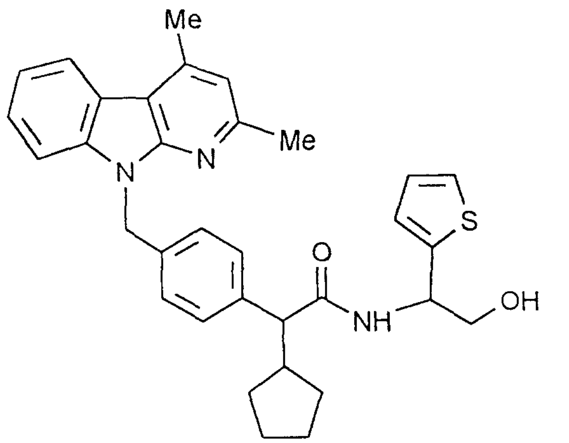

Die Titelverbindung wird analog der Synthesevorschrift von Beispiel Nr. 70 aus der Verbindung von Beispiel Nr. 154 hergestellt.
$R_f$ = 0,21 (Dichlormethan : Ethanol = 50 : 1)

**Beispiel Nr. 156**

2-(S)-2-[4-(2,4-Dimethyl-α-carbolin-9-yl)-methyl-phenyl]-2-cyclopentyl-essigsäure-N-[1-(R)-1-phenyl-2-(2,4,6-trimethyl-benzoyl-oxy)-ethyl]-amid

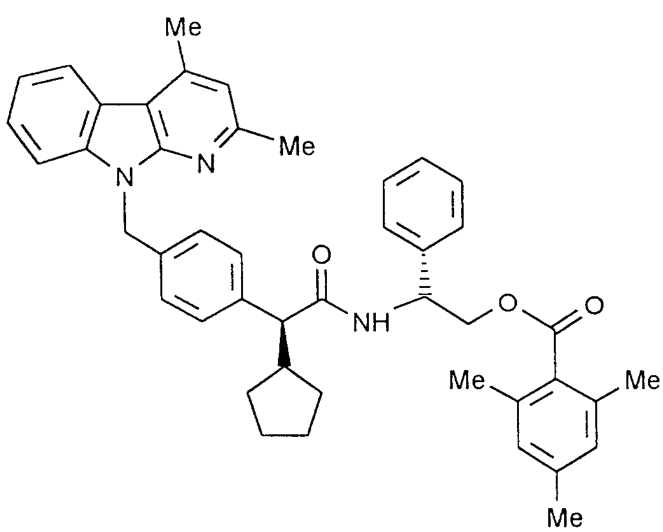

Analog der Vorschrift aus Beispiel Nr. 142 wird die Verbindung aus Beispiel Nr. 2 zur Titelverbindung umgesetzt.
$R_f$ = 0,26 (Laufmittel D)

**Beispiel Nr. 157**

2-(R,S)-2-[4-(2,4-Dimethyl-α-carbolin-9-yl)-methyl-phenyl]-2-cyclopentyl-essigsäure-[-(R,S)-1-phenyl-2-triphenylmethyloxy-ethyl]ester

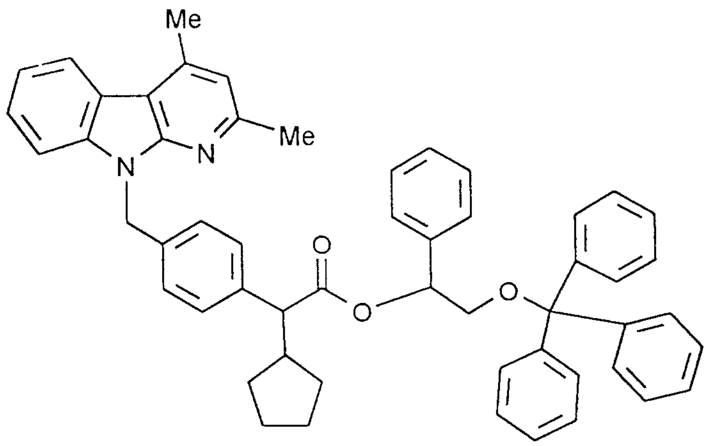

1,0 g (2,42 mmol) der Verbindung aus Beispiel Nr. LXI wird 2 h bei -30°C in 30 ml DMF mit 1 ml (7,27 mmol) Triethylamin und 206 µl (2,67 mmol) Mesylchlorid umgesetzt, darauf tropfenweise mit einer Lösung von 1,1 g (2,9 mmol) der Verbindung aus Beispiel Nr. CXI und 296 mg (2,42 mmol) DMAP in 10 ml DMF versetzt und ca. 20 h unter allmählicher Erwärmung auf 20°C nachgerührt. Zur Aufarbeitung rührt man in Ether/Wasser ein, trennt die Phasen, extrahiert die organische Phase mit wäßriger 1M Natronlauge und wäscht mit Wasser nach. Die organische Phase wird mit Magnesiumsulfat getrocknet und - zuletzt im Hochvakuum - eingedampft; Ausbeute: 1,0 g.
$R_f$ = 0,44 (Petrolether : Essigsäureethylester = 5 : 1)

**Beispiel Nr. 158 und 159**

2-(R,S)-2-[4-(2,4-Dimethyl-α-carbolin-9-yl)-methyl-phenyl]-2-cyclopentyl-essigsäure-[1-(R,S)-1-phenyl-2-triphenylmethyloxy-ethyl]ester

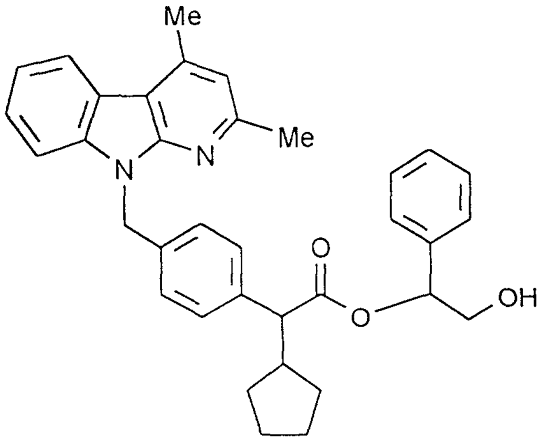

1,0 g (1,29 mmol) der Verbindung aus Beispiel Nr. 157 wird 48 h bei 20°C in 10 ml THF und 5 ml Wasser mit 5 ml Trifluoressigsäure gerührt. Darauf wird der Ansatz mit 300 ml Ether und 200 ml wäßriger Natriumhydrogencarbonatlösung verührt, die Phasen nach Abklingen der Kohlendioxidentwicklung getrennt und die organische Phase mit Puffer vom pH = 7 (Merck) extrahiert und mit Magnesiumsulfat getrocknet. Nach Abdampfen des Lösungsmittels fällt ein Rohprodukt an, das chromatographisch an Kieselgel (Merck / Petrolether : Essigsäureethylester = 5 : 1) gereinigt und in die Diastereomeren getrennt wird.

racemisches Diastereomer A)
Ausbeute: 300 mg
$R_f$ = 0,54 (Petrolether : Essigsäureethylester = 2 : 1)

racemisches Diastereomer B)
Ausbeute: 320 mg
$R_f$ = 0,42 (Petrolether : Essigsäureethylester = 2 : 1)

Die Verbindungen der Tabelle 17 werden analog der Vorschrift von Beispiel Nr. 1, 2 und 3 hergestellt:

**Tabelle 17**

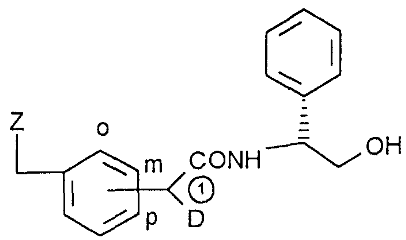

| Bsp.-Nr. | Z | Position (o, m oder p) | ① | D | $R_f$(Solvens) | MS | Ausgangsmaterial Bsp.-Nr. |
|---|---|---|---|---|---|---|---|
| 161 | 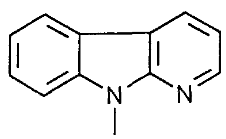 | p | rac | cPent | 0,35 (C) | FAB: 504 (95 %) | CXLII |
| 162 | 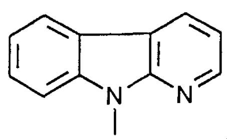 | p | S | cPent | 0,35 (C) | | CXLII |

| Bsp.-Nr. | Z | Position (o, m oder p) | ① | D | $R_f$(Solvens) | MS | Ausgangsmaterial Bsp.-Nr. |
|---|---|---|---|---|---|---|---|
| 163 | 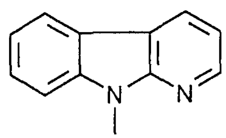 | p | R | cPent | (O,35 C) | | CXLII |
| 164 | 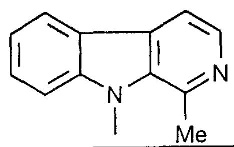 | p | rac | cPent | 0,23 /(C)<br>0,25 | FAB:<br>518 (51 %) | CXLIII |
| 165 | 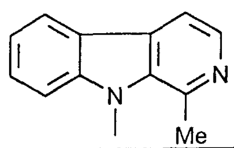 | p | R | cPent | 0,29 (C) | | CXLIII |
| 166 | 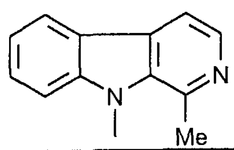 | p | S | cPent | 0,25 (C) | | CXLIII |
| 167 | 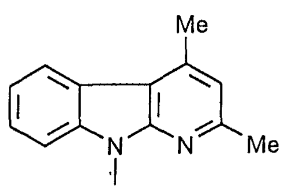 | m | rac | cPent | 0,40 (C) | FAB:<br>532 (100 % | CXLIV |

| Bsp.-Nr. | Z | Position (o, m oder p) | (1) | D | $R_f$(Solvens) | MS | Ausgangs-material Bsp.-Nr. |
|---|---|---|---|---|---|---|---|
| 168 | 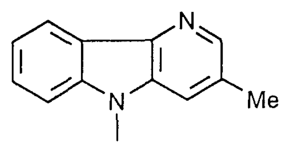 | p | rac | cPent | 0,26/ 0,22 (D) | FAB: 518 (100 %) | CVL |
| 169 | 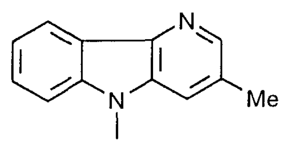 | p | | cPent | 0,26 (D) | | CVL |
| 170 | 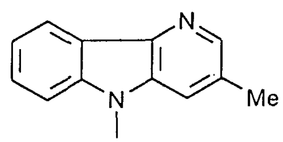 | p | | cPent | 0,22 (D) | | CVL |
| 171 | 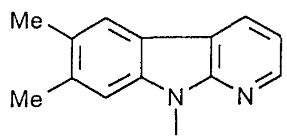 | p | rac | cPent | 0,37 (C) | | CVLI |
| 172 | 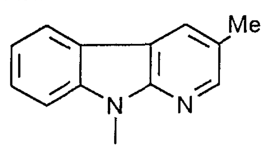 | p | rac | cPent | | | CVLII |

| Bsp.-Nr. | Z | Position (o, m oder p) | (1) | D | $R_f$(Solvens) | MS | Ausgangs-material Bsp.-Nr. |
|---|---|---|---|---|---|---|---|
| 173 | 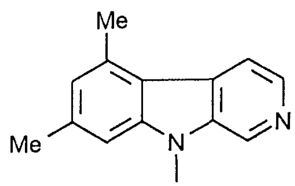 | p | rac | cPent | 0,19 (C) | FAB: 532 (100 %) | CVLIII |
| 174 | 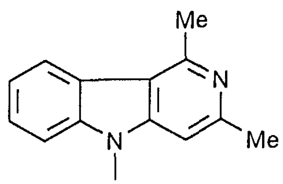 | p | rac | cPent | | | CIL |
| 175 | 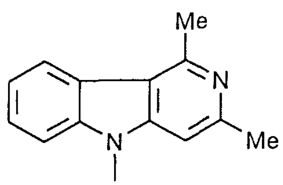 | p | | cPent | | | CIL |
| 176 | 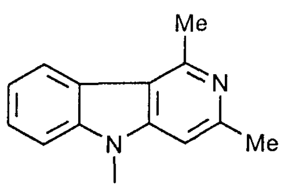 | p | | cPent | | | CIL |

| Bsp.-Nr. | Z | Position (o, m oder p) | ① | D | $R_f$(Solvens) | MS | Ausgangs-material Bsp.-Nr. |
|---|---|---|---|---|---|---|---|
| 177 | 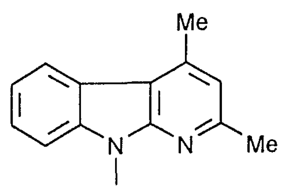 | p | rac | Et | | | Cl |
| 178 | 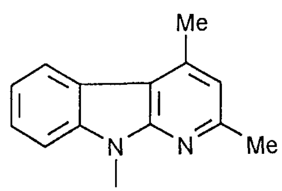 | p | rac | Me | | | CLI |
| 179 | 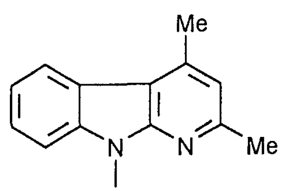 | p | rac | nPent | | | CLII |
| 180 | 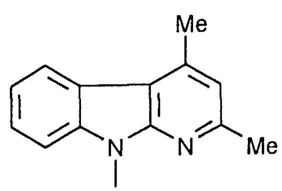 | p | diaA | nPent | | | CLII |

| Bsp.-Nr. | Z | Position (o, m oder p) | ① | D | $R_f$(Solvens) | MS | Ausgangs-material Bsp.-Nr. |
|---|---|---|---|---|---|---|---|
| 181 | 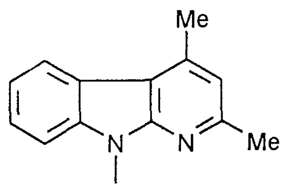 | p | diaB | nPent | | | CLII |
| 182 | 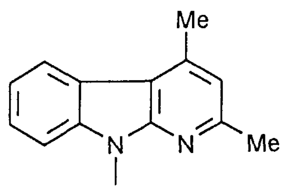 | p | rac | 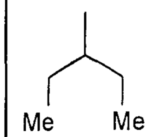 | | | CLIII |
| 183 | 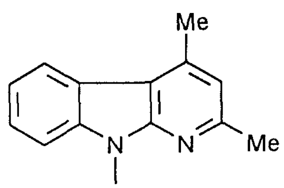 | p | diaA | 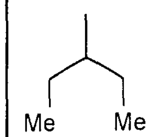 | | | CLIII |
| 184 | 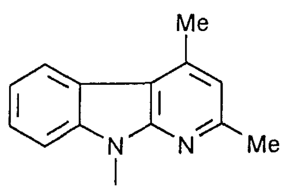 | p | diaB | 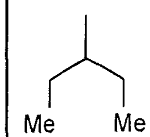 | | | CLIII |

**Beispiel 185**

2-(R,S)-2-[4-(2,4-Dimethyl-α-carbolin-9-yl)-methyl-phenyl]-2-cyclopentyl-essigsäure-[N-benzyl-N-benzoyl]-amid

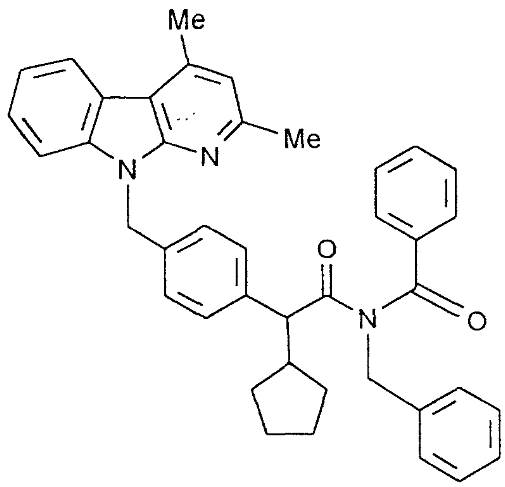

2,0 g (4,8 mmol) der Verbindung aus Beispiel-Nr. LXI werden 1 h in wasserfreiem DMF bei -30°C mit 0,74 ml (5,3 mmol) Triethylamin und 0,41 ml (5,3 mmol) Mesylchlorid umgesetzt. Darauf wird eine Lösung von 1,07 g (5,1 mmol) N-Benzyl-benzamid und 1,42 ml (10,2 mmol) Triethylamin in 10 ml wasserfreiem DMF bei -30°C zugetropft und unter allmählicher Erwärmung auf 20°C 16 h nachgerührt. Das Reaktionsgemisch wird mit Ether und Wasser verrührt, die Phasen getrennt und die wäßrige Phase jeweils nach Einstellen eines pH-Wertes von 4 und 7 nachgewaschen. Die vereinigten organischen Lösungen werden eingedampft und chromatographisch an Kieselgel 60 (Merck / zuerst "Dichlormethan : Ethanol : Ethanol = 60 : 1"; dann "Petrolether : Essigsäureethylester = 4 : 1") gereinigt.
$R_f$ = 0,58 (Petrolether : Essigsäureethylester = 2: 1)

**Beispiel 186**

2-(R,S)-2-[4-(2,4-Dimethyl-α-carbolin-9-yl)-methyl-phenyl]-2-cyclopentyl-essigsäure-[N-benzoyl]-amid

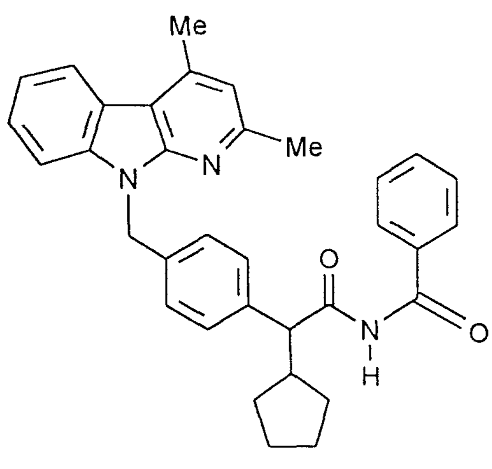

2,0 g (3,3 mmol) der Verbindung aus Beispiel Nr. 185 werden ca. 40 h in Dioxan an 2 g Palladium auf Tierkohle (5 %) bei 20°C unter einem Wasserstoffdruck von ca. 1 bar umgesetzt. Darauf wird über einen Seitz-Filter abgesaugt,

mit Dioxan 2,0 g (3,3 mmol) der Verbindung aus Beispiel Nr. 185 werden ca. 40 h in Dioxan an 2 g Palladium auf Tierkohle (5 %) bei 20°C unter einem Wasserstoffdruck von ca. 1 bar umgesetzt. Darauf wird über einen Seitz-Filter abgesaugt, mit Dioxan nachgewaschen und das Filtrat eingedampft. Das Rohprodukt wird bei 60°C mit Methanol ausgerührt und bei 20°C abgesaugt, mit kaltem Methanol gewaschen und im Vakuum über Phosphorpentoxid getrocknet.
$R_f$ = 0,49 (Petrolether : Essigsäureethylester = 2:1)

**Beispiel 187**

2-(R,S)-2-[4-(2,4-Dimethyl-α-carbolin-9-yl)-methyl-phenyl]-2-cyclopentyl-essigsäure-N-[ 1-(R,S)-1-phenyl-1-ethoxy-1-ethoxycarbonyl-methyl]-amid

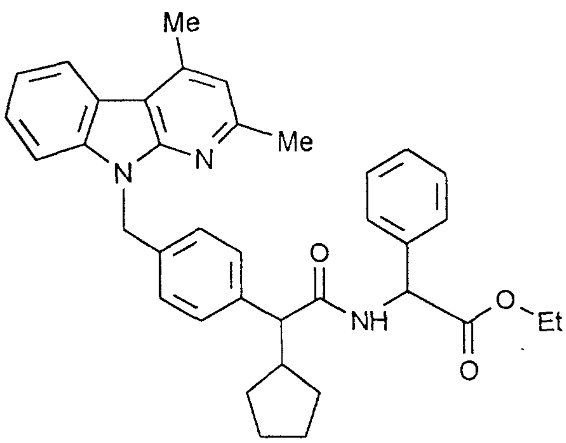

Analog der Vorschrift aus Beispiel Nr. 1, 2 und 3 wird die Verbindung aus Beispiel Nr. LXI zur Titelverbindung umgesetzt.

**Beispiel 188**

2-(R,S)-2-[4-(2,4-Dimethyl-α-carbolin-9-yl)-methyl-phenyl]-2-cyclopentyl-essigsäure-N-[1-(R,S)-1-phenyl-1-carboxy-methyl]-amid

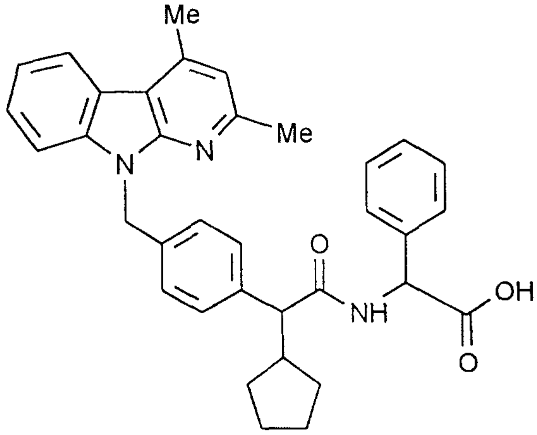

Analog der Vorschrift aus Beispiel Nr. 73 wird die Verbindung aus Beispiel Nr. 187 zur Titelverbindung umgesetzt.

**Beispiel 189**

2-(R,S)-2-[4-(2,4-Dimethyl-α-carbolin-9-yl)-methyl-phenyl]-2-cyclopentylthioessigsäure-[1-(R,S)-2-hydroxy-1-phenylethyl]-ester

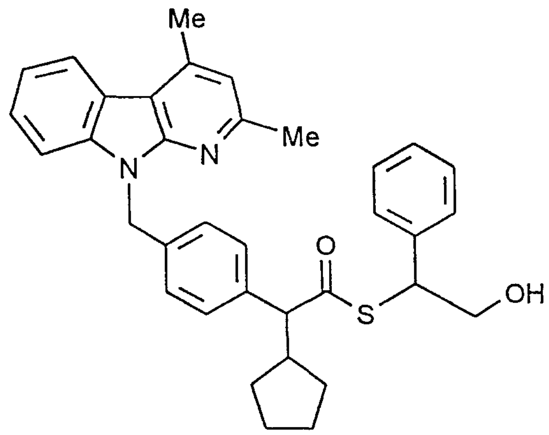

1 g (2,42 mmol) der Verbindung aus Beispiel Nr. LXI werden bei -30°C in 30 ml DMF 1 h mit 1 ml (7,27 mmol) Triethylamin und 206 µl (2,67 mmol) Mesylchlorid umgesetzt. Dann tropft man bei der genannten Temperatur eine Lösung von 1-(R,S)-1-Phenyl-2-hydroxy-thioethanol in 10 ml DMF zu und rührt eine weitere Stunde nach. Zur Aufarbeitung rührt man die Reaktionsmischung in Ether und wäßrige Natriumhydrogencarbonatlösung ein. Die organische Phase wird mit Puffer vom pH = 2 und daruf pH = 7 nachgewaschen, mit Magnesiumsulfat getrocknet und eingedampft. Das roh Produkt wird an Kieselgel 60 (Merck / Petrolether : Essigsäureethylester = 5 : 1) aufgereinigt; Ausbeute: 660 mg $R_f$ = 0,58 (Petrolether : Essigsäureethylester = 2: 1)

## Patentansprüche

1. Cycloalkano-indol- und -azaindol-Derivate der allgemeinen Formel (I)

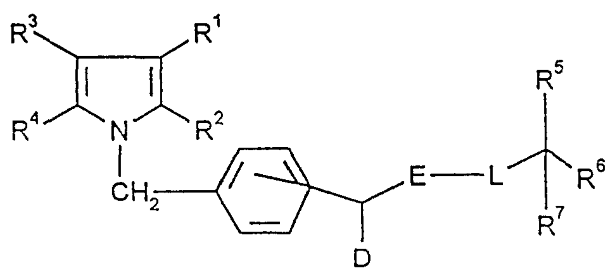

(I),

in welcher

$R^1$ und $R^2$ unter Einbezug der sie verbindenden Doppelbindung gemeinsam einen Phenyl- oder Pyridylring oder einen Ring der Formel

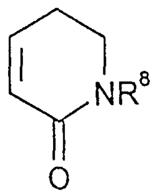

bilden,
worin

$R^8$ Wasserstoff oder geradkettiges oder verzweigtes Alkyl mit bis zu 4 Kohlenstoffatomen bedeutet,

$R^3$ und $R^4$ unter Einbezug der sie verbindenden Doppelbindung gemeinsam einen Phenylring oder einen 4- bis 8-gliedrigen Cycloalken- oder Oxocycloalken-Rest bilden,
wobei alle unter $R^1$/$R^2$ und $R^3$/$R^4$ aufgeführten Ringsysteme gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,

D für Cycloalkyl mit 4 bis 12 Kohlenstoffatomen oder für geradkettiges oder verzweigtes Alkyl mit bis zu 12 Kohlenstoffatomen steht,

E für die -CO- oder -CS-Gruppe steht,

L für ein Sauerstoff- oder Schwefelatom steht oder für eine Gruppe der Formel $-NR^9$ steht,
worin

$R^9$ Wasserstoff oder geradkettiges oder verzweigtes Alkyl mit bis zu 6 Kohlenstoffatomen steht, das gegebenenfalls durch Hydroxy oder Phenyl substituiert ist,

$R^5$ für Phenyl oder für einen 5- bis 7-gliedrigen gesättigten oder ungesättigten Heterocyclus mit bis zu 3 Heteroatomen aus der Reihe S, N und/oder O steht,
wobei die Cyclen gegebenenfalls bis zu 3-fach gleich oder verschieden durch Nitro, Carboxy, Halogen, Cyano oder durch geradkettiges oder verzweigtes Alkenyl oder Alkoxycarbonyl mit jeweils bis zu 6 Kohlenstoffatomen oder durch geradkettiges oder verzweigtes Alkyl mit bis zu 6 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 6 Kohlenstoffatomen substituiert ist,
und/oder die Cyclen gegebenenfalls durch eine Gruppe der Formel $-OR^{10}$ oder $-NR^{11}R^{12}$ substituiert sind,
worin

$R^{10}$ Wasserstoff oder geradkettiges oder verzweigtes Alkyl oder Alkenyl mit jeweils bis zu 6 Kohlenstoffatomen bedeutet,
$R^{11}$ bzw. $R^{12}$ gleich oder verschieden sind und Phenyl, Wasserstoff oder geradkettiges oder verzweigtes Alkyl mit bis zu 6 Kohlenstoffatomen bedeuten
oder geradkettiges oder verzweigtes Acyl mit bis zu 8 Kohlenstoffatomen bedeuten, das gegebenenfalls durch eine Gruppe der Formel $-NR^{13}R^{14}$ substituiert ist,
worin

$R^{13}$ und $R^{14}$ gleich oder verschieden sind und Wasserstoff oder geradkettiges oder verzweigtes Acyl mit bis zu 8 Kohlenstoffatomen bedeuten,

$R^6$ für Wasserstoff, Carboxy oder für geradkettiges oder verzweigtes Alkoxycarbonyl mit bis zu 5 Kohlenstoffatomen steht, oder für geradkettiges oder verzweigtes Alkyl mit bis zu 6 Kohlenstoffatomen steht, das gegebenenfalls durch Hydroxyl oder durch eine Gruppe der Formel $-O-CO-R^{15}$ substituiert ist,
worin

$R^{15}$ Phenyl bedeutet, das gegebenenfalls bis zu 3-fach gleich oder verschieden durch Halogen, Hydroxy oder durch geradkettiges oder verzweigtes Alkyl mit bis zu 5 Kohlenstoffatomen substituiert ist,
oder geradkettiges oder verzweigtes Alkyl oder Alkenyl mit jeweils bis zu 22 Kohlenstoffatomen bedeutet, die gegebenenfalls durch eine Gruppe der Formel $-OR^{16}$ substituiert sind,
worin

$R^{16}$ Wasserstoff, Benzyl, Triphenylmethyl oder geradkettiges oder verzweigtes Acyl mit bis zu 6 Kohlenstoffatomen bedeutet,

$R^7$ für Wasserstoff steht oder

$R^6$ und $R^7$ gemeinsam für die Gruppe der Formel =O stehen,

gegebenenfalls in einer isomeren Form und deren Salze.

**2.** Cycloalkano-indol- und -azaindol-Derivate der Formel nach Anspruch 1
worin

$R^1$ und $R^2$ unter Einbezug der sie verbindenden Doppelbindung gemeinsam einen Phenyl- oder Pyridylring oder einen Ring der Formel

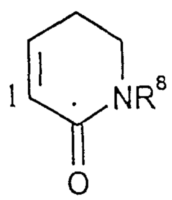

bilden,
worin

$R^8$ Wasserstoff oder geradkettiges oder verzweigtes Alkyl mit bis zu 3 Kohlenstoffatomen bedeutet,

$R^3$ und $R^4$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
wobei alle unter $R^1/R^2$ und $R^3/R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert sein kann,

D für Cyclobutyl, Cyclopentyl, Cyclohexyl, Cycloheptyl, Cyclooctyl oder für geradkettiges oder verzweigtes Alkyl mit bis zu 10 Kohlenstoffatomen steht,

E für die -CO- oder -CS-Gruppe steht,

L für ein Sauerstoff- oder Schwefelatom steht oder für eine Gruppe der Formel $-NR^9$ steht, worin

$R^9$ Wasserstoff oder geradkettiges oder verzweigtes Alkyl mit bis zu 5 Kohlenstoffatomen steht, das gegebenenfalls durch Hydroxy oder Phenyl substituiert ist,

$R^5$ für Phenyl, Pyridyl, Furyl, Thienyl oder Imidazolyl steht, die gegebenenfalls bis zu 2-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 4 Kohlenstoffatomen oder durch geradkettiges oder verzweigtes Alkyl mit bis zu 5 Kohlenstoffatomen substituiert sind, das gegebenenfalls durch Hydroxy, Carboxy oder durch geradkettiges oder verzweigtes Alkoxy oder Alkoxycarbonyl mit jeweils bis zu 5 Kohlenstoffatomen substituiert ist,
und/oder die Cyclen gegebenenfalls durch eine Gruppe der Formel $-OR^{10}$ oder $-NR^{11}R^{12}$ substituiert sind,
worin

$R^{10}$ Wasserstoff oder geradkettiges oder verzweigtes Alkyl oder Alkenyl mit jeweils bis zu 4 Kohlenstoffatomen bedeutet,

$R^{11}$ und $R^{12}$ gleich oder verschieden sind und Phenyl, Wasserstoff oder geradkettiges oder verzweigtes Alkyl mit bis zu 5 Kohlenstoffatomen bedeuten,
oder geradkettiges oder verzweigtes Acyl mit bis zu 6 Kohlenstoffatomen bedeuten, das gegebenenfalls durch eine Gruppe der Formel $-NR^{13}R^{14}$ subsituiert ist,
worin

$R^{13}$ und $R^{14}$ gleich oder verschieden sind und Wasserstoff oder geradkettiges oder verzweigtes Acyl mit bis zu 6 Kohlenstoffatomen bedeuten,

$R^6$ für Wasserstoff, Carboxy oder für geradkettiges oder verzweigtes Alkoxycarbonyl mit bis zu 4 Kohlenstoffatomen steht, oder für geradkettiges oder verzweigtes Alkyl mit bis zu 5 Kohlenstoffatomen steht, das gegebenenfalls durch Hydroxyl oder durch eine Gruppe der Formel $-O-CO-R^{15}$ substituiert ist,
worin

$R^{15}$ Phenyl bedeutet, das gegebenenfalls bis zu 3-fach gleich oder verschieden durch Fluor, Chlor, Brom, Hydroxy oder durch geradkettiges oder verzweigtes Alkyl bis zu 4 Kohlenstoffatomen substituiert ist,
oder geradkettiges oder verzweigtes Alkyl oder Alkenyl mit jeweils bis zu 20 Kohlenstoffatomen bedeutet, die gegebenenfalls durch eine Gruppe der Formel $-OR^{16}$ substituiert sind,
worin

$R^{16}$ Wasserstoff, Benzyl, Triphenylmethyl oder geradkettiges oder verzweigtes Acyl mit bis zu 5 Kohlenstoffatomen bedeutet,

$R^7$ für Wasserstoff steht oder

$R^6$ und $R^7$ gemeinsam für die Gruppe der Formel =O stehen,

gegebenenfalls in einer isomeren Form und deren Salze.

**3.** Cycloalkano-indol- und -azaindol-Derivate der Formel nach Anspruch 1
worin

$R^1$ und $R^2$ unter Einbezug der sie verbindenden Doppelbindung gemeinsam einen Phenyl- oder Pyridylring oder einen Ring der Formel

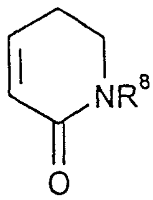

bilden,
worin

$R^8$ Wasserstoff oder Methyl bedeutet,

$R^3$ und $R^4$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
wobei alle unter $R^1$/$R^2$ und $R^3$/$R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert sein kann,

D für Cyclopentyl, Cyclohexyl, Cycloheptyl, Cylcooctyl oder für geradkettiges oder verzweigtes Alkyl mit bis zu 6 Kohlenstoffatomen steht,

E für die -CO- oder -CS-Gruppe steht,

L für ein Sauerstoff- oder Schwefelatom steht oder für eine Gruppe der Formel $-NR^9$ steht,
worin

$R^9$ Wasserstoff oder geradkettiges oder verzweigtes Alkyl mit bis zu 4 Kohlenstoffatomen steht, das gegebenenfalls durch Hydroxy oder Phenyl substituiert ist,

$R^5$ für Phenyl, Pyridyl oder Thienyl steht, die gegebenenfalls bis zu 2-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 3 Kohlenstoffatomen oder durch geradkettiges oder verzweigtes Alkyl mit bis zu 4 Kohlenstoffatomen substituiert sind, das gegebenenfalls durch Hydroxy, Carboxy oder durch geradkettiges oder. verzweigtes Alkoxy oder Alkoxycarbonyl mit jeweils bis zu 4 Kohlenstoffatomen substituiert ist,
und/oder die Cyclen gegebenenfalls durch eine Gruppe der Formel - $OR^{10}$ oder $-NR^{11}R^{12}$ substituiert sind,
worin

$R^{10}$ Wasserstoff oder geradkettiges oder verzweigtes Alkyl oder Alkenyl mit jeweils bis zu 3 Kohlenstoffatomen bedeutet,

$R^{11}$ und $R^{12}$ gleich oder verschieden sind und Phenyl, Wasserstoff oder geradkettiges oder verzweigtes Alkyl mit bis zu 4 Kohlenstoffatomen bedeuten,
oder geradkettiges oder verzweigtes Acyl mit bis zu 5 Kohlenstoffatomen bedeuten, das gegebenenfalls durch eine Gruppe der Formel $-NR^{13}R^{14}$ substituiert ist,
worin

$R^{13}$ und $R^{14}$ gleich oder verschieden sind und Wasserstoff oder geradkettiges oder verzweigtes Acyl mit bis zu 5 Kohlenstoffatomen bedeuten,

$R^6$ für Wasserstoff, Carboxy oder für geradkettiges oder verzweigtes Alkoxycarbonyl mit bis zu 3 Kohlenstoffatomen steht,

oder für geradkettiges oder verzweigtes Alkyl mit bis zu 4 Kohlenstoffatomen steht, das gegebenenfalls durch Hydroxyl oder durch eine Gruppe der Formel -O-CO-$R^{15}$ substituiert ist,
worin

$R^{15}$ Phenyl bedeutet, das gegebenenfalls bis zu 3-fach gleich oder verschieden durch geradkettiges oder verzweigtes Alkyl mit bis zu 3 Kohlenstoffatomen substituiert ist,
oder geradkettiges oder verzweigtes Alkyl oder Alkenyl mit jeweils bis zu 19 Kohlenstoffatomen bedeutet, die gegebenenfalls durch eine Gruppe der Formel -$OR^{16}$ substituiert sind,
worin

$R^{16}$ Wasserstoff, Benzyl, Triphenylmethyl oder geradkettiges oder verzweigtes Acyl mit bis zu 4 Kohlenstoffatomen bedeutet,

$R^7$ für Wasserstoff steht oder

$R^6$ und $R^7$ gemeinsam für die Gruppe der Formel =O stehen,

gegebenenfalls in einer isomeren Form und deren Salze.

**4.** 2-*[*4-(2,4-Dimethyl-5,6,7,8-tetrahydro-α-carbolin-9-yl)methyl-phenyl*]*-2-cyclopentyl-essigsäure N-*[*(R)-phenylglycinolamid*]* der Formel

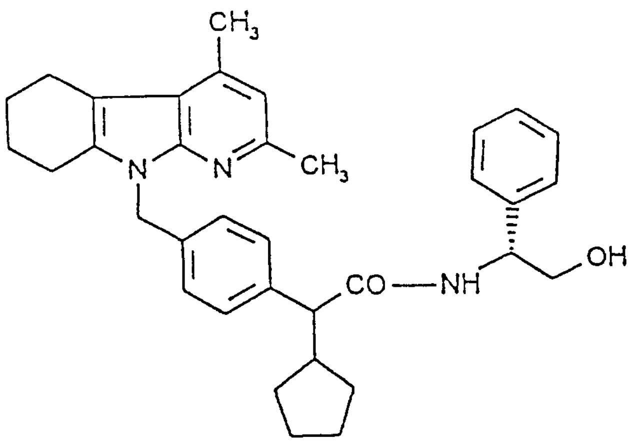

und dessen Salze.

**5.** 2-*[*4-(2,4-Dimethyl-α-carbolin-9-yl)methyl-phenyl*]*-2-cyclopentyl-essigsäure N-*[*(R)-phenylglycinolamid*]* der Formel

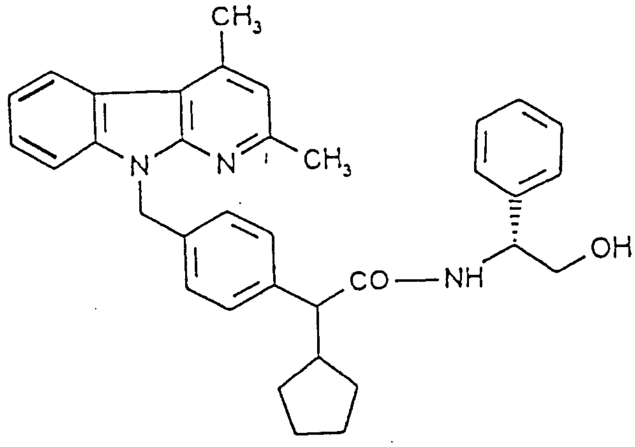

und dessen Salze.

**6.** 2-(R,S)-2-/4-(2,4-Dimethyl-α-carbolin-9-yl)methylphenyl/-2-cyclopentyl-essigsäure-/1-(R,S)-1-phenyl-2-hydroxy-ethyl/ester der Formel

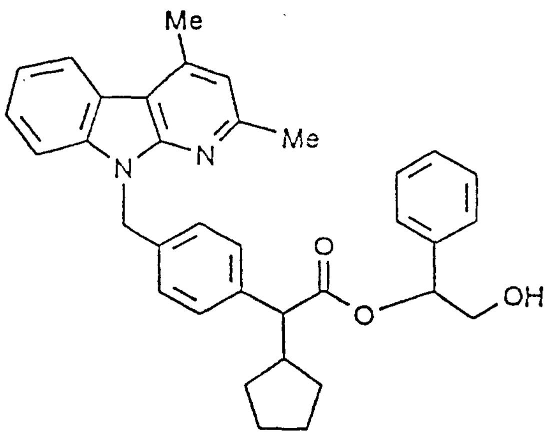

und dessen Salze.

**7.** 2-(R,S)-2-/4-(2,4-Dimethyl-α-carbolin-9-yl)-methylphenyl]-2-cyclopentyl-essigsäure [N-(1-(R,S)-1-phenyl-1 carboxy-methyl]-amid der Formel

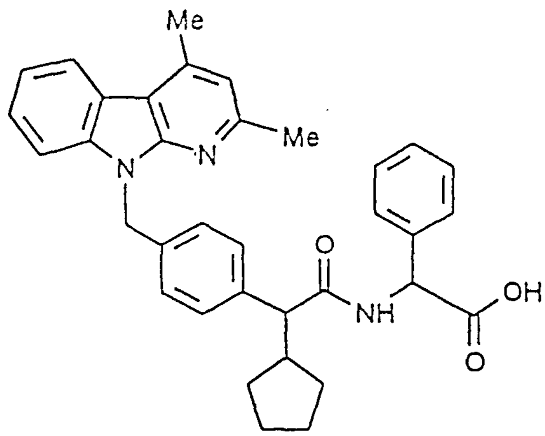

und dessen Salze.

8. Cycloalkano-indol- und -azaindol-Derivate gemäß einem der Ansprüche 1 bis 7 zur therapeutischen Anwendung.

9. Verfahren zur Herstellung von Cycloalkano-indol- und -azaindol-Derivaten gemäß einem der Ansprüche 1 bis 7, **dadurch gekennzeichnet daß** man
Carbonsäuren der allgemeinen Formel (II)

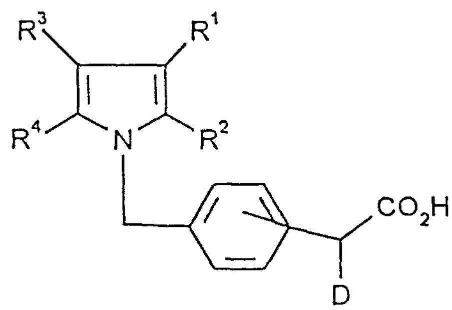

(II)

in welcher
$R^1$, $R^2$, $R^3$, $R^4$ und D die in Anspruch 1 angegebenen Bedeutungen haben,
mit Verbindungen der allgemeinen Formel (III)

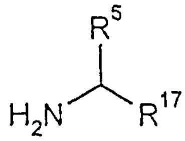

(III)

in welcher

$R^5$ die in Anspruch 1 angegebene Bedeutung hat
und

$R^{17}$ die angegebene Bedeutung von $R^6$ hat, aber nicht für Carboxy steht,

in einem inerten Lösemittel und in Anwesenheit von Basen und/oder Hilfsstoffen amidiert,

und gegebenenfalls funktionelle Gruppen durch Hydrolyse, Veresterung oder Reduktion variiert.

**10.** Arzneimittel enthaltend mindestens ein Cycloalkano-indol- oder -azaindol-Derivat gemäß einem der Ansprüche 1 bis 7.

**11.** Verwendung von Cycloalkano-indol- und -azaindol-Derivaten gemäß einer der Ansprüche 1 bis 7 zur Herstellung von Arzneimitteln.

**12.** Carbonsäuren der allgemeinen Formel (II)

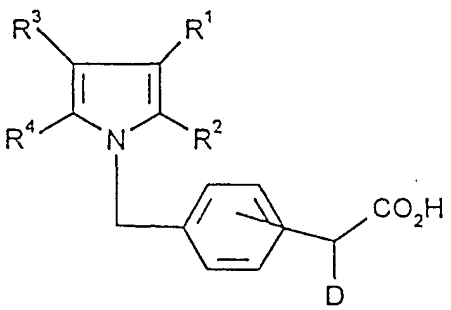

(II)

in welcher

$R^1$ und $R^2$ unter Einbezug der sie verbindenden Doppelbindung gemeinsam einen Phenyl- oder Pyridylring oder einen Ring der Formel

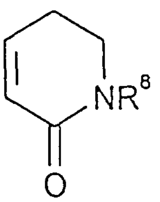

bilden,

worin

$R^8$ Wasserstoff oder geradkettiges oder verzweigtes Alkyl mit bis zu 4 Kohlenstoffatomen bedeutet,

$R^3$ und $R^4$ unter Einbezug der sie verbindenden Doppelbindung gemeinsam einen Phenylring oder einen 4- bis 8-gliedrigen Cycloalken- oder Oxocycloalken-Rest bilden,
wobei alle unter $R^1$/$R^2$ und $R^3$/$R^4$ aufgeführten Ringsysteme gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,

D für Cycloalkyl mit 4 bis 12 Kohlenstoffatomen oder für geradkettiges oder verzweigtes Alkyl mit bis zu 12 Kohlenstoffatomen steht.

**13.** Verfahren zur Herstellung von Carbonsäuren nach Anspruch 12, **dadurch gekennzeichnet, daß** man Verbindungen der allgemeinen Formel (IV)

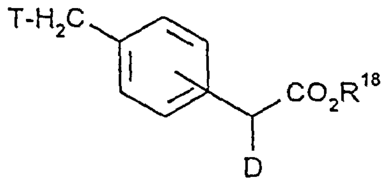

(IV)

in welcher

D die in Anspruch 12 angegebene Bedeutung hat,

T für eine typische Abgangsgruppe wie beispielsweise Chlor, Brom, Jod, Tosylat oder Mesylat, vorzugsweise für Brom steht,
und

$R^{18}$ für ($C_1$-$C_4$)-Alkyl steht,

mit Verbindungen der allgemeinen Formel (V)

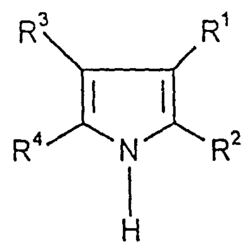

(V)

in welcher

$R^1$, $R^2$, $R^3$ und $R^4$ die in Anspruch 12 angegebenen Bedeutungen haben

in inerten Lösemitteln, gegebenenfalls in Anwesenheit einer Base, umsetzt.

**Claims**

1. Cycloalkano-indole and -azaindole derivatives of the general formula (I)

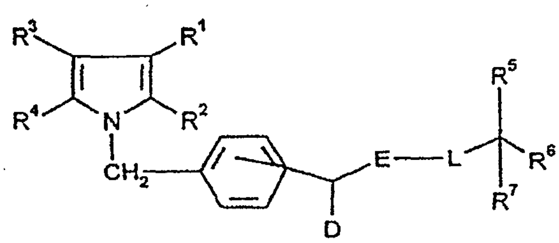

(I),

in which

$R^1$ and $R^2$, including the double bond connecting them, together form a phenyl or pyridyl ring or a ring of the formula

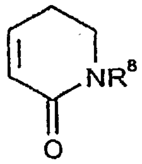

wherein

$R^8$ denotes hydrogen or straight-chain or branched alkyl having up to 4 carbon atoms,

$R^3$ and $R^4$, including the double bond connecting them, together form a phenyl ring or a 4- to 8-membered cycloalkene or oxocycloalkene radical,
or ring systems mentioned under $R^1/R^2$ and $R^3/R^4$ optionally being substituted up to 3 times by identical or different halogen, trifluoromethyl, carboxyl or hydroxyl substituents, by straight-chain or branched alkoxy or alkoxycarbonyl each having up to 6 carbon atoms or by straight-chain or branched alkyl having up to 6 carbon atoms, which, for its part, can be substituted by hydroxyl or by straight-chain or branched alkoxy having up to 4 carbon atoms,

D represents cycloalkyl having 4 to 12 carbon atoms or straight-chain or branched alkyl having up to 12 carbon atoms,

E represents the -CO- or -CS- group,

L represents an oxygen or sulphur atom or a group of the formula $-NR^9$,
wherein

$R^9$ denotes hydrogen or straight-chain or branched alkyl having up to 6 carbon atoms, which is optionally substituted by hydroxyl or phenyl,

$R^5$ represents phenyl or a 5- to 7-membered saturated or unsaturated heterocycle having up to 3 heteroatoms from the series consisting of S, N and/or O,
the cycles optionally being substituted up to 3 times by identical or different nitro, carboxyl, halogen or cyano substituents or by straight-chain or branched alkenyl or alkoxycarbonyl each having up to 6 carbon atoms or by straight-chain or branched alkyl having up to 6 carbon atoms, which is optionally substituted by hydroxyl, carboxyl or by straight-chain or branched alkoxy or alkoxycarbonyl each having up to 6 carbon atoms,
and/or the cycles optionally being substituted by a group of the formula $-OR^{10}$ or $-NR^{11}R^{12}$,
wherein

$R^{10}$ denotes hydrogen or straight-chain or branched alkyl or alkenyl each having up to 6 carbon atoms,

$R^{11}$ and $R^{12}$ are identical or different and denote phenyl, hydrogen or straight-chain or branched alkyl having up to 6 carbon atoms or straight-chain or branched acyl having up to 8 carbon atoms, which is optionally substituted by a group of the formula $-NR^{13}R^{14}$,
wherein

$R^{13}$ and $R^{14}$ are identical or different and denote hydrogen or straight-chain or branched acyl having up to 8 carbon atoms,

$R^6$ represents hydrogen, carboxyl or straight-chain or branched alkoxycarbonyl having up to 5 carbon atoms,
or represents straight-chain or branched alkyl having up to 6 carbon atoms, which is optionally substituted by hydroxyl or by a group of the formula $-O-CO-R^{15}$,
wherein

$R^{15}$ denotes phenyl which is optionally substituted up to 3 times by identical or different halogen or hydroxyl substituents or by straight-chain or branched alkyl having up to 5 carbon atoms, or straight-chain or branched alkyl or alkenyl each having up to 22 carbon atoms, each of which is optionally substituted by a group of the formula $-OR^{16}$, wherein

$R^{16}$ is hydrogen, benzyl, triphenylmethyl or straight-chain or branched acyl having up to 6 carbon atoms,

$R^7$ represents hydrogen or

$R^6$ and $R^7$ together represent the group of the formula =O,

if appropriate in an isomeric form, and their salts.

**2.** Cycloalkano-indole and -azaindole derivatives of the formula according to Claim 1 wherein

$R^1$ and $R^2$, including the double bond connecting them, together form a phenyl or pyridyl ring or a ring of the formula

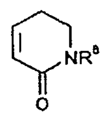 ,

wherein

$R^8$ denotes hydrogen or straight-chain or branched alkyl having up to 3 carbon atoms,

$R^3$ and $R^4$, including the double bond connecting them, together form a phenyl ring or a cyclopentene, cyclohexene, cycloheptene, cyclooctene, oxocyclopentene, oxocyclohexene, oxocycloheptene or oxocyclooctene radical,
or ring systems mentioned under $R^1/R^2$ and $R^3/R^4$ optionally being substituted up to 2 times by identical or different fluorine, chlorine, bromine, trifluoromethyl, carboxyl or hydroxyl substituents, by straight-chain or branched alkoxy or alkoxycarbonyl each having up to 4 carbon atoms or by straight-chain or branched alkyl having up to 4 carbon atoms, which, in turn, can be substituted by hydroxyl or by straight-chain or branched alkoxy having up to 3 carbon atoms,

D represents cyclobutyl, cyclopentyl, cyclohexyl, cycloheptyl, cyclooctyl or straight-chain or branched alkyl having up to 10 carbon atoms,

E represents the -CO- or -CS- group,

L represents an oxygen or sulphur atom or represents a group of the formula $-NR^9$, wherein

$R^9$ denotes hydrogen or straight-chain or branched alkyl having up to 5 carbon atoms, which is optionally substituted by hydroxyl or phenyl,

$R^5$ represents phenyl, pyridyl, furyl, thienyl or imidazolyl, each of which is optionally substituted up to 2 times by identical or different nitro, carboxyl, fluorine, chlorine, bromine or cyano substituents, by straight-chain or branched alkenyl or alkoxy carbonyl each having up to 4 carbon atoms or by straight-chain or branched alkyl having up to 5 carbon atoms, which is optionally substituted by hydroxyl, carboxyl or by straight-chain or branched alkoxy or alkoxycarbonyl each having up to 5 carbon atoms,
and/or the cycles are optionally substituted by a group of the formula $-OR^{10}$ or $-NR^{11}R^{12}$,

wherein

$R^{10}$ denotes hydrogen or straight-chain or branched alkyl or alkenyl each having up to 4 carbon atoms,

$R^{11}$ and $R^{12}$ are identical or different and denote phenyl, hydrogen or straight-chain or branched alkyl having up to 5 carbon atoms or denote straight-chain or branched acyl having up to 6 carbon atoms, which is optionally substituted by a group of the formula $-NR^{13}R^{14}$,
wherein

$R^{13}$ and $R^{14}$ are identical or different and denote hydrogen or straight-chain or branched acyl having up to 6 carbon atoms,

$R^6$ represents hydrogen, carboxyl or straight-chain or branched alkoxycarbonyl having up to 4 carbon atoms,
or represents straight-chain or branched alkyl having up to 5 carbon atoms, which is optionally substituted by hydroxyl or by a group of the formula $-O-CO-R^{15}$,
wherein

$R^{15}$ denotes phenyl which is optionally substituted up to 3 times by identical or different fluorine, chlorine, bromine or hydroxyl substituents or by straight-chain or branched alkyl having up to 4 carbon atoms,
or straight-chain or branched alkyl or alkenyl each having up to 20 carbon atoms, each of which is optionally substituted by a group of the formula $-OR^{16}$,
wherein

$R^{16}$ is hydrogen, benzyl, triphenylmethyl or straight-chain or branched acyl having up to 5 carbon atoms,

$R^7$ represents hydrogen or

$R^6$ and $R^7$ together represent the group of the formula =O,

if appropriate in an isomeric form, and their salts.

3. Cycloalkano-indole and -azaindole derivatives of the formula according to Claim 1
wherein

$R^1$ and $R^2$, including the double bond connecting them, together form a phenyl or pyridyl ring or a ring of the formula

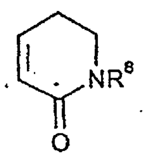
,

wherein

$R^8$ denotes hydrogen or methyl,

$R^3$ and $R^4$, including the double bond connecting them, together form a phenyl ring or a cyclopentene, cyclohexene, cycloheptene, cyclooctene, oxocyclopentene, oxocyclohexene, oxocycloheptene or oxocyclooctene radical,
or ring systems mentioned under $R^1/R^2$ and $R^3/R^4$ optionally being substituted up to 2 times by

identical or different fluorine, chlorine, bromine, trifluoromethyl, carboxyl or hydroxyl substituents, by straight-chain or branched alkoxy or alkoxycarbonyl each having up to 3 carbon atoms or by straight-chain or branched alkyl having up to 3 carbon atoms, which, for its part, can be substituted by hydroxyl, methoxy or ethoxy,

D represents, cyclopentyl, cyclohexyl, cycloheptyl, cyclooctyl or straight-chain or branched alkyl having up to 6 carbon atoms,

E represents the -CO- or -CS- group,

L represents an oxygen or sulphur atom or represents a group of the formula $-NR^9$, wherein

$R^9$ denotes hydrogen or straight-chain or branched alkyl having up to 4 carbon atoms, which is optionally substituted by hydroxyl or phenyl,

$R^5$ represents phenyl, pyridyl or thienyl, each of which is optionally substituted up to 2 times by identical or different nitro, carboxyl, fluorine, chlorine, bromine or cyano substituents, by straight-chain or branched alkenyl or alkoxycarbonyl each having up to 3 carbon atoms or by straight-chain or branched alkyl having up to 4 carbon atoms, which is optionally substituted by hydroxyl, carboxyl or by straight-chain or branched alkoxy or alkoxycarbonyl each having up to 4 carbon atoms, and/or the cycles are optionally substituted by a group of the formula $-OR^{10}$ or $-NR^{11}R^{12}$, wherein

$R^{10}$ denotes hydrogen or straight-chain or branched alkyl or alkenyl each having up to 3 carbon atoms,

$R^{11}$ and $R^{12}$ are identical or different and denote phenyl, hydrogen or straight-chain or branched alkyl having up to 4 carbon atoms or denote straight-chain or branched acyl having up to 5 carbon atoms, which is optionally substituted by a group of the formula $-NR^{13}R^{14}$, wherein

$R^{13}$ and $R^{14}$ are identical or different and denote hydrogen or straight-chain or branched acyl having up to 5 carbon atoms,

$R^6$ represents hydrogen, carboxyl or straight-chain or branched alkoxycarbonyl having up to 3 carbon atoms, or represents straight-chain or branched alkyl having up to 4 carbon atoms, which is optionally substituted by hydroxyl or by a group of the formula $-O-CO-R^{15}$, wherein

$R^{15}$ denotes phenyl which is optionally substituted up to 3 times by identical or different straight-chain or branched alkyl having up to 3 carbon atoms, or denotes straight-chain or branched alkyl or alkenyl each having up to 19 carbon atoms, each of which is optionally substituted by a group of the formula $-OR^{16}$, wherein

$R^{16}$ denotes hydrogen, benzyl, triphenylmethyl or straight-chain or branched acyl having up to 4 carbon atoms,

$R^7$ represents hydrogen or

$R^6$ and $R^7$ together represent the group of the formula =O,

if appropriate in an isomeric form, and their salts.

**4.** 2-[4-(2,4-Dimethyl-5,6,7,8-tetrahydro-α-carbolin-9-yl)methylphenyl]-2-cyclopentylacetic acid N-[(R)-phenylglyci-

nolamide] of the formula

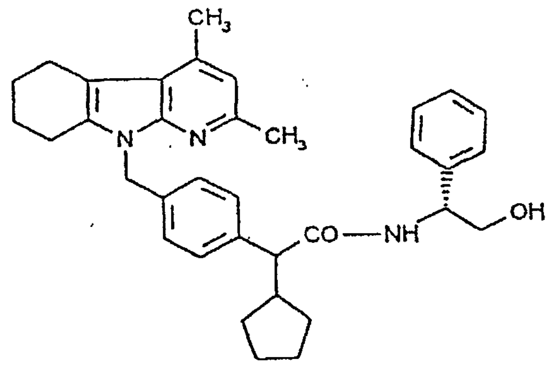

and its salts.

5. 2-[4-(2,4-Dimethyl-α-carbolin-9-yl)methylphenyl]-2-cyclopentylacetic acid N-[(R)-phenylglycinolamide] of the formula

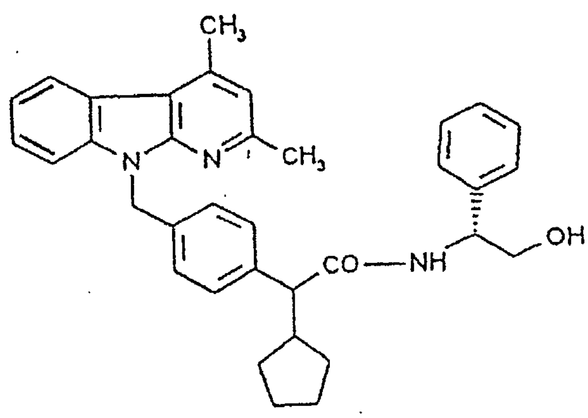

and its salts.

6. 1-(R,S)-1-Phenyl-2-hydroxyethyl 2-(R,S)-2-/4-(2,4-dimethyl-α-carbolin-9-yl)methylphenyl/2-cyclopentylacetate of the formula

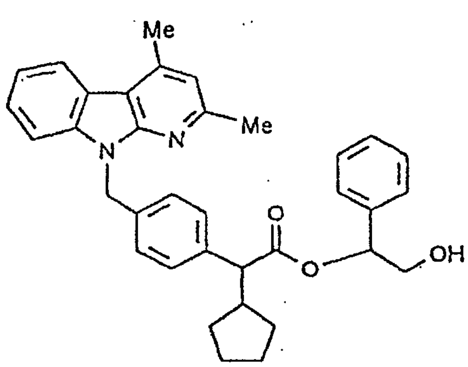

and its salts.

**7.** 2-(R,S)-2-/4-(2,4-Dimethyl-a-carbolin-9-yl)methylphenyl]-2-cyclopentylacetic acid [N-(1-(R,S)-1-phenyl-1-carboxymethyl]amide of the formula

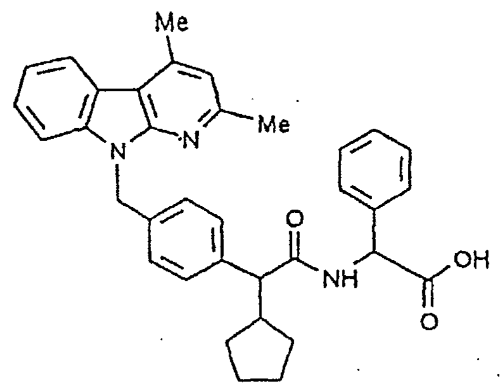

and its salts.

**8.** Cycloalkano-indole and -azaindole derivatives according to one of Claims 1 to 7 for therapeutic use.

**9.** Process for the preparation of cycloalkano-indole and -azaindole derivatives according to one of Claims 1 to 7, **characterized in that**
carboxylic acids of the general formula (II)

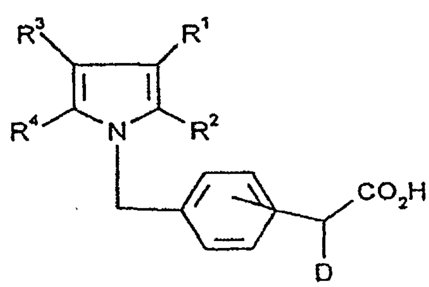

(II)

in which
$R^1$, $R^2$, $R^3$, $R^4$ and D have the meanings indicated in Claim 1,
are amidated using compounds of the general formula (III)

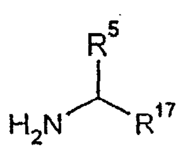

(III)

in which

$R^5$ has the meaning indicated in Claim 1
and
$R^{17}$ has the indicated meaning of $R^6$, but does not represent carboxyl,

in an inert solvent and in the presence of bases and/or auxiliaries,
and, if appropriate, functional groups are varied by hydrolysis, esterification or reduction.

10. Medicaments containing at least one cycloalkano-indole or -azaindole derivative according to one of Claims 1 to 7.

11. Use of cycloalkano-indole and -azaindole derivatives according to one of Claims 1 to 7 for the production of medicaments.

12. Carboxylic acids of the general formula (II)

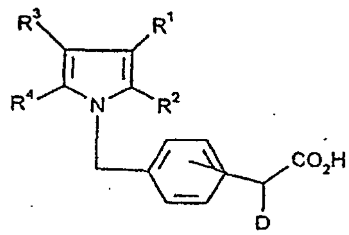
(II)

in which

$R^1$ and $R^2$, including the double bond connecting them, together form a phenyl or pyridyl ring or a ring of formula

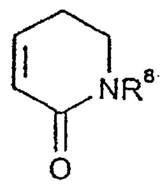

wherein

$R^8$ denotes hydrogen or straight-chain or branched alkyl having up to 4 carbon atoms,

$R^3$ and $R^4$, including the double bond connecting them, together form a phenyl ring or a 4- to 8-membered cycloalkene or oxocycloalkene radical, or ring systems mentioned under $R^1$/$R^2$ and $R^3$/$R^4$ optionally being substituted up to 3 times by identical or different halogen, trifluoromethyl, carboxyl or hydroxyl substituents, by straight-chain or branched alkoxy or alkoxycarbonyl each having up to 6 carbon atoms or by straight-chain or branched alkyl having up to 6 carbon atoms, which, for its part, can be substituted by hydroxyl or by straight-chain or branched alkoxy having up to 4 carbon atoms,

D represents cycloalkyl having 4 to 12 carbon atoms or straight-chain or branched alkyl having up to 12 carbon atoms.

13. Process for the preparation of carboxylic acids according to Claim 12, **characterized in that** compounds of the general formula (IV)

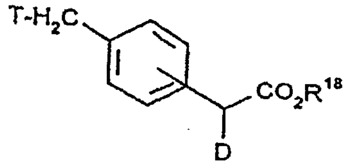
(IV)

in which

D has the meaning indicated in Claim 12,

T represents a typical leaving group such as, for example, chlorine, bromine, iodine, tosylate or mesylate, preferably bromine,
and

$R^{18}$ represents $(C_1\text{-}C_4)$-alkyl,

are reacted with compounds of the general formula (V)

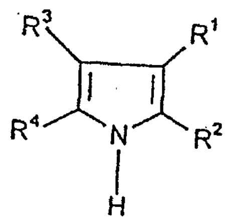

(V)

in which

$R^1$, $R^2$, $R^3$ and $R^4$ have the meanings indicated in Claim 12

in inert solvents, if appropriate in the presence of a base.

## Revendications

**1.** Dérivés de cycloalkano-indoles et azaindoles de formule générale (I)

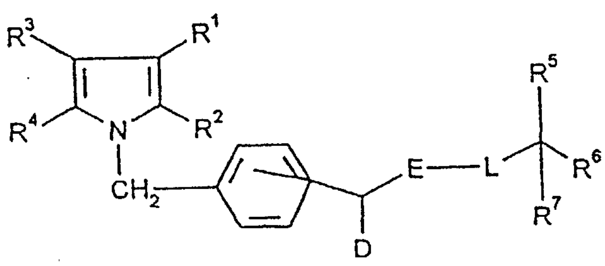

(I),

dans laquelle

$R^1$ et $R^2$ forment, conjointement avec la double liaison qui les lie, un noyau phényle ou pyridyle ou un noyau de formule

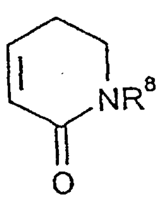

dans laquelle

$R^8$ est l'hydrogène ou un reste alkyle linéaire ou ramifié ayant jusqu'à 4 atomes de carbone,
$R^3$ et $R^4$ forment, conjointement avec la double liaison qui les lie, un noyau phényle ou un reste de cycloalcène ou d'oxocycloalcène tétragonal à octogonal,
tous les systèmes de noyau indiqués pour $R^1/R^2$ et $R^3/R^4$ étant éventuellement substitués jusqu'à 3 fois identiques ou différentes par un halogène, un reste trifluorométhyle, carboxy, hydroxy, par un reste alkoxy ou alkoxycarbonyle linéaire ou ramifié ayant chacun jusqu'à 6 atomes de carbone ou par un reste alkyle linéaire ou ramifié ayant jusqu'à 6 atomes de carbone, qui peut lui-même être substitué par un reste hydroxy ou par un reste alkoxy linéaire ou ramifié ayant jusqu'à 4 atomes de carbone,

D est un reste cycloalkyle ayant 4 à 12 atomes de carbone ou un reste alkyle linéaire ou ramifié ayant jusqu'à 12 atomes de carbone,
E est un groupe -CO- ou -CS-,
L est un atome d'oxygène ou de soufre ou un groupe de formule $-NR^9$,
dans laquelle

$R^9$ représente l'hydrogène ou un reste alkyle linéaire ou ramifié ayant jusqu'à 6 atomes de carbone, qui est éventuellement substitué par un reste hydroxy ou phényle,

$R^5$ est un reste phényle ou hétérocycle saturé ou non saturé, pentagonal à heptagonal ayant jusqu'à 3 hétéroatomes de la série S, N et/ou O,
les cycles étant éventuellement substitués jusqu'à 3 fois identiques ou différentes par un groupe nitro, carboxy, un halogène, un groupe cyano ou par un reste alcényle ou alkoxycarbonyle linéaire ou ramifié ayant chacun jusqu'à 6 atomes de carbone ou par un reste alkyle linéaire ou ramifié ayant jusqu'à 6 atomes de carbone, qui est éventuellement substitué par un reste hydroxy, carboxy ou par un reste alkoxy ou alkoxycarbonyle linéaire ou ramifié ayant chacun jusqu'à 6 atomes de carbone, et/ou les cycles sont éventuellement substitués par un groupe de formule $-OR^{10}$ ou $-NR^{11}R^{12}$,
où

$R^{10}$ représente l'hydrogène ou un reste alkyle ou alcényle linéaire ou ramifié ayant chacun jusqu'à 6 atomes de carbone,
$R^{11}$ et $R^{12}$ sont identiques ou différents et représentent un reste phényle, hydrogène ou un reste alkyle linéaire ou ramifié ayant jusqu'à 6 atomes de carbone
ou bien un reste acyle linéaire ou ramifié ayant jusqu'à 8 atomes de carbone, qui est éventuellement substitué par un groupe de formule $-NR^{12}R^{14}$,
dans laquelle

$R^{13}$ et $R^{14}$ sont identiques ou différents et représentent l'hydrogène ou un reste acyle linéaire ou ramifié ayant jusqu'à 8 atomes de carbone,

$R^6$ représente l'hydrogène, un reste carboxy ou un reste alkoxycarbonyle linéaire ou ramifié ayant jusqu'à 5 atomes de carbone, ou un reste alkyle linéaire ou ramifié ayant jusqu'à 6 atomes de carbone, qui est éventuellement substitué par un reste hydroxyle ou par un groupe de formule $-O-CO-R^{15}$,
où

$R^{15}$ désigne un reste phényle qui. est éventuellement substitué jusqu'à 3 fois identiques ou différentes par un halogène, un reste hydroxy ou par un reste alkyle linéaire ou ramifié ayant jusqu'à 5 atomes de carbone, ou un reste alkyle ou alcényle linéaire ou ramifié ayant chacun jusqu'à 22 atomes de carbone, qui sont éventuellement substitués par un groupe de formule $-OR^{16}$,
où
$R^{16}$ représente l'hydrogène, un reste benzyle, triphénylméthyle ou un reste acyle linéaire ou ramifié ayant jusqu'à 6 atomes de carbone,

$R^7$ représente l'hydrogène, ou bien
$R^6$ et $R^7$ forment ensemble le groupe de formule =O,

le cas échéant, sous une forme isomère, et leurs sels.

**2.** Dérivés de cycloalkano-indoles et azaindoles de formule suivant la revendication 1, dans lesquels

$R^1$ et $R^2$ forment, conjointement avec la double liaison qui les lie, un noyau phényle ou pyridyle ou un noyau de formule

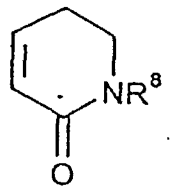

dans laquelle

$R^8$ représente l'hydrogène ou un reste alkyle linéaire ou ramifié ayant jusqu'à 3 atomes de carbone,

$R^3$ et $R^4$ forment, conjointement avec la double liaison qui les lie, un noyau phényle ou un reste de cyclopentène, cyclohéxène, cycloheptène, cyclooctène, oxocyclopentène, oxocyclohéxène, oxocycloheptène ou oxocyclooctène,
tous les systèmes de noyau indiqués pour $R^1$/$R^2$ et $R^3$/$R^4$ étant éventuellement substitués jusqu'à 2 fois identiques ou différentes par du fluor, du chlore, du brome, un reste trifluorométhyle, carboxy, hydroxy, par un reste alkoxy ou alkoxycarbonyle linéaire ou ramifié ayant chacun jusqu'à 4 atomes de carbone ou par un reste alkyle linéaire ou ramifié ayant jusqu'à 4 atomes de carbone, qui peut lui-même être substitué par un reste hydroxy ou par un reste alkoxy linéaire ou ramifié ayant jusqu'à 3 atomes de carbone,

D représente un reste cyclobutyle, cyclopentyle, cyclohéxyle, cycloheptyle, cyclo-octyle ou un reste alkyle linéaire ou ramifié ayant jusqu'à 10 atomes de carbone,

E est un groupe -CO- ou -CS-,

L est un atome d'oxygène ou de soufre ou un groupe de formule $-NR^9$,
dans laauelle

$R^9$ représente l'hydrogène ou un reste alkyle linéaire ou ramifié ayant jusqu'à 5 atomes de carbone, qui est éventuellement substitué par un reste hydroxy ou phényle,

$R^5$ est un reste phényle, pyridyle, furyle, thiényle ou imidazolyle, ces restes étant éventuellement substitués jusqu'à 2 fois identiques ou différentes par un groupe nitro, carboxy, du fluor, du chlore, du brome, un groupe cyano, par un reste alcényle ou alkoxycarbonyle linéaire ou ramifié ayant chacun jusqu'à 4 atomes de carbone ou par un reste alkyle linéaire ou ramifié ayant jusqu'à 5 atomes de carbone, qui est éventuellement substitué par un reste hydroxy, carboxy ou par un reste alkoxy ou alkoxycarbonyle linéaire ou ramifié ayant chacun jusqu'à 5 atomes de carbone,
et/ou les cycles sont éventuellement substitués par un groupe de formule $-OR^{10}$ ou $-NR^{11}R^{12}$,
dans laquelle
$R^{10}$ représente l'hydrogène ou un reste alkyle ou alcényle linéaire ou ramifié ayant chacun jusqu'à 4 atomes de carbone,
$R^{11}$ et $R^{12}$ sont identiques ou différents et représentent un reste phényle, l'hydrogène ou un reste alkyle linéaire ou ramifié ayant jusqu'à 5 atomes de carbone,
ou bien un reste acyle linéaire ou ramifié ayant jusqu'à 6 atomes de carbone, qui est éventuellement substitué par un groupe de formule $-NR^{13}R^{14}$,
dans laquelle

$R^{13}$ et $R^{14}$ sont identiques ou différents et représentent l'hydrogène ou un reste acyle linéaire ou ramifié ayant jusqu'à 6 atomes de carbone,

$R^6$ représente l'hydrogène, un groupe carboxy ou un reste alkoxycarbonyle linéaire ou ramifié ayant jusqu'à 4 atomes de carbone, ou un reste alkyle linéaire ou ramifié ayant jusqu'à 5 atomes de carbone, qui est éventuellement substitué par un groupe hydroxyle ou par un groupe de formule

-O-CO-$R^{15}$,
dans laquelle

$R^{15}$ désigne un reste phényle qui est éventuellement substitué jusqu'à 3 fois identiques ou différentes par du fluor, du chlore, du brome, un reste hydroxy ou par un reste alkyle linéaire ou ramifié ayant jusqu'à 4 atomes de carbone, ou un reste alkyle ou alcényle linéaire ou ramifié ayant chacun jusqu'à 20 atomes de carbone, chacun étant éventuellement substitué par un groupe de formule -$OR^{16}$,
dans laquelle

$R^{16}$ désigne l'hydrogène, un reste benzyle, triphénylméthyle ou un reste acyle linéaire ou ramifié ayant jusqu'à 5 atomes de carbone,

$R^7$ représente l'hydrogène, ou bien
$R^6$ et $R^7$ forment conjointement le groupe de formule =O,

le cas échéant sous une forme isomère, et leurs sels.

**3.** Dérivés de cycloalkano-indoles et azaindoles de formule suivant la revendication 1,
dans lesquels

$R^1$ et $R^2$ forment, conjointement avec la double liaison qui les lie, un noyau phényle ou pyridynile ou un noyau de formule

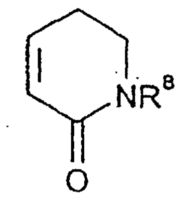

dans laquelle

$R^8$ représente l'hydrogène ou le reste méthyle,

$R^3$ et $R^4$ forment, conjointement avec la double liaison qui les lie, un noyau phényle ou un reste de cyclopentène, cyclohéxène, cycloheptène, cyclooctène, oxocyclopentène, oxocyclohéxène, oxocycloheptène ou oxocyclo-octène,
Tous les systèmes de noyau indiqués pour $R^1$/$R^2$ et $R^3$/$R^4$ étant éventuellement substitués jusqu'à 2 fois identiques ou différentes par du fluor, du chlore, du brome, un reste trifluorométhyle, carboxy, hydroxy, par un reste alkoxy ou alkoxycarbonyle linéaire ou ramifié ayant chacun jusqu'à 3 atomes de carbone ou par un reste alkyle linéaire ou ramifié ayant jusqu'à 3 atomes de carbone, qui peut lui-même être substitué par un reste hydroxy, méthoxy ou éthoxy,
D est un reste cyclopentyle, cyclohéxyle, cycloheptyle, cyclo-octyle ou un reste alkyle linéaire ou ramifié ayant jusqu'à 6 atomes de carbone,
E est un groupe -CO- ou -CS-,
L est un atome d'oxygène ou de soufre ou un groupe de formule -$NR^9$,
dans laquelle

$R^9$ représente l'hydrogène ou un reste alkyle linéaire ou ramifié ayant jusqu'à 4 atomes de carbone, qui est éventuellement substitué par un reste hydroxy ou phényle,

$R^5$ est un reste phényle, pyridyle ou thiényle, ces restes étant éventuellement substitués jusqu'à 2 fois identiques ou différentes par un groupe nitro, carboxy, du fluor, du chlore, du brome, un groupe cyano, par un reste alcényle ou alkoxycarbonyle linéaire ou ramifié ayant chacun jusqu'à 3 atomes de carbone ou par un reste alkyle linéaire ou ramifié ayant jusqu'à 4 atomes de carbone, qui est éventuellement substitué par un reste hydroxy, carboxy ou par un reste alkoxy ou alkoxycarbonyle

linéaire ou ramifié ayant chacun jusqu'à 4 atomes de carbone, et/ou les cycles sont éventuellement substitués par un groupe de formule $OR^{10}$ ou $-NR^{11}R^{12}$, où

$R^{10}$ désigne l'hydrogène ou un reste alkyle ou alcényle linéaire ou ramifié ayant chacun jusqu'à 3 atomes de carbone,

$R^{11}$ et $R^{12}$ sont identiques ou différents et représentent un reste phényle, l'hydrogène ou un reste alkyle linéaire ou ramifié ayant jusqu'à 4 atomes de carbone, ou un reste acyle linéaire ou ramifié ayant jusqu'à 5 atomes de carbone, qui est éventuellement substitué par un groupe de formule $-NR^{13}R^{14}$, où

$R^{13}$ et $R^{14}$ sont identiques ou différents et représentent l'hydrogène ou un reste acyle linéaire ou ramifié ayant jusqu'à 5 atomes de carbone,

$R^6$ représente l'hydrogène, un groupe carboxy ou un reste alkoxycarbonyle linéaire ou ramifié ayant jusqu'à 3 atomes de carbone, ou un reste alkyle linéaire ou ramifié ayant jusqu'à 4 atomes de carbone, qui est éventuellement substitué par un reste hydroxyle ou par un groupe de formule $-O-CO-R^{15}$, où

$R^{15}$ désigne un reste phényle qui est éventuellement substitué jusqu'à 3 fois identiques ou différentes par un reste alkyle linéaire ou ramifié ayant jusqu'à 3 atomes de carbone, ou un reste alkyle ou alcényle linéaire ou ramifié avec chacun jusqu'à 19 atomes de carbone, qui sont éventuellement substitués par un groupe de formule $-OR^{16}$, dans laquelle

$R^{16}$ représente l'hydrogène, un reste benzyle, triphénylméthyle ou un reste acyle linéaire ou ramifié ayant jusqu'à 4 atomes de carbone,

$R^7$ représente l'hydrogène, ou bien
$R^6$ et $R^7$ forment ensemble le groupe de formule =O,

éventuellement sous une forme isomère, et leurs sels.

**4.** Le N-[(R)-phénylglycinolamide] de l'acide 2-[4-(2,4-diméthyl-5,6,7,8-tétrahydro-α-carboline-9-yl)méthylphényl]-2-cyclopentyle acétique de formule

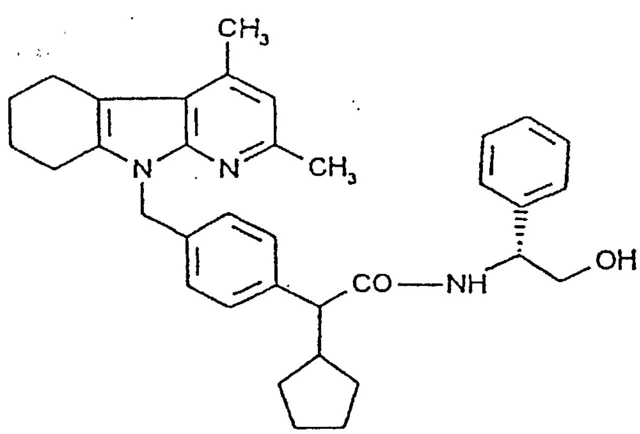

et ses sels.

**5.** Le N-[(R)-phénylglycinolamide] de l'acide 2-[4-(2,4-diméthyl-α-carboline-9-yl)méthyl-phényl]-2-cyclopentyle acétique de formule

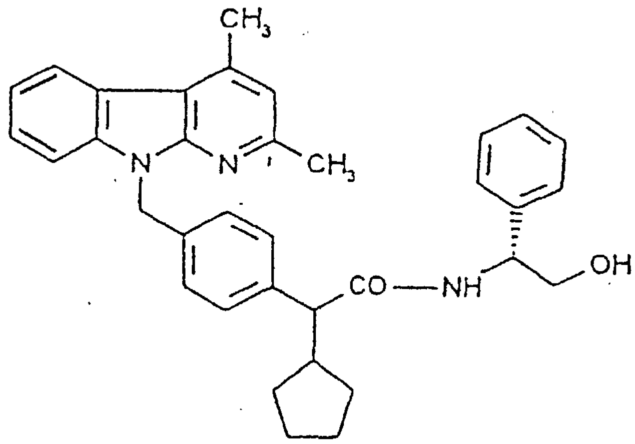

et ses sels.

6. L'ester 1-(R,S)-1-phényl-2-hydroxy-éthylique de l'acide 2-(R,S)-2-/4-(2,4-diméthyl-α-carboline-9-yl)méthylphényl]-2-cyclopentyle acétique de formule

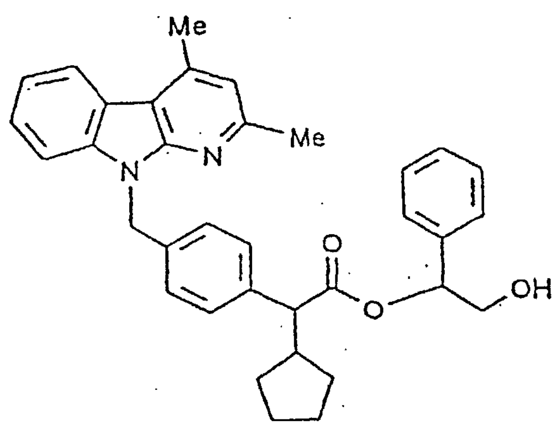

et ses sels.

7. Le [N-(1-(R,S)-1-phényl-1-carboxy-méthyl)amide de l'acide 2-(R,S)-2-/4-(2,4-diméthyl-α-carboline-9-yl)méthylphényl]-2-cyclopentyle acétique de formule

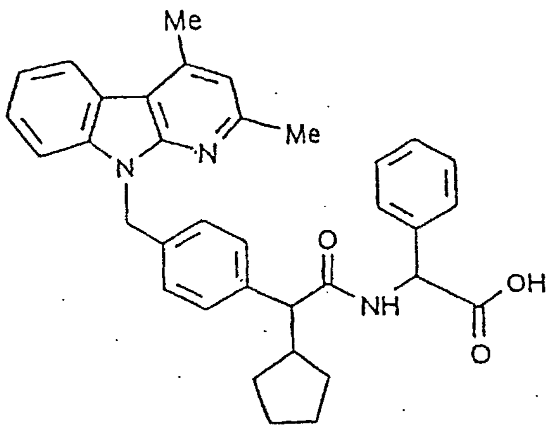

et ses sels

**8.** Dérivés de cycloalkano-indoles et azaindoles suivant l'une des revendications 1 à 7, destinés à une utilisation thérapeutique.

**9.** Procédé de production de dérivés de cycloalkano-indoles et azaindoles suivant l'une des revendications 1 à 7, **caractérisé en ce qu'**on effectue l'amidation d'acides carboxyliques de formule générale (II)

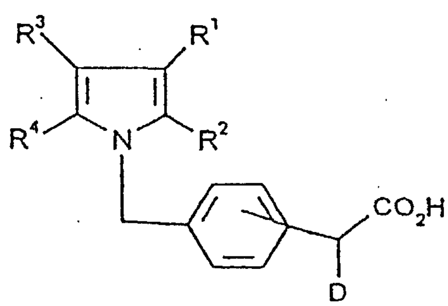

(II)

dans laquelle
$R^1$, $R^2$, $R^3$, $R^4$ et D ont les définitions indiquées dans la revendication 1
avec des composés de formule générale (III)

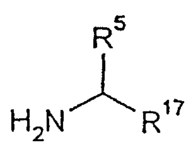

(III)

dans laquelle
$R^5$ a la définition indiquée dans la revendication 1
et
$R^{17}$ a la définition indiquée pour $R^6$, mais ne représente pas un groupe carboxy,

dans un solvant inerte et en présence de bases et/ou de substances auxiliaires,
et on fait varier éventuellement des groupes fonctionnels par hydrolyse, estérification ou réduction.

**10.** Médicament contenant au moins un dérivé de cycloalkano-indole ou azaindole suivant l'une des revendications 1 à 7.

**11.** Utilisation de dérivés de cycloalkano-indoles ou azaindoles suivant l'une des revendications 1 à 7 pour la préparation de médicaments.

**12.** Acides carboxyliques de formule générale (II)

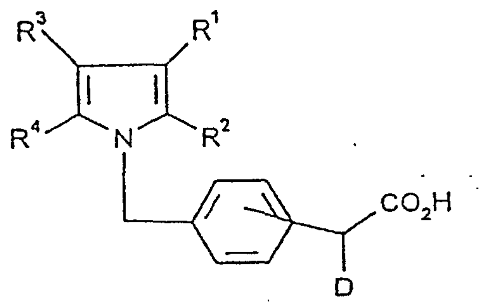

(II)

dans laquelle

$R^1$ et $R^2$ forment, conjointement avec la double liaison qui les lie, un noyau phényle ou pyridyle ou un noyau de formule

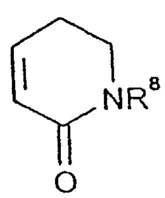

dans laquelle

$R^8$ représente l'hydrogène ou un reste alkyle linéaire ou ramifié ayant jusqu'à 4 atomes de carbone,

$R^3$ et $R^4$ forment, conjointement avec la double liaison qui les lie, un noyau phényle ou un reste de cycloalcène ou d'oxocycloalcène, tétragonal à octogonal,
tous les systèmes de noyaux indiqués pour $R^1$/$R^2$ et $R^3$/$R^4$ étant éventuellement substitués jusqu'à 3 fois identiques ou différentes par un halogène, un reste trifluorométhyle, carboxy, hydroxy, par un reste alkoxy ou alkoxycarbonyle linéaire ou ramifié ayant chacun jusqu'à 6 atomes de carbone ou par un reste alkyle linéaire ou ramifié ayant jusqu'à 6 atomes de carbone, qui peut lui-même être substitué par un reste hydroxy ou par un reste alkoxy linéaire ou ramifié ayant jusqu'à 4 atomes de carbone,

D est un reste cycloalkyle ayant 4 à 12 atomes de carbone ou un reste alkyle linéaire ou ramifié ayant jusqu'à 12 atomes de carbone.

**13.** Procédé de production d'acides carboxyliques suivant la revendication 12, **caractérisé en ce qu'**on fait réagir des composés de formule générale (IV)

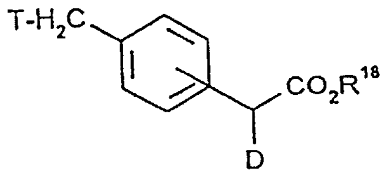

(IV)

dans laquelle

D a la définition indiquée dans la revendication 12,

T est un groupe partant typique tel que par exemple chlore, brome, iode, tosylate ou mesylate, de préférence brome,

et

$R^{18}$ est un reste alkyle en $C_1$ à $C_4$,

avec des composés de formule générale (V)

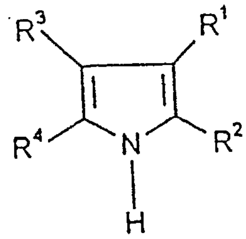

(V)

dans laquelle

$R^1$, $R^2$, $R^3$ et $R^4$ ont les définitions indiquées dans la revendication 12,

dans des solvants inertes, éventuellement en présence d'une base.